# EUROPEAN PATENT APPLICATION

(11) **EP 4 843 983 A1**
(43) Date of publication of application: **25.11.2026**
(21) Application number: 25742075.2
(22) Date of filing: 14.01.2025
(51) Int. Cl.: C07D 403/10, C07D 405/14, C07D 409/14, H10K 85/60

(54) **COMPOUND FOR ORGANIC OPTOELECTRONIC DIODE, COMPOSITION FOR ORGANIC OPTOELECTRONIC DIODE, ORGANIC OPTOELECTRONIC DIODE, AND DISPLAY DEVICE**

(30) Priority: 15.01.2024 KR 20240006321
(71) Applicant: SAMSUNG SDI CO., LTD., Yongin-si, Gyeonggi-do 17084 (KR)
(72) Inventor: KIM, Jonghoon, Suwon-si, Gyeonggi-do 16678 (KR); LEE, Hanill, Suwon-si, Gyeonggi-do 16678 (KR); IM, Suheon, Suwon-si, Gyeonggi-do 16678 (KR); LEE, Mijin, Suwon-si, Gyeonggi-do 16678 (KR); LEE, Seungjae, Suwon-si, Gyeonggi-do 16678 (KR); KIM, Wook, Suwon-si, Gyeonggi-do 16678 (KR); KWON, Jiyun, Suwon-si, Gyeonggi-do 16678 (KR); GWAK, Seonyeong, Suwon-si, Gyeonggi-do 16678 (KR); PARK, Minji, Suwon-si, Gyeonggi-do 16678 (KR); HUH, Dalho, Suwon-si, Gyeonggi-do 16678 (KR); KIM, Hyung Sun, Suwon-si, Gyeonggi-do 16678 (KR); JUNG, Ho Kuk, Suwon-si, Gyeonggi-do 16678 (KR); YOON, Jiah, Suwon-si, Gyeonggi-do 16678 (KR); HWANG, Seunghoon, Suwon-si, Gyeonggi-do 16678 (KR)
(74) Representative: Michalski Hüttermann & Partner mbB
(86) International application number: PCT/KR2025/000776
(87) International publication number: WO 2025/155059

(57) **Abstract**

Disclosed are a compound for an organic optoelectronic device represented by Chemical Formula 1A or Chemical Formula 1B, a composition for an organic optoelectronic device including the same, an organic optoelectronic device, and a display device.

Details of Chemical Formula 1A and Chemical Formula 1B are as defined in the specification.

## Description

### [Technical Field]

A compound for an organic optoelectronic device, a composition for an organic optoelectronic device, an organic optoelectronic device, and a display device are disclosed.

### [Background Art]

An organic optoelectronic device (organic optoelectronic diode) is a device capable of converting electrical energy and optical energy to each other.

Organic optoelectronic devices may be largely divided into two types according to a principle of operation. One is a photoelectric device that generates electrical energy by separating excitons formed by light energy into electrons and holes, and transferring the electrons and holes to different electrodes, respectively and the other is light emitting device that generates light energy from electrical energy by supplying voltage or current to the electrodes.

Examples of the organic optoelectronic device include an organic photoelectric device, an organic light emitting diode, an organic solar cell, and an organic photoconductor drum.

Among them, organic light emitting diodes (OLEDs) are attracting much attention in recent years due to increasing demands for flat panel display devices. The organic light emitting diode is a device that converts electrical energy into light, and the performance of the organic light emitting diode is greatly influenced by an organic material between electrodes.

### [Disclosure]

### [Technical Problem]

An embodiment provides a compound for an organic optoelectronic device capable of lowering the driving voltage and implementing high-efficiency and long life-span organic optoelectronic device.

Another embodiment provides a composition for an organic optoelectronic device including the compound for the organic optoelectronic device.

Another embodiment provides an organic optoelectronic device including the compound for the organic optoelectronic device or composition for the organic optoelectronic device.

Another embodiment provides a display device including the organic optoelectronic device.

### [Technical Solution]

According to an embodiment, a compound for an organic optoelectronic device represented by Chemical Formula 1A or Chemical Formula 1B is provided.

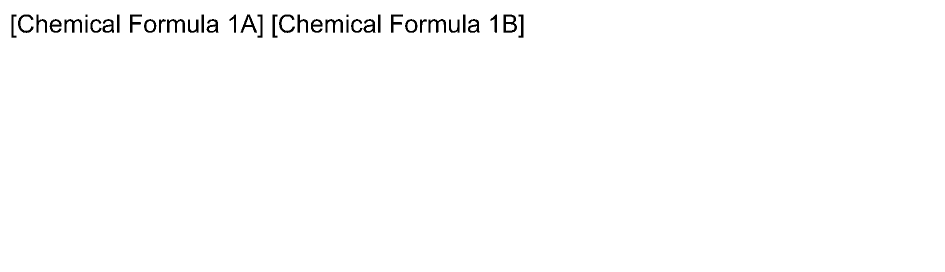

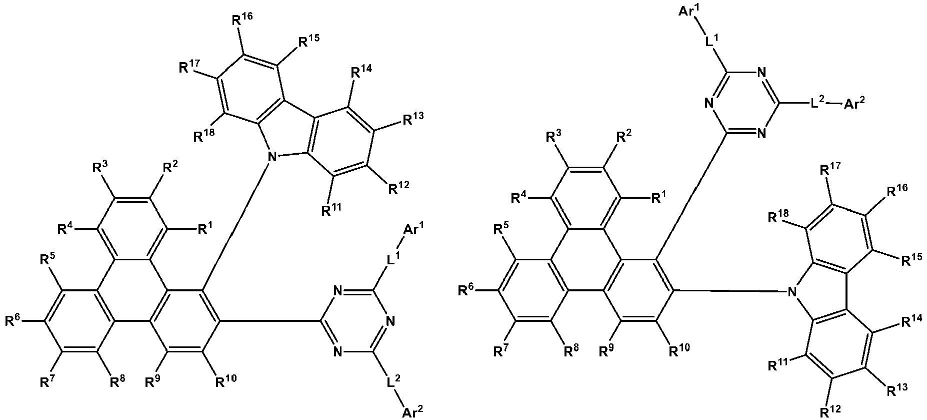

In Chemical Formula 1A and Chemical Formula 1B,
Ar¹ and Ar² are each independently a substituted or unsubstituted C6 to C30 aryl group, or a substituted or unsubstituted C2 to C30 heterocyclic group,
L¹ and L² are each independently a single bond, a substituted or unsubstituted C6 to C30 arylene group, or a substituted or unsubstituted C2 to C30 heterocyclic group, and
R¹ to R¹⁸ are each independently hydrogen, deuterium, a cyano group, a halogen, a substituted or unsubstituted C1 to C30 alkyl group, a substituted or unsubstituted C6 to C30 aryl group, or a substituted or unsubstituted C2 to C30 heterocyclic group.

According to another embodiment, a composition for an organic optoelectronic device including a first compound and a second compound is provided.

The first compound may be the aforementioned compound for the organic optoelectronic device, and the second compound may be represented by Chemical Formula 2; a combination of Chemical Formula 3 and Chemical Formula 4; or Chemical Formula 5.

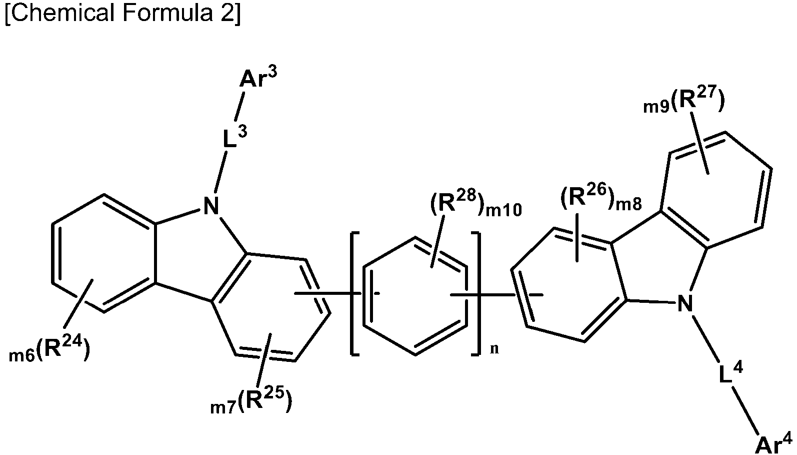

In Chemical Formula 2,
R²⁴ to R²⁸ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar³ and Ar⁴ are each independently a substituted or unsubstituted C6 to C20 aryl group or a substituted or unsubstituted C2 to C30 heterocyclic group,
L³ and L⁴ are each independently a single bond or a substituted or unsubstituted C6 to C20 arylene group,
when m6, m9, and m10 are each independently one of integers of 1 to 4,
when m6 is 2 or more, each R²⁴ is the same as or different from each other,
when m9 is 2 or more, each R²⁷ is the same as or different from each other,
when m10 is 2 or more, each R²⁸ is the same as or different from each other,
m7 and m8 are each independently one of integers of 1 to 3,
when m7 is 2 or more, each R²⁵ is the same as or different from each other,
when m8 is 2 or more, each R²⁶ is the same as or different from each other, and
n is one of integers of 0 to 2; 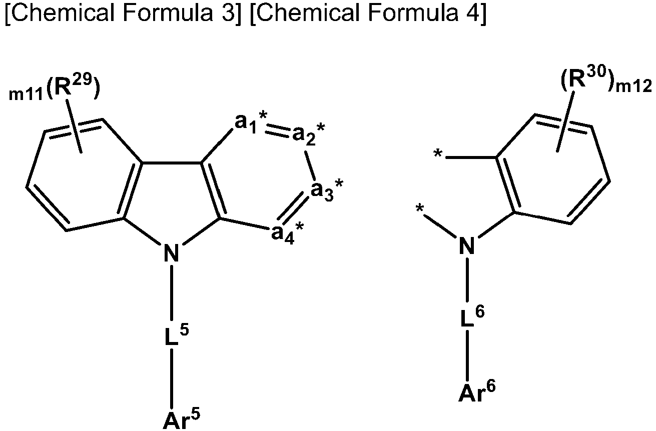
wherein, in Chemical Formula 3 and Chemical Formula 4,
among a1* to a4* in Chemical Formula 3, two adjacent ones are each a linking carbon (C) that is linked to *s in Chemical Formula 4,
among a₁* to a₄* of Chemical Formula 3, the remaining two that are not linked to Chemical Formula 4 are C-L^{a}-R^{a},
L^{a}, L⁵, and L⁶ are each independently a single bond or a substituted or unsubstituted C6 to C20 arylene group,
R^{a}, R²⁹, and R³⁰ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group,
m11 and m12 are each independently one of integers of 1 to 4,
when m11 is 2 or more, each R²⁹ is the same as or different from each other, and
when m12 is 2 or more, each R³⁰ is the same as or different from each other; 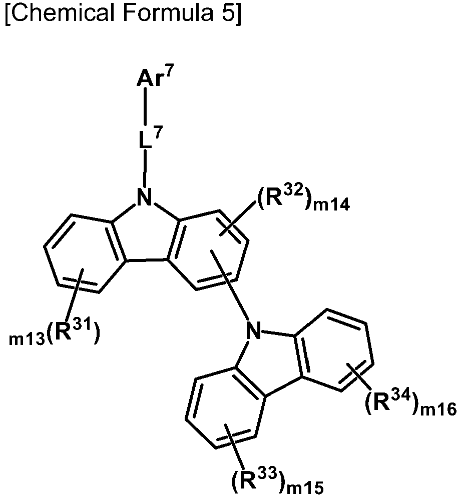
wherein, in Chemical Formula 5,
L⁷s are each independently a single bond or a substituted or unsubstituted C6 to C20 arylene group,
R³¹ to R³⁴ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁷ is a substituted or unsubstituted C6 to C20 aryl group or a substituted or unsubstituted C2 to C30 heterocyclic group,
m13, m15, and m16 are each independently one of integers of 1 to 4,
m14 is one of integers of 1 to 3,
when m13 is 2 or more, each R³¹ is the same as or different from each other,
when m14 is 2 or more, each R³² is the same as or different from each other,
when m15 is 2 or more, each R³³ is the same as or different from each other, and
when m16 is 2 or more, each R³⁴ is the same as or different from each other.

According to another embodiment, an organic optoelectronic device includes an anode and a cathode facing each other, and at least one organic layer between the anode and the cathode, and the organic layer includes the compound for the organic optoelectronic device or the composition for the organic optoelectronic device.

According to another embodiment, a display device including the organic optoelectronic device is provided.

### [Advantageous Effects]

High-efficiency, long life-span organic optoelectronic devices may be realized while lowering the driving voltage.

### [Description of the Drawings]

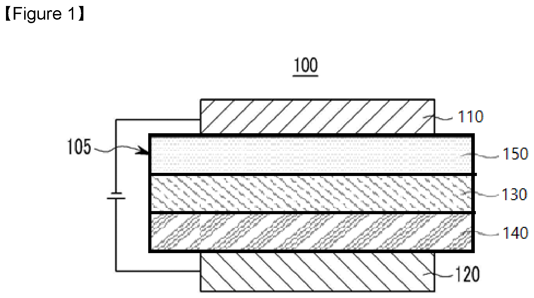

FIG. 1 is a cross-sectional view illustrating an organic light emitting diode according to an embodiment.

### <Description of Symbols>

100: organic light emitting diode
105: organic layer
110: cathode
120: anode
130: light emitting layer
140: hole transport region
150: electron transport region

### [Best Mode]

Hereinafter, embodiments of the present invention are described in detail. However, these are presented as examples, and the present invention is not limited thereto, and the present invention is only defined by the scope of the claims to be described later.

In the present specification, when a definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a cyano group, or a combination thereof.

In one example of the present invention, the "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, or a cyano group. In a specific example of the present invention, the "substituted" refers to replacement of at least one hydrogen of a substituent or a compound by deuterium, a C1 to C20 alkyl group, a C1 to C5 alkylsilyl group, a C6 to C20 aryl group, a C2 to C20 heteroaryl group, or a cyano group. In a specific example of the present invention, the "substituted" refers to replacement of at least one hydrogen of a substituent or a compound by deuterium, a C1 to C5 alkyl group, a C1 to C5 alkylsilyl group, a C6 to C18 aryl group, a C2 to C18 heteroaryl group, or a cyano group. In a specific example of the present invention, the "substituted" refers to replacement of at least one hydrogen of a substituent or a compound by deuterium, a cyano group, a methyl group, an ethyl group, an n-propyl group, an iso-propyl group, an n-butyl group, an iso-butyl group, a sec-butyl group, a tert-butyl group, a trimethylsilyl group, a phenyl group, a biphenyl group, a terphenyl group, or a naphthyl group.

In the present specification, "unsubstituted" refers to non-replacement of a hydrogen atom by another substituent and remaining of the hydrogen atom.

In the present specification, hydrogen (-H)" may include "deuterium substitution (-D)" or "tritium substitution (-T).".

In the present specification, when a definition is not otherwise provided, "hetero" refers to one including one to three heteroatoms selected from N, O, S, P, and Si, and remaining carbons in one functional group.

In the present specification, "aryl group" refers to a group including at least one hydrocarbon aromatic moiety, and all elements of the hydrocarbon aromatic moiety have p-orbitals which form conjugation, for example a phenyl group, a naphthyl group, and the like, two or more hydrocarbon aromatic moieties may be linked by a sigma bond and may be, for example a biphenyl group, a terphenyl group, a quaterphenyl group, and the like, and two or more hydrocarbon aromatic moieties are fused directly or indirectly to provide a non-aromatic fused ring, for example a fluorenyl group.

The aryl group may include a monocyclic, polycyclic, or fused ring polycyclic (i.e., rings sharing adjacent pairs of carbon atoms) functional group.

In the present specification, "heterocyclic group" is a generic concept of a heteroaryl group, and may include at least one heteroatom selected from N, O, S, P, and Si instead of carbon (C) in a cyclic compound such as aryl group, a cycloalkyl group, a fused ring thereof, or a combination thereof. When the heterocyclic group is a fused ring, the entire ring or each ring of the heterocyclic group may include one or more heteroatoms.

For example, "heteroaryl group" may refer to aryl group including at least one heteroatom selected from N, O, S, P, and Si. Two or more heteroaryl groups are linked by a sigma bond directly, or when the heteroaryl group includes two or more rings, the two or more rings may be fused. When the heteroaryl group is a fused ring, each ring may include one to three heteroatoms.

More specifically, the substituted or unsubstituted C6 to C30 aryl group may be a substituted or unsubstituted phenyl group, a substituted or unsubstituted naphthyl group, a substituted or unsubstituted anthracenyl group, a substituted or unsubstituted phenanthrenyl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted o-terphenyl group, a substituted or unsubstituted chrysenyl group, a substituted or unsubstituted benzophenanthrenyl group, a substituted or unsubstituted triphenylene group, a substituted or unsubstituted perylenyl group, a substituted or unsubstituted fluorenyl group, a substituted or unsubstituted indenyl group, or a combination thereof, but is not limited thereto.

More specifically, the substituted or unsubstituted C2 to C30 heterocyclic group may be a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted carbazolyl group, a substituted or unsubstituted dibenzofuranyl group, or a substituted or unsubstituted dibenzothiophenyl group, a substituted or unsubstituted benzonaphthol furanyl group, a substituted or unsubstituted benzonaphthothiophenyl group, a substituted or unsubstituted benzofuranofluorenyl group, a substituted or unsubstituted benzothiophenefluorenyl group, or a combination thereof, but is not limited thereto.

In the present specification, hole characteristics refer to an ability to donate an electron to form a hole when an electric field is applied and that a hole formed in the anode may be easily injected into the light emitting layer and transported in the light emitting layer due to conductive characteristics according to a highest occupied molecular orbital (HOMO) level.

In addition, electron characteristics refer to an ability to accept an electron when an electric field is applied and that electron formed in the cathode may be easily injected into the light emitting layer and transported in the light emitting layer due to conductive characteristics according to a lowest unoccupied molecular orbital (LUMO) level.

Hereinafter, a compound for an organic optoelectronic device according to an embodiment is described.

A compound for an organic optoelectronic device according to an embodiment is represented by Chemical Formula 1A or Chemical Formula 1B.

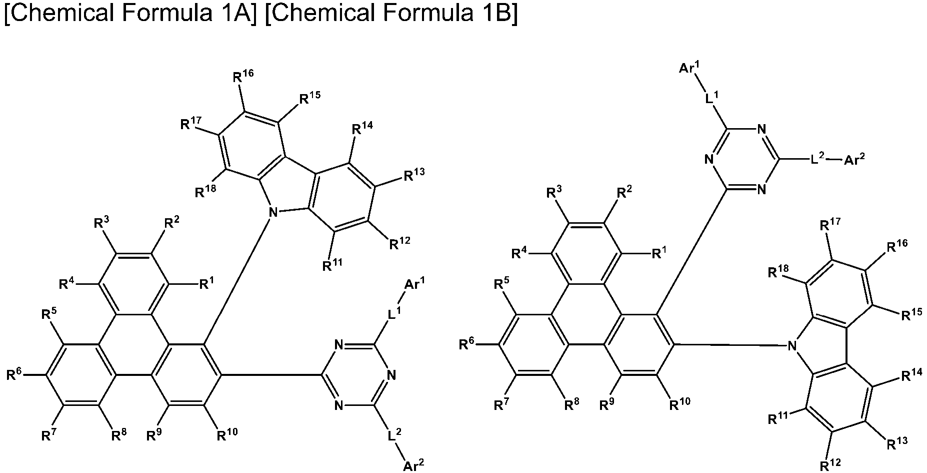

In Chemical Formula 1A and Chemical Formula 1B,
Ar¹ and Ar² are each independently a substituted or unsubstituted C6 to C30 aryl group, or a substituted or unsubstituted C2 to C30 heterocyclic group,
L¹ and L² are each independently a single bond, a substituted or unsubstituted C6 to C30 arylene group, or a substituted or unsubstituted C2 to C30 heterocyclic group, and
R¹ to R¹⁸ are each independently hydrogen, deuterium, a cyano group, a halogen, a substituted or unsubstituted C1 to C30 alkyl group, a substituted or unsubstituted C6 to C30 aryl group, or a substituted or unsubstituted C2 to C30 heterocyclic group.

The compound represented by Chemical Formula 1A and Chemical Formula 1B is substituted with triazine and N-carbazole at 1st and 2nd positions of triphenylene.

The compound represented by the Chemical Formula 1A or Chemical Formula 1B is a structure in which triazine and N-carbazole are simultaneously substituted at 1st and 2nd positions of triphenylene, and has particularly excellent energy transfer efficiency to a phosphorescent dopant, so it may be used as an advantageous material as a phosphorescent host. The triazine that substitutes triphenylene included in the Chemical Formula 1A or Chemical Formula 1B speeds up electron mobility and increases stability, thereby improving the life-span when applied as a phosphorescent host. Also, by substituted with triphenylene and N-carbazole, it helps to expand the resonance structure of the carbazole moiety, which helps to stabilize the hole transport part in the molecule. As a result, the effect of optimizing the charge balance between the electron transport and hole transport parts in the molecule is obtained, which further helps to improve the life-span.

Also, as the triazine and N-carbazole are substituted at adjacent positions of the triphenylene core, the dihedral angle increases due to steric hindrance between triazine and carbazole, and the triazine moiety and the carbazole moiety are twisted with each other, increasing the dihedral angle. This results in a form in which most of the electron cloud overlap between the HOMO level and the LUMO level is separated, and rapid energy transfer is possible because of having a small ΔEst, so it shows high efficiency characteristics especially when applied as a phosphorescent host. In addition, side reaction paths in the excited state are reduced, so that a further life-span increasing effect may be obtained.

For example, Ar¹ and Ar² may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, a substituted or unsubstituted triphenylene group, a substituted or unsubstituted dibenzofuranyl group, a substituted or unsubstituted dibenzothiophenyl group, a substituted or unsubstituted carbazolyl group, or a substituted or unsubstituted dibenzosilolyl group.

As a specific example, Ar¹ and Ar² may each independently be a substituted or unsubstituted phenyl group, a substituted or unsubstituted biphenyl group, a substituted or unsubstituted dibenzofuranyl group, or a substituted or unsubstituted dibenzothiophenyl group.

For example, L¹-Ar¹ and L²-Ar² may each independently be one selected from substituents listed in the following Group I .

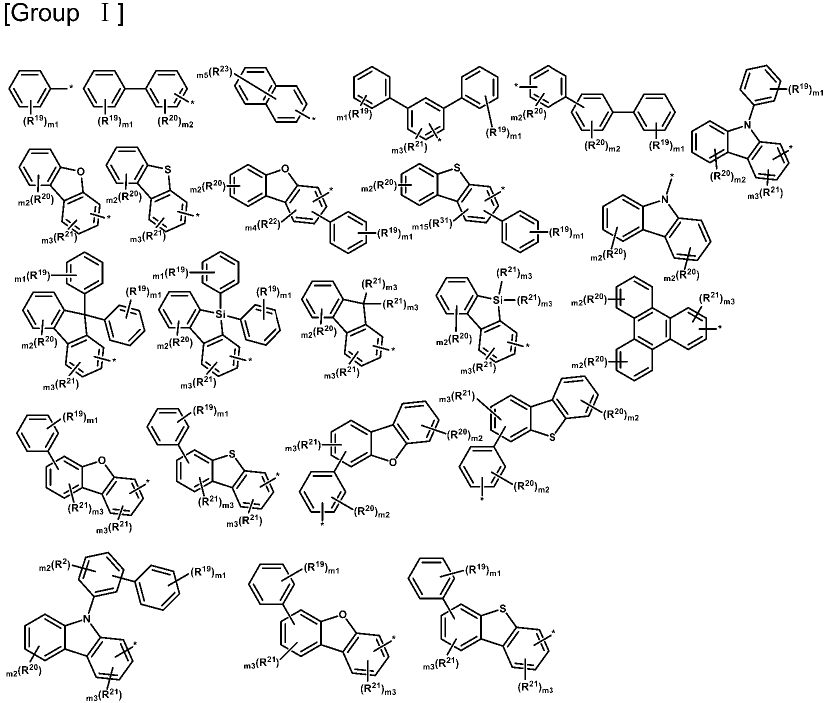

In Group I ,
R¹⁹ to R²³ are each independently hydrogen, deuterium, a cyano group, a C1 to C10 alkyl group, or a C6 to C12 aryl group,
m1 is one of integers of 1 to 5,
m2 is one of integers of 1 to 4,
m3 is one of integers of 1 to 3,
m4 is an integer of 1 or 2,
m5 is one of integers of 1 to 7, and
* is a linking point.

When m1 is 2 or more, each R¹⁹ may be the same or different from each other.

When m2 is 2 or more, each R²⁰ may be the same or different from each other.

When m3 is 2 or more, each R²¹ may be the same or different from each other.

When m5 is 2 or more, each R²³ may be the same or different from each other.

For example, R¹ to R¹⁸ may each independently be hydrogen, deuterium, or a substituted or unsubstituted C6 to C12 aryl group.

As a specific example, R¹ to R¹⁸ may each independently be hydrogen, deuterium, a substituted or unsubstituted phenyl group, or a substituted or unsubstituted biphenyl group.

In a specific embodiment, the compound for the organic optoelectronic device represented by Chemical Formula 1A or Chemical Formula 1B may be one selected from compounds listed in Group 1 below, but is not limited thereto. 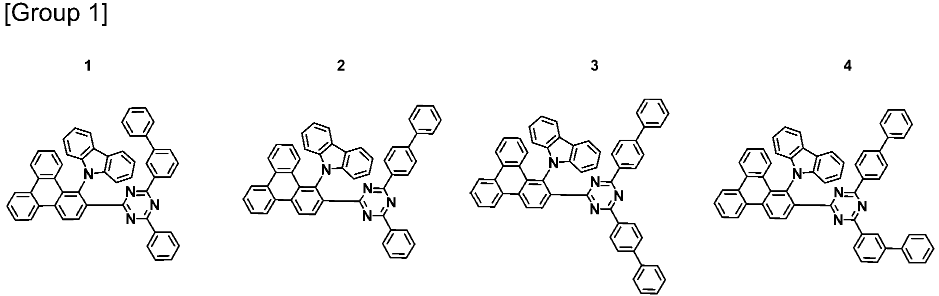 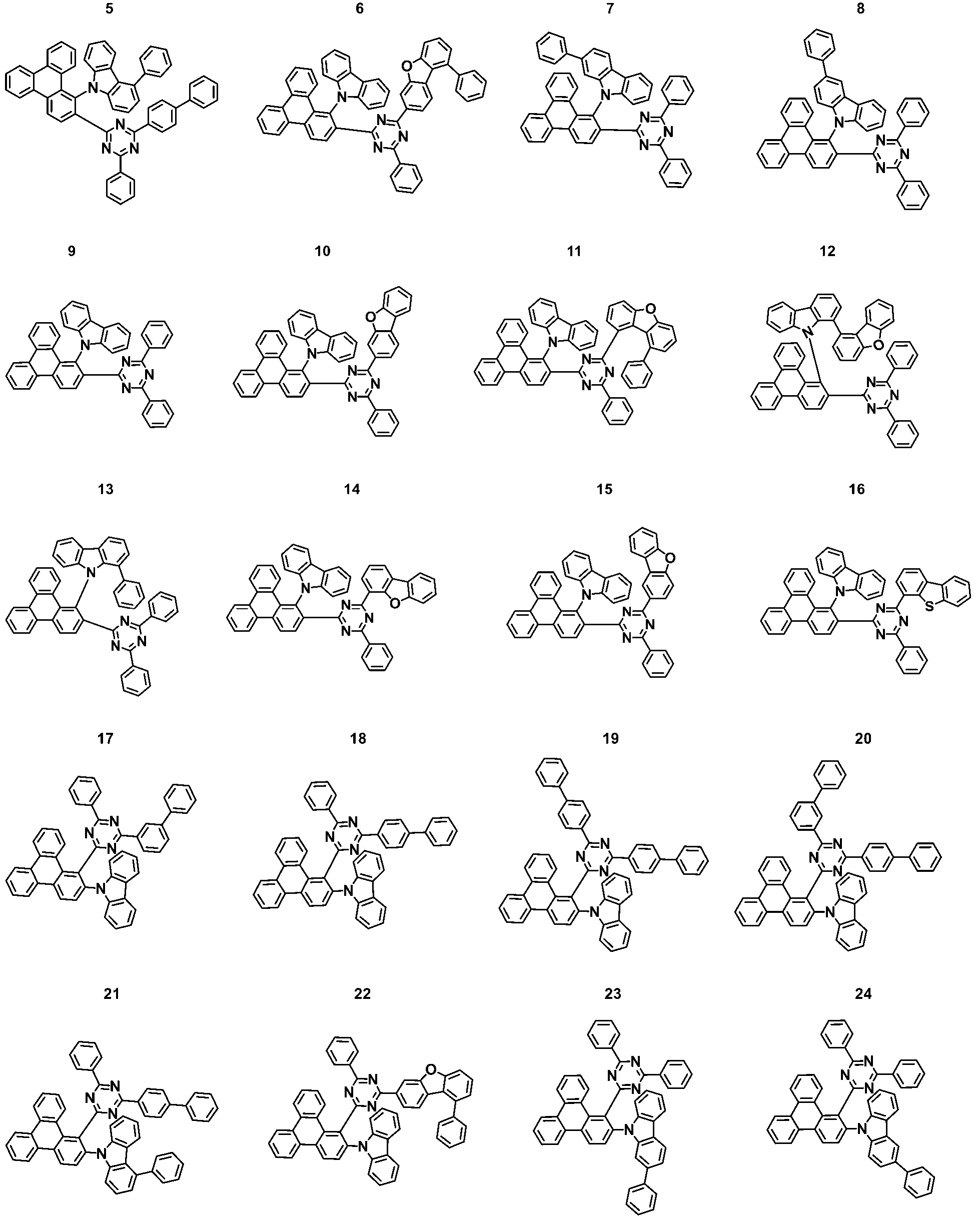 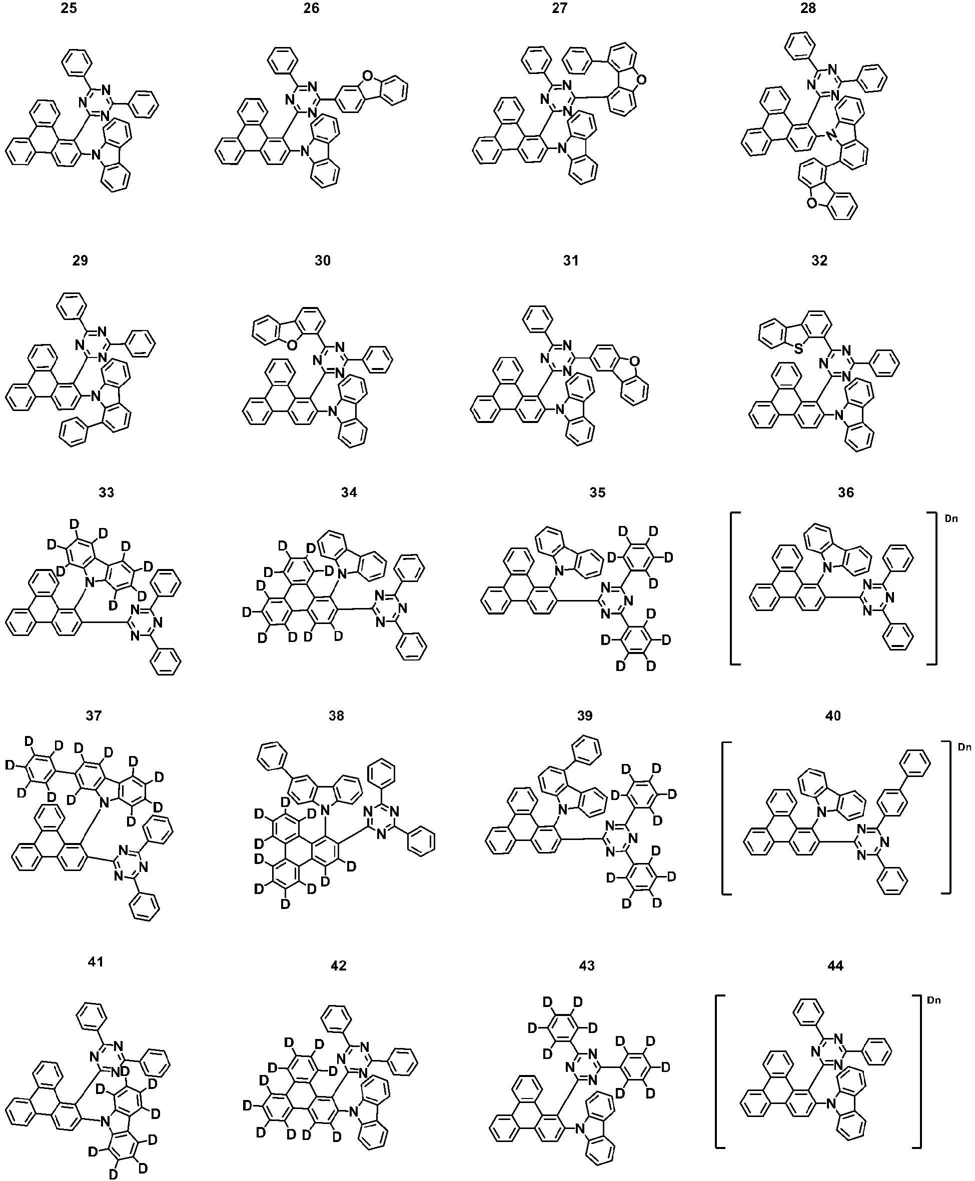 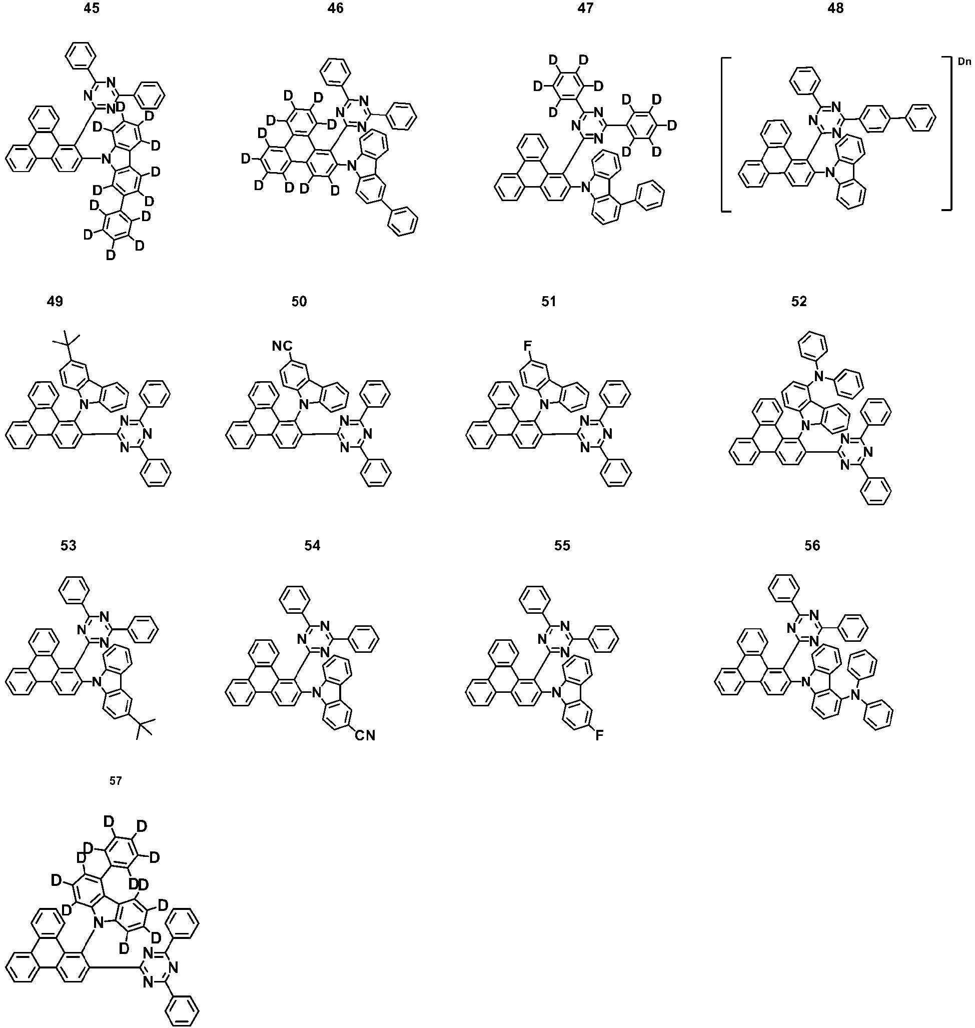 (Dn refers to the number of deuterium substitutions and indicates a structure substituted with one or more deuteriums).

A composition for an organic optoelectronic device according to another embodiment includes a first compound and a second compound, wherein the first compound is the aforementioned compound for the organic optoelectronic device, and the second compound may be represented by Chemical Formula 2; a combination of Chemical Formula 3 and Chemical Formula 4; or Chemical Formula 5.

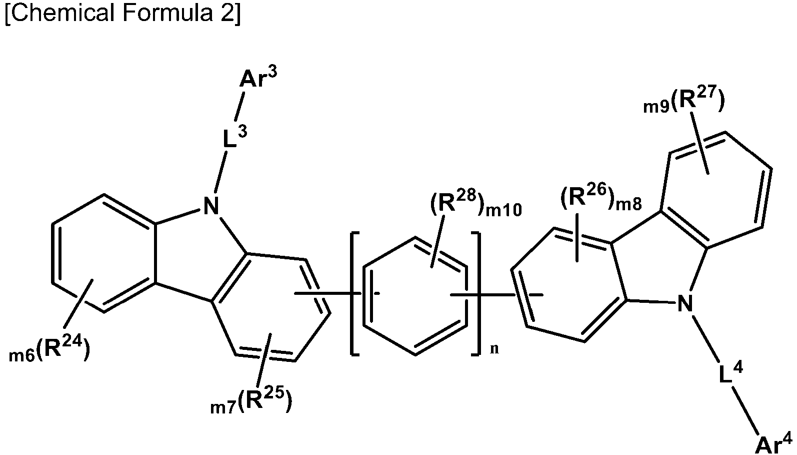

In Chemical Formula 2,
R²⁴ to R²⁸ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar³ and Ar⁴ are each independently a substituted or unsubstituted C6 to C20 aryl group or a substituted or unsubstituted C2 to C30 heterocyclic group,
L³ and L⁴ are each independently a single bond or a substituted or unsubstituted C6 to C20 arylene group,
m6, m9, and m10 are each independently one of integers of 1 to 4,
m7 and m8 are each independently one of integers of 1 to 3, and
n is one of integers of 0 to 2; 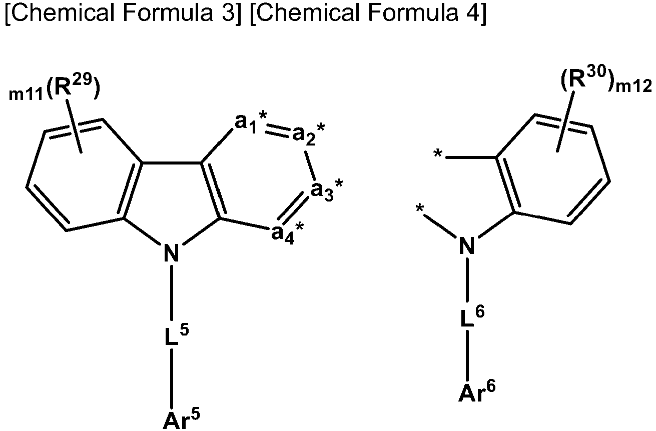
wherein, in Chemical Formula 3 and Chemical Formula 4,
among a1* to a4* in Chemical Formula 3, two adjacent ones are each a linking carbon (C) that is linked to *s in Chemical Formula 4,
among a₁* to a₄* of Chemical Formula 3, the remaining two that are not linked to Chemical Formula 4 are C-L^{a}-R^{a},
L^{a}, L⁵, and L⁶ are each independently a single bond or a substituted or unsubstituted C6 to C20 arylene group,
R^{a}, R²⁹, and R³⁰ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group, and
m11 and m12 are each independently one of integers of 1 to 4; 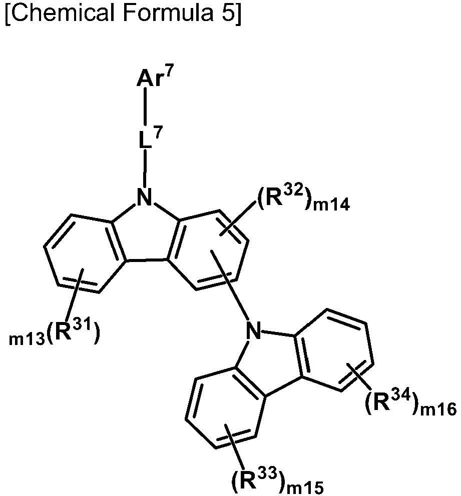
wherein, in Chemical Formula 5,
L⁷s are each independently a single bond or a substituted or unsubstituted C6 to C20 arylene group,
R³¹ to R³⁴ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁷ is a substituted or unsubstituted C6 to C20 aryl group or a substituted or unsubstituted C2 to C30 heterocyclic group,
m13, m15, and m16 are each independently one of integers of 1 to 4, and
m14 is one of integers of 1 to 3.

The second compound may be used in the light emitting layer together with the first compound to increase the mobility of charges and increase stability, thereby improving luminous efficiency and life-span characteristics.

In Chemical Formula 2, when m6 is 2 or more, each R²⁴ may be the same or different from each other.

In Chemical Formula 2, when m7 is 2 or more, each R²⁵ may be the same or different from each other.

In Chemical Formula 2, when m8 is 2 or more, each R²⁶ may be the same or different from each other.

In Chemical Formula 2, when m9 is 2 or more, each R²⁷ may be the same or different from each other.

In Chemical Formula 2, when m10 is 2 or more, each R²⁸ may be the same or different from each other.

In Chemical Formula 3 and Chemical Formula 4, when m11 is 2 or more, each R²⁹ may be the same or different from each other.

In Chemical Formula 3 and Chemical Formula 4, when m12 is 2 or more, each R³⁰ may be the same or different from each other.

In Chemical Formula 3 and Chemical Formula 4, when substituted with 2 or more R^{a}s, each R^{a} may be the same or different from each other.

In Chemical Formula 5, when m13 is 2 or more, each R³¹ may be the same or different from each other.

In Chemical Formula 5, when m14 is 2 or more, each R³² may be the same or different from each other.

In Chemical Formula 5, when m15 is 2 or more, each R³³ may be the same or different from each other.

In Chemical Formula 5, when m16 is 2 or more, each R³⁴ may be the same or different from each other.

As an example, in Chemical Formula 2, Ar³ and Ar⁴ may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group,
in Chemical Formula 2, L³ and L⁴ may each independently be a single bond, a substituted or unsubstituted phenylene group, or a substituted or unsubstituted biphenylene group,
in Chemical Formula 2, R²⁴ to R²⁸ may each independently be hydrogen, deuterium, or a substituted or unsubstituted C6 to C12 aryl group, and
n may be 0 or 1.

As an example, in Chemical Formula 2, "substituted" means that at least one hydrogen is substituted with deuterium, a C1 to C4 alkyl group, a C6 to C18 aryl group, or a C2 to C30 heteroaryl group.

For example, in Chemical Formula 2, Ar³ and Ar⁴ may each independently be a substituted or unsubstituted carbazolyl group, a substituted or unsubstituted dibenzothiophenyl group, a substituted or unsubstituted dibenzofuranyl group, or a substituted or unsubstituted fluorenyl group.

In another specific embodiment of the present invention, Chemical Formula 2 may be represented by one of Chemical Formula 2-1 to Chemical Formula 2-15.

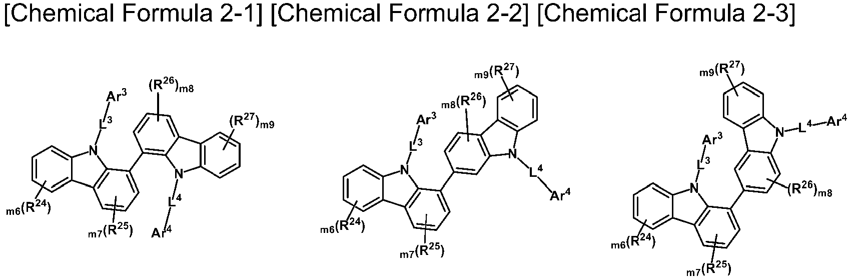

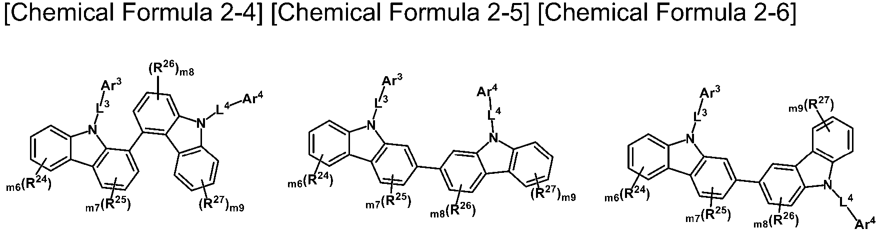

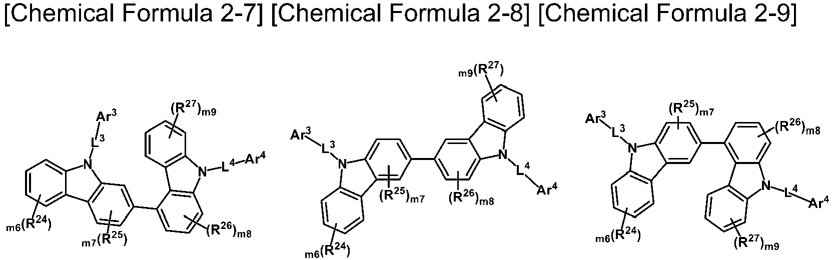

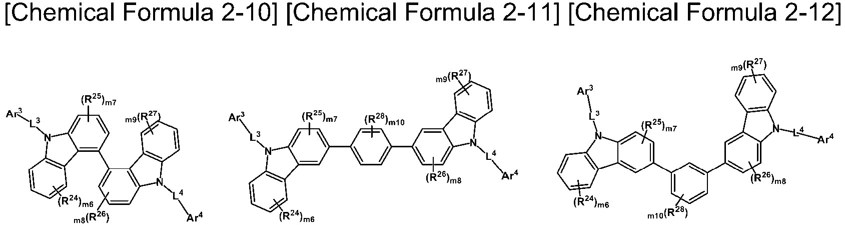

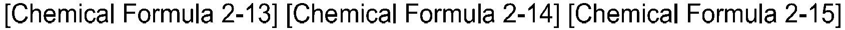

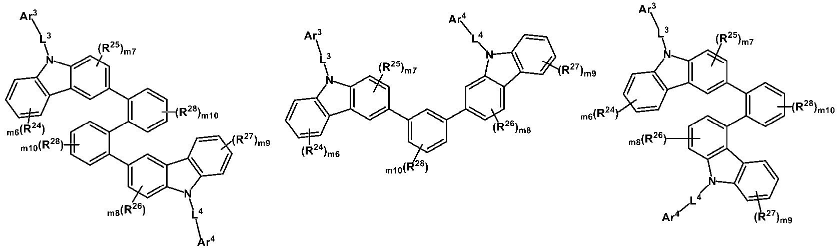

In Chemical Formula 2-1 to Chemical Formula 2-15, R²⁴ to R²⁸ may each independently be hydrogen, deuterium, or a substituted or unsubstituted C6 to C12 aryl group, and L³-Ar³ and L⁴-Ar⁴ may each independently be one of substituents listed in Group II.

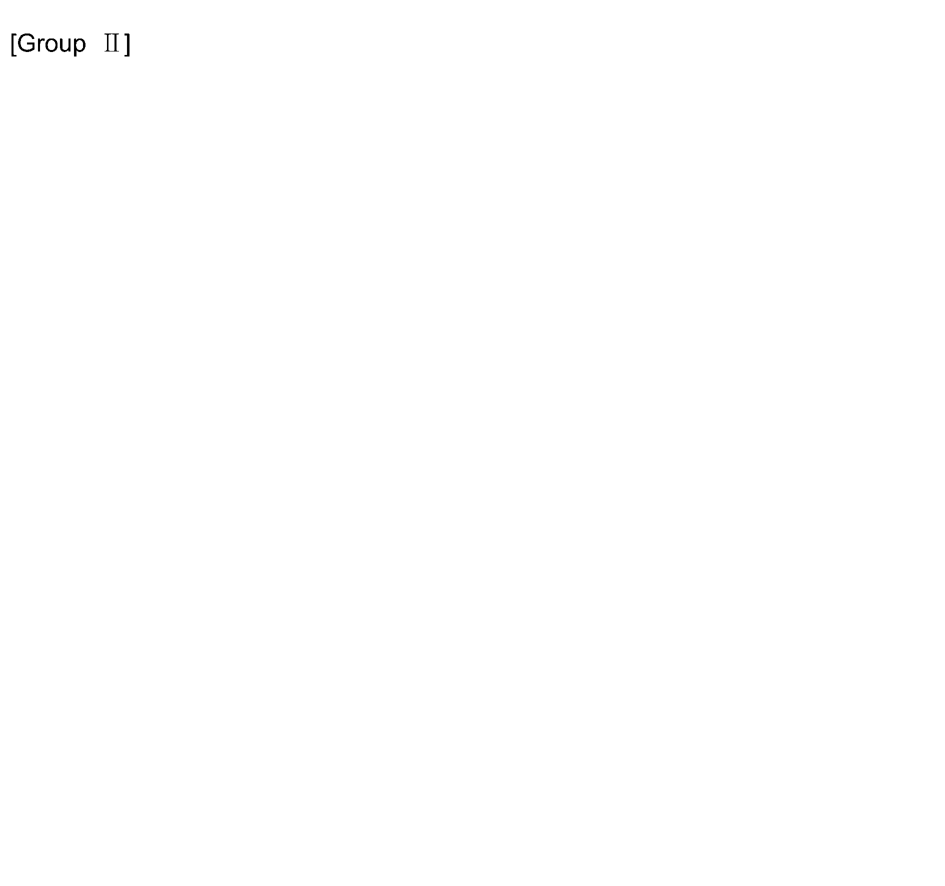

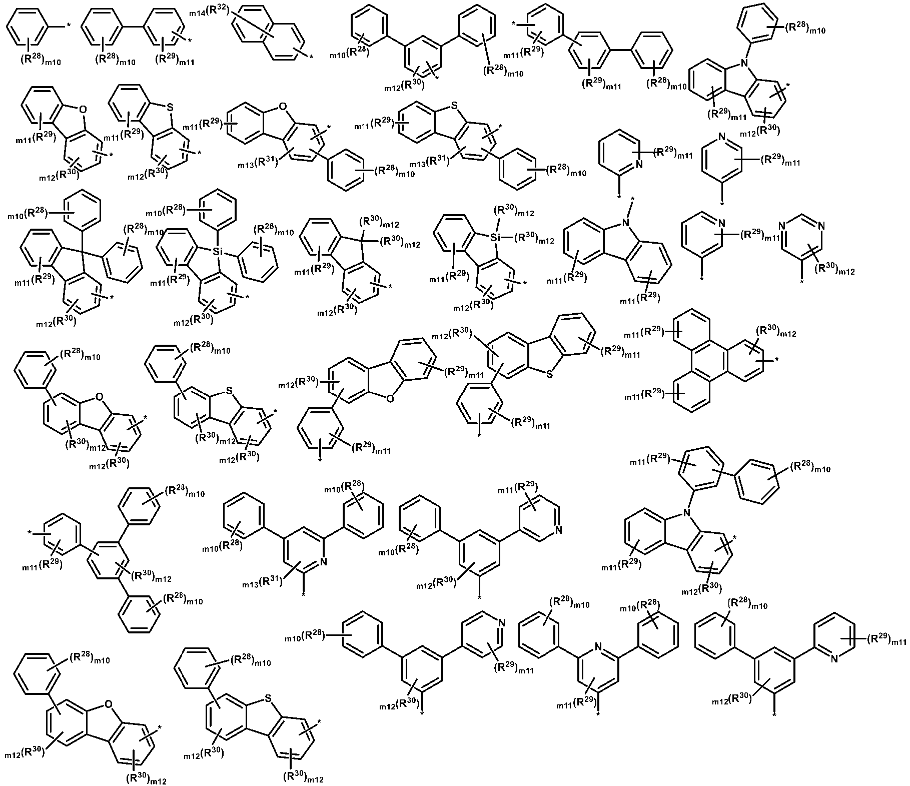

In Group II,
R²⁸ to R³² are each independently hydrogen, deuterium, a cyano group, a C1 to C10 alkyl group, or a C6 to C12 aryl group,
m10 is one of integers of 1 to 5,
m11 is one of integers of 1 to 4,
m12 is one of integers of 1 to 3,
m13 is an integer of 1 or 2,
m14 is one of integers of 1 to 7, and
* is a linking point.

In Group II, when m10 is 2 or more, each R²⁸ may be the same or different from each other.

In Group II, when m11 is 2 or more, each R²⁹ may be the same or different from each other.

In Group II, when m12 is 2 or more, each R³⁰ may be the same or different from each other.

In Group II, when m13 is 2, each R³¹ may be the same or different from each other.

In Group II, when m14 is 2 or more, each R³² may be the same or different from each other.

The combination of Chemical Formula 3 and Chemical Formula 4 may be represented by, for example, any one of Chemical Formula 3A, Chemical Formula 3B, Chemical Formula 3C, Chemical Formula 3D, and Chemical Formula 3E.

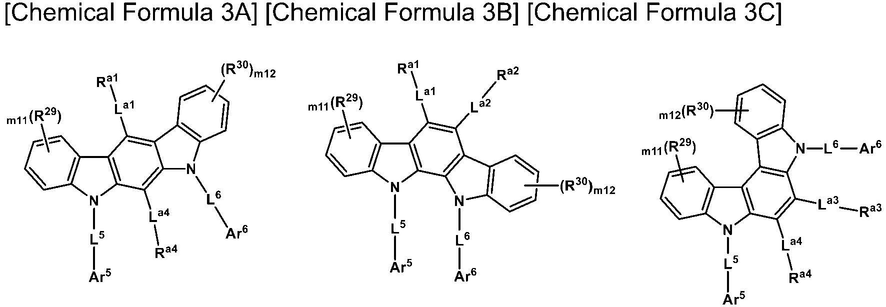

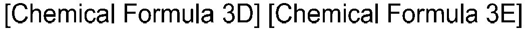

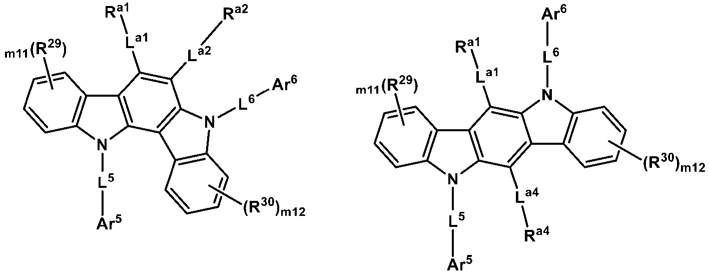

In Chemical Formula 3A to Chemical Formula 3E, L⁵, L⁶, Ar⁵, Ar⁶, R²⁹, R³⁰, m11 and m12 are the same as described above,
L^{a1} to L^{a4} are as defined for L⁵ and L⁶ above, and
R^{a1} to R^{a4} are as defined for R²⁹ and R³⁰ above.

For example, in Chemical Formulas 3 and 4, Ar⁵ and Ar⁶ may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group, and

R^{a1} to R^{a4}, R²⁹, and R³⁰ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In another specific embodiment of the present invention, in Chemical Formulas 3 and 4, L⁵-Ar⁵ and L⁶-Ar⁶ may each independently be selected from the substituents listed in Group I .

In an embodiment, R^{a1} to R^{a4}, R²⁹, and R³⁰ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, R^{a1} to R^{a4}, R²⁹, and R³⁰ may each independently be hydrogen, deuterium, a cyano group, or a substituted or unsubstituted phenyl group, and
in a specific embodiment, R^{a1} to R^{a4}, R²⁹, and R³⁰ may each independently be hydrogen, deuterium, or a substituted or unsubstituted phenyl group.

In a specific embodiment of the present invention, the second compound may be represented by the Chemical Formula 2-8, and in Chemical Formula 2-8, Ar³ and Ar⁴ may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group, L³ and L⁴ may each independently be a single bond, or a substituted or unsubstituted C6 to C20 arylene group, and R²⁴ to R²⁸ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, in Chemical Formula 2-8, R²⁴ to R²⁷ may each independently be hydrogen, deuterium, or a substituted or unsubstituted C6 to C12 aryl group, and L³-Ar³ and L⁴-Ar⁴ may each independently be one of the substituents listed in Group II.

In another specific embodiment of the present invention, the second compound may be represented by Chemical Formula 3C, and in Chemical Formula 3C, L^{a3} and L^{a4} may be a single bond, L⁵ and L⁶ may each independently be a single bond or a substituted or unsubstituted C6 to C12 arylene group, R²⁹, R³⁰, R^{a3}, and R^{a4} may each be hydrogen, deuterium, or a phenyl group, and Ar⁵ and Ar⁶ may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, in Chemical Formula 3C, L^{a3} and L^{a4} may be a single bond, R²⁹, R³⁰, ^{Ra3}, and R^{a4} may each independently be hydrogen, deuterium, or a C6 to C12 aryl group, and L⁵-Ar⁵ and L⁶-Ar⁶ may each independently be one of the substituents listed in Group II.

Chemical Formula 5 may be represented by, for example, any one of Chemical Formula 5-1 to Chemical Formula 5-4.

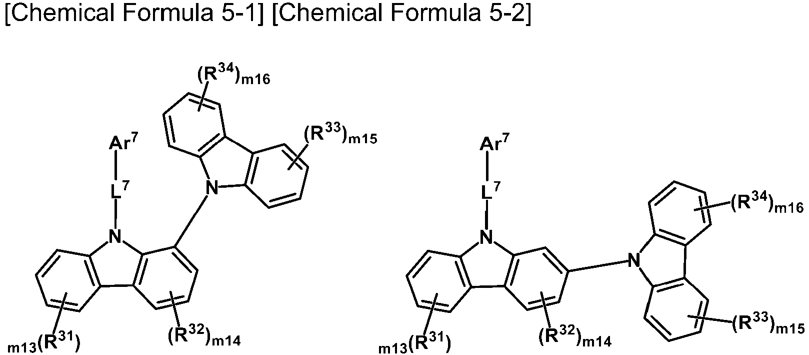

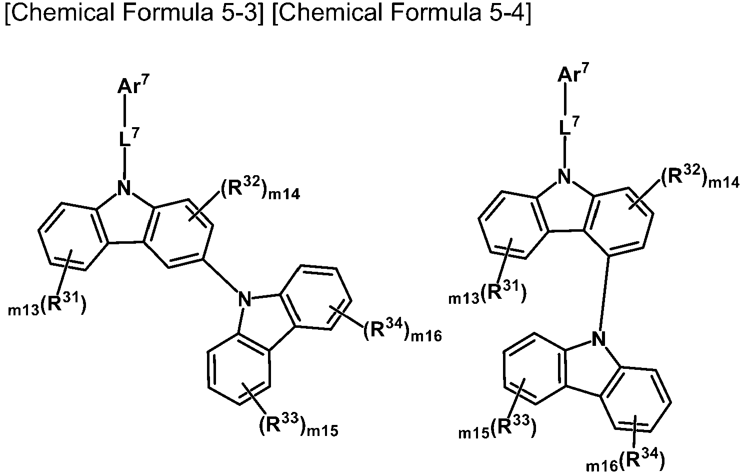

In Chemical Formula 5-1 to Chemical Formula 5-4, L⁷, Ar⁷, R³¹ to R³⁴, and m13 to m16 are as described above.

For example, Ar⁷ may be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group, and

R³¹ to R³⁴ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In another specific embodiment of the present invention, in Chemical Formula 5, L⁷-Ar⁷ may be selected from the substituents listed in Group II.

For example, R³¹ to R³⁴ may each independently be hydrogen, deuterium, a cyano group, or a substituted or unsubstituted phenyl group.

For example, the second compound for the organic optoelectronic device may be, for example, one selected from compounds listed in Group 2 below, but is not limited thereto.

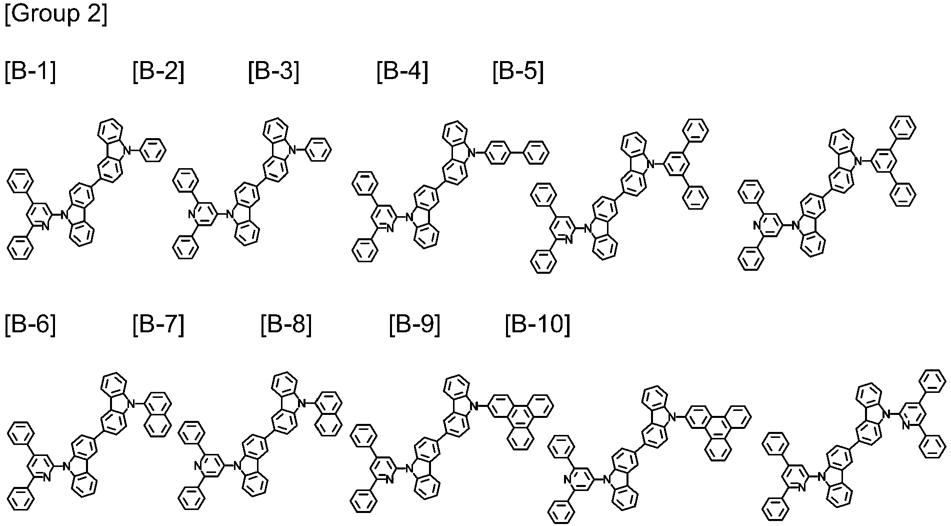

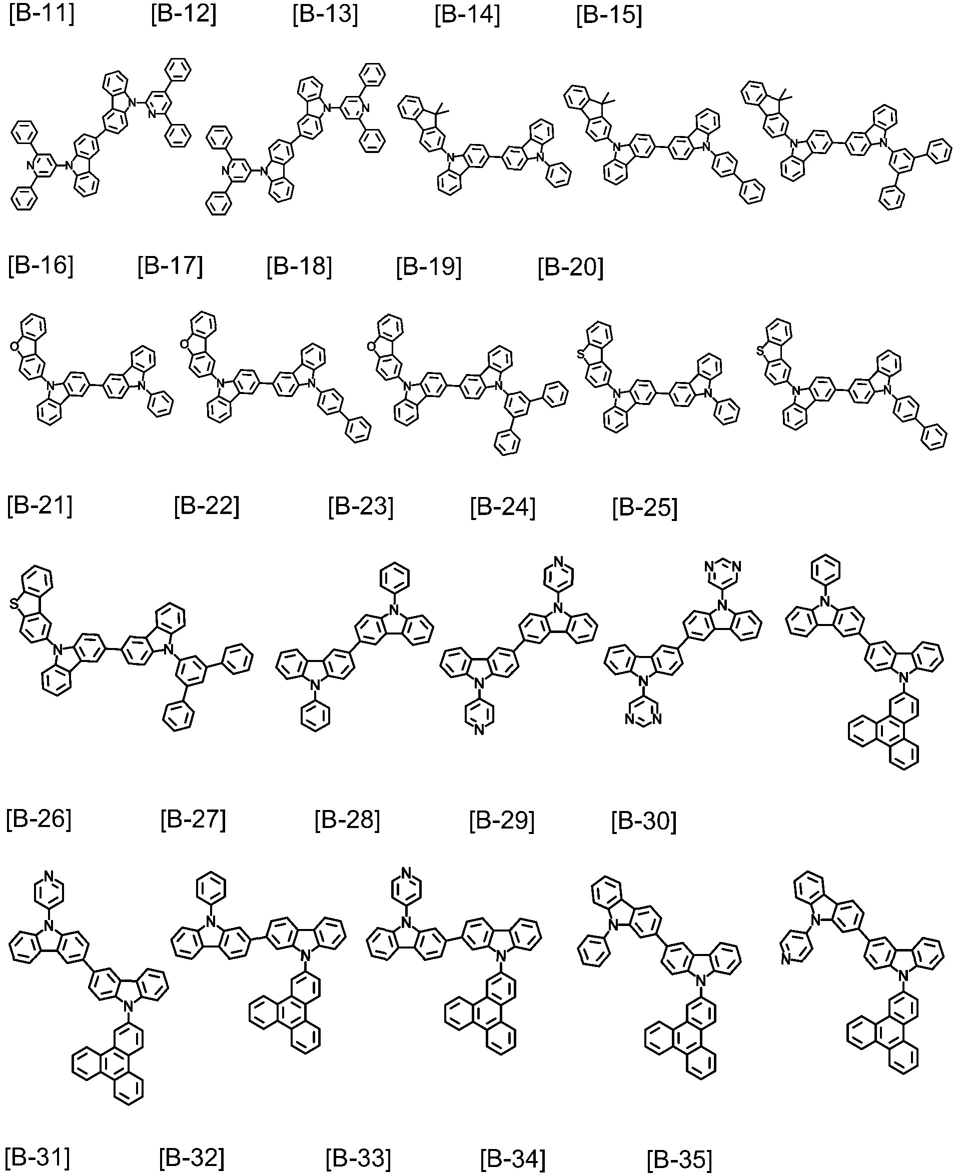

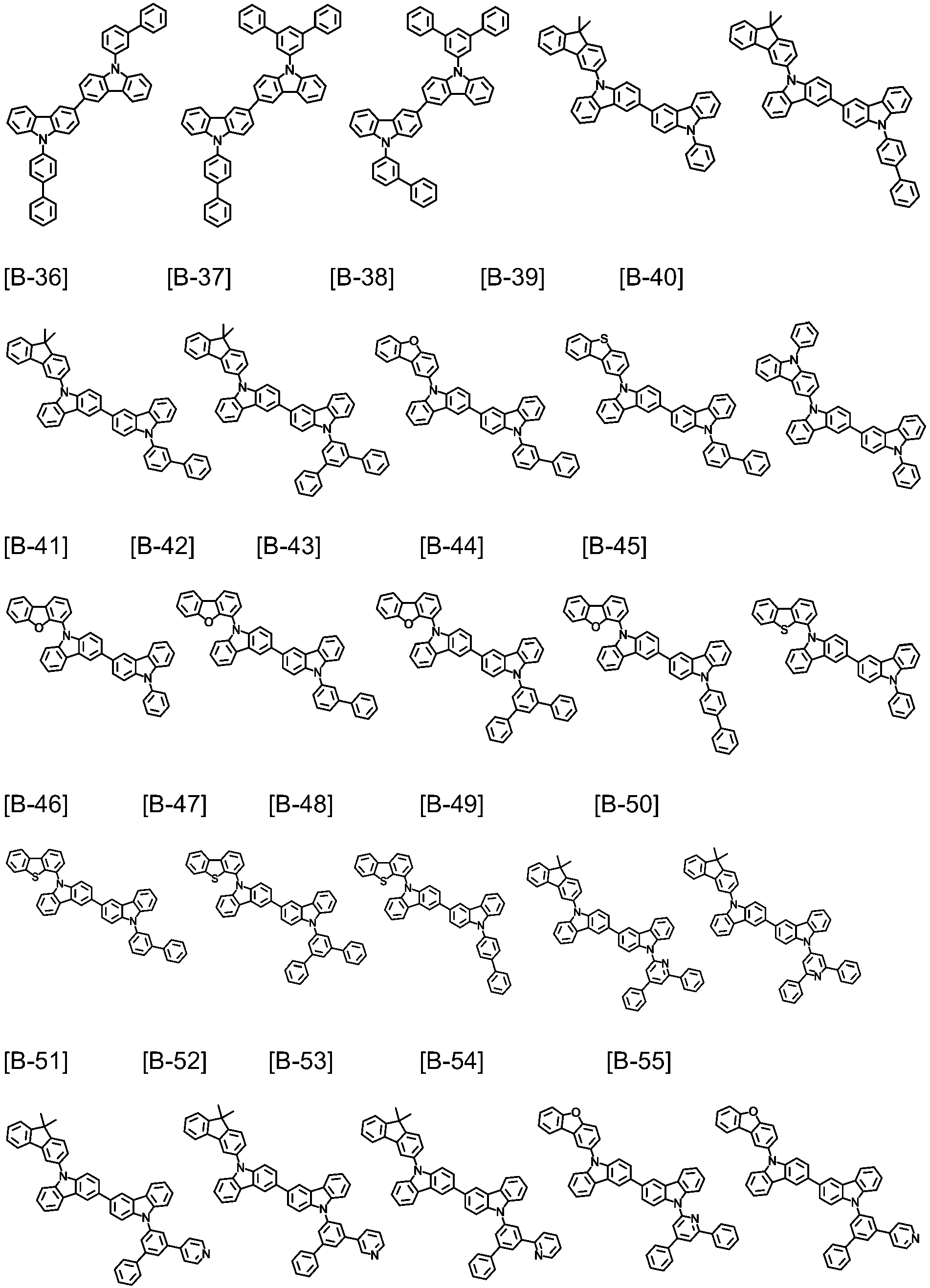

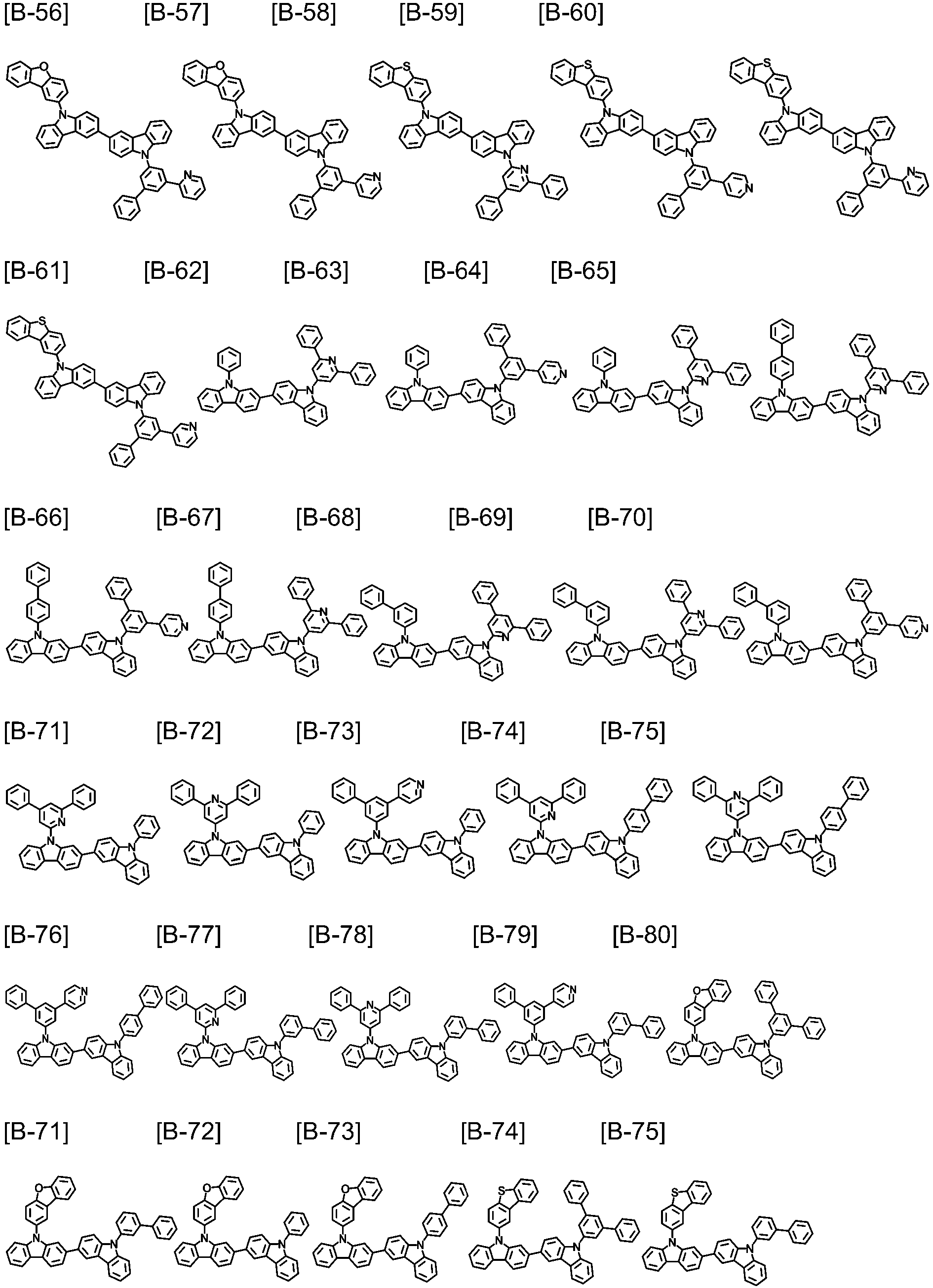

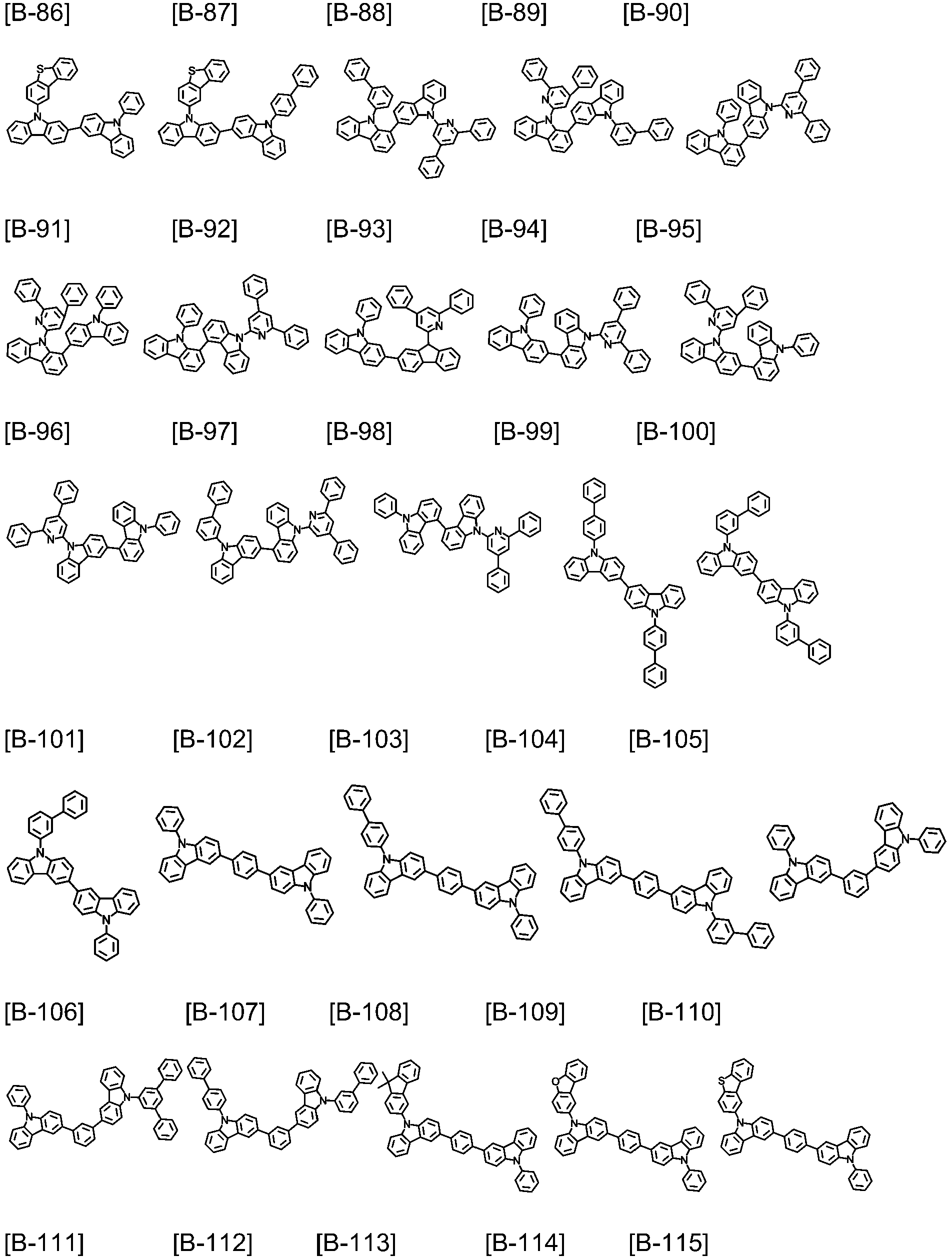

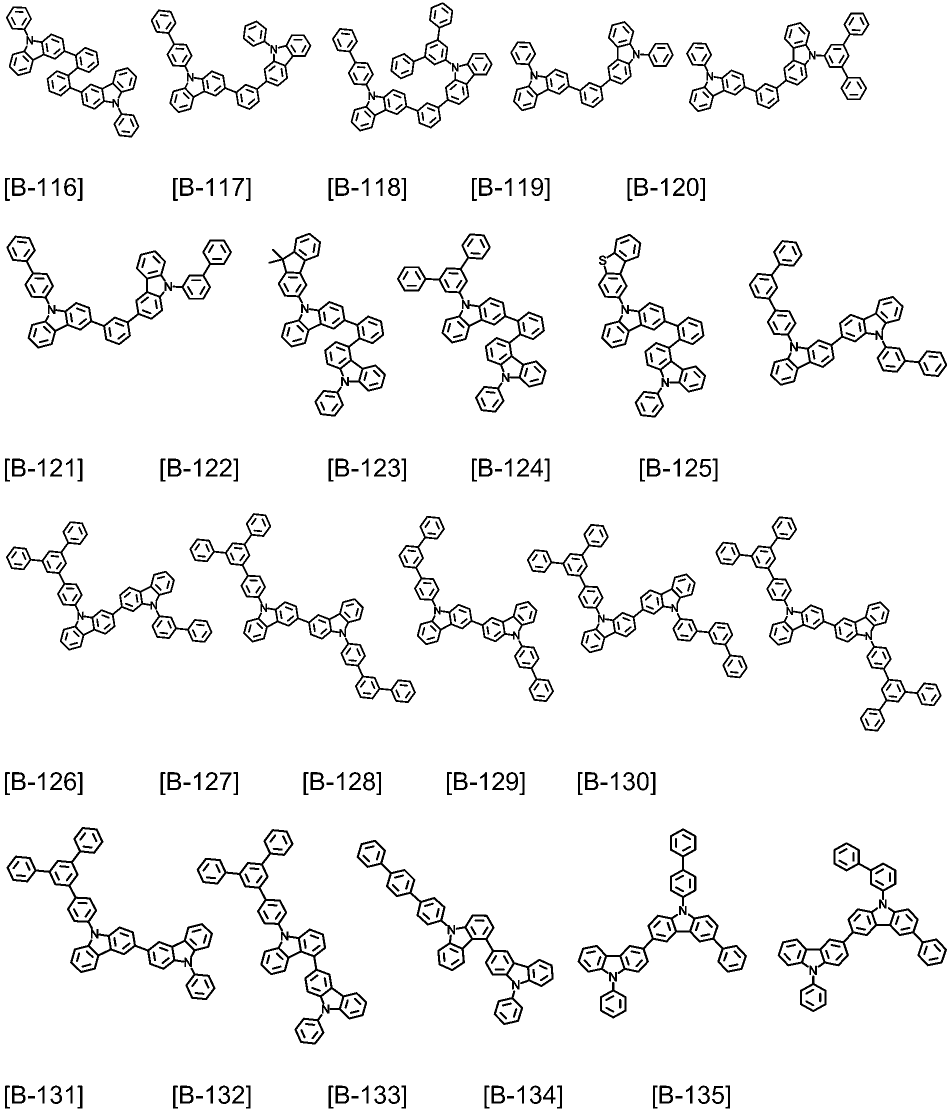

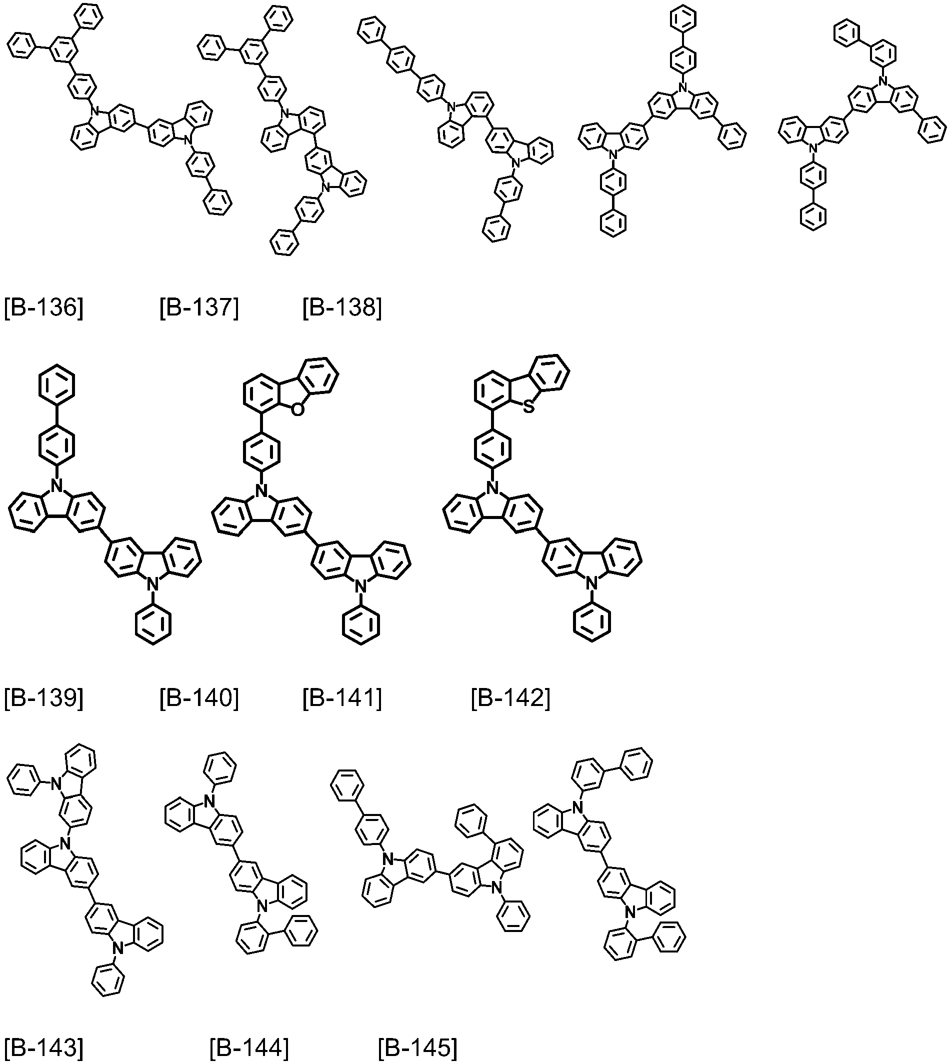

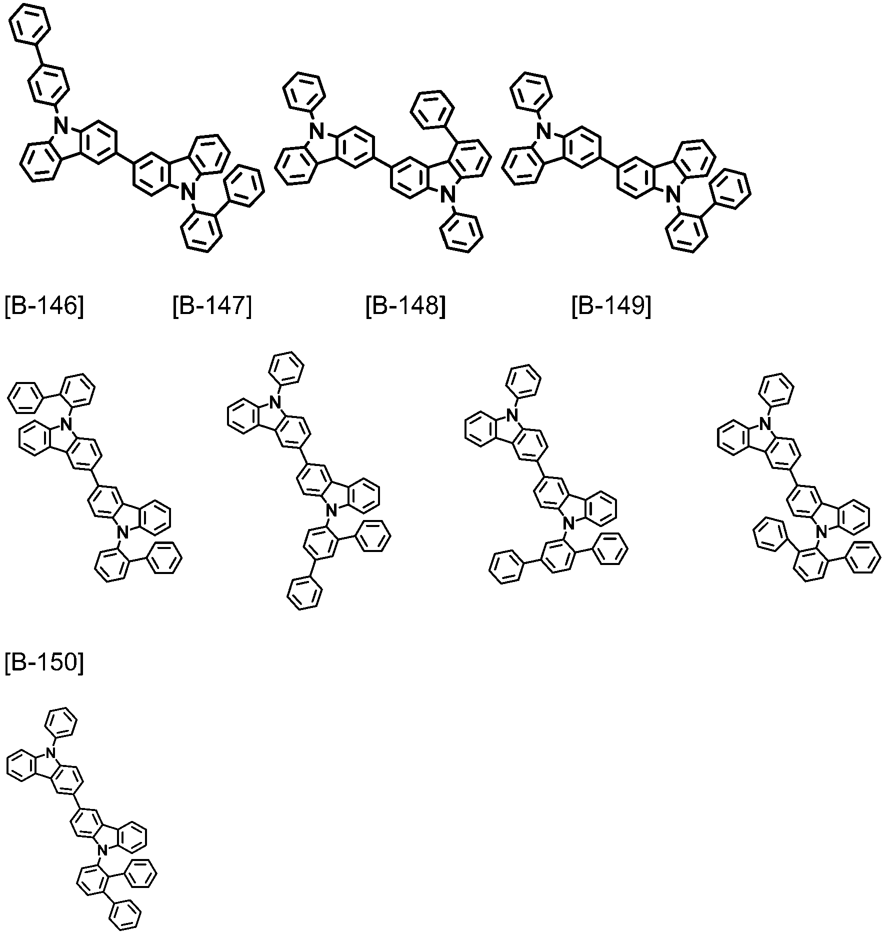

In addition, an example of a form in which at least one hydrogen is substituted with deuterium in the compounds B-1 to B-150 listed in Group 2 is presented below, but is not limited thereto. 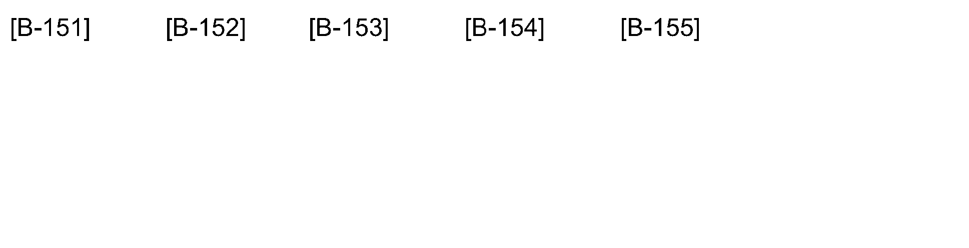 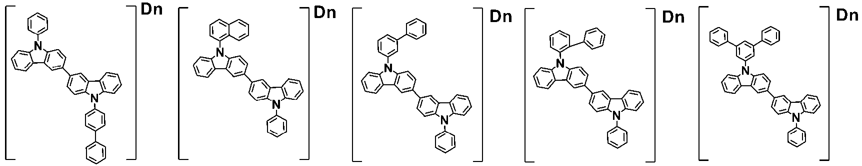 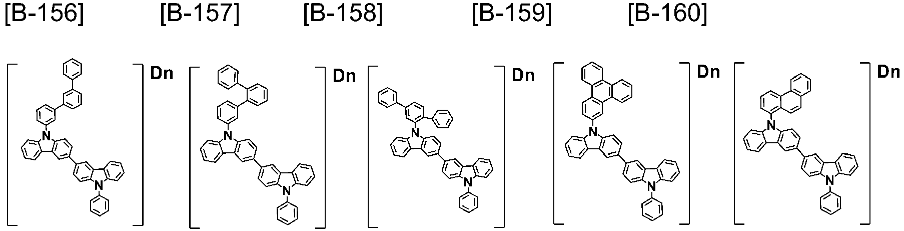 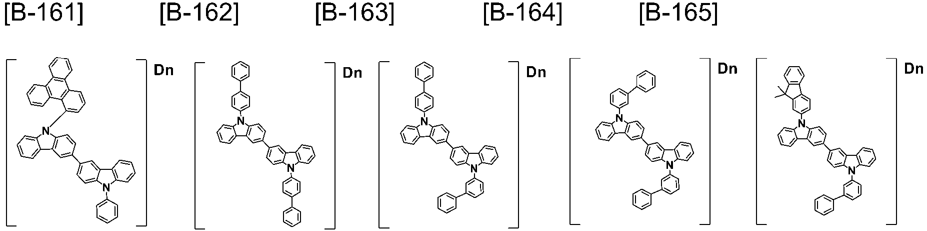 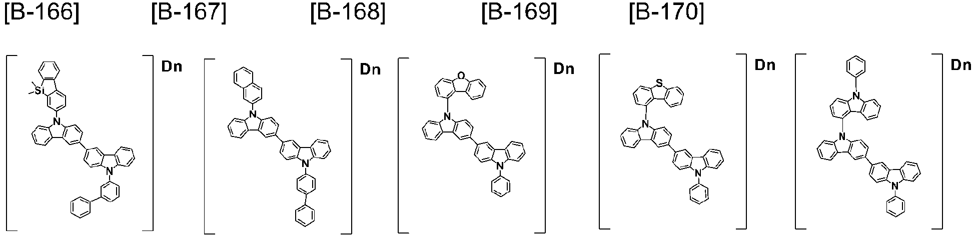 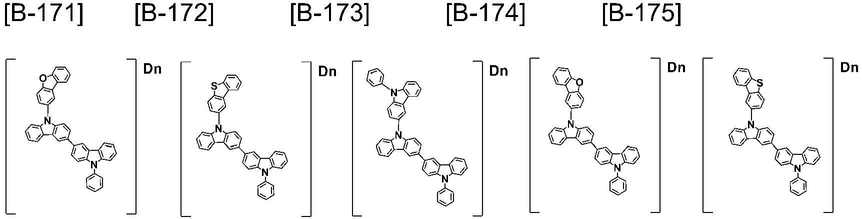 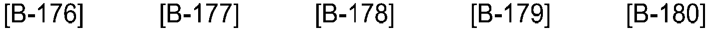 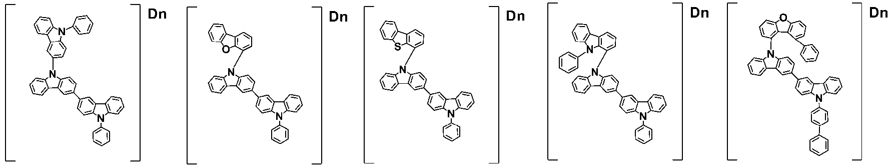 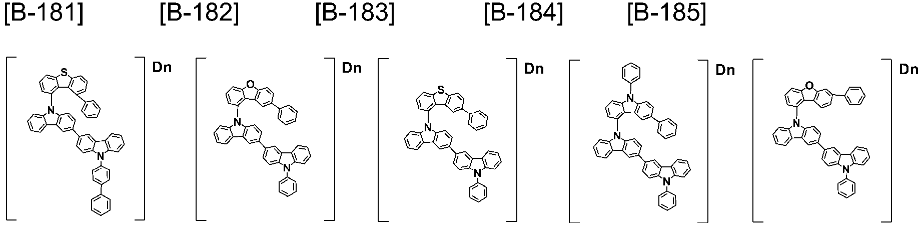 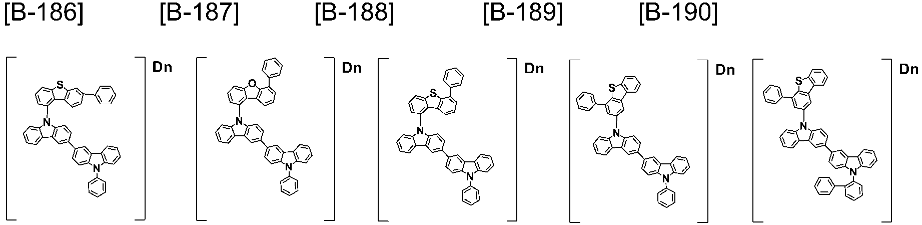 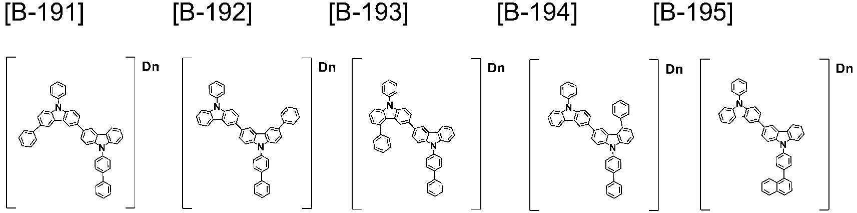 (Dn refers to the number of deuterium substitutions and indicates a structure substituted with one or more deuteriums)

With respect to compounds B-151 to B-195 of Group 2, only the most specific structures according to the deuterium substitution position and substitution rate are exemplarily presented below, and there is no intention to limit the scope of rights for compounds not presented below.

The scope of the present invention is determined by the claims, and when deuterium is substituted, it is not limited to the compounds exemplified below, and the position of deuterium substitution and the substitution degree of deuterium substitution may include all changeable ranges within the range of Compound B-1 to Compound B-195.

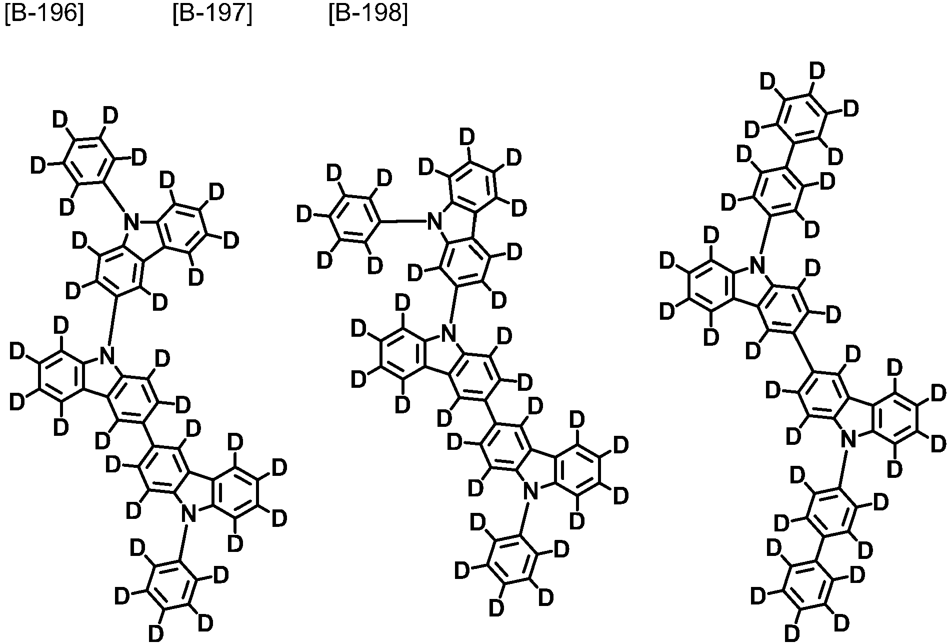

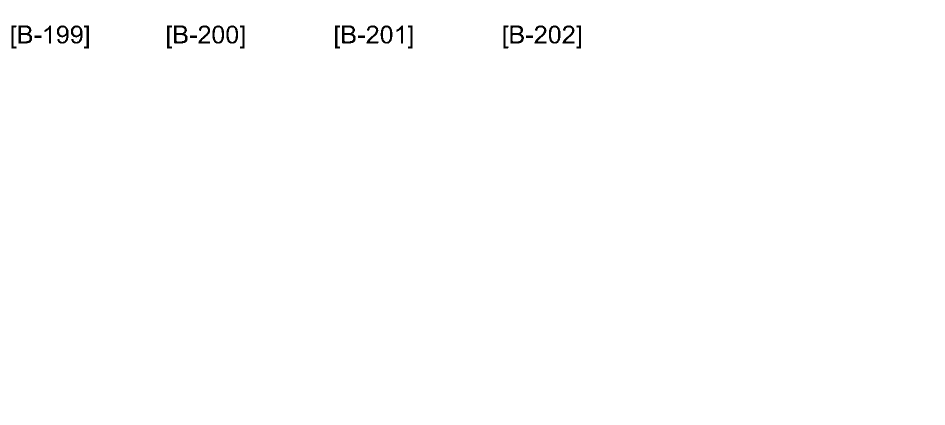

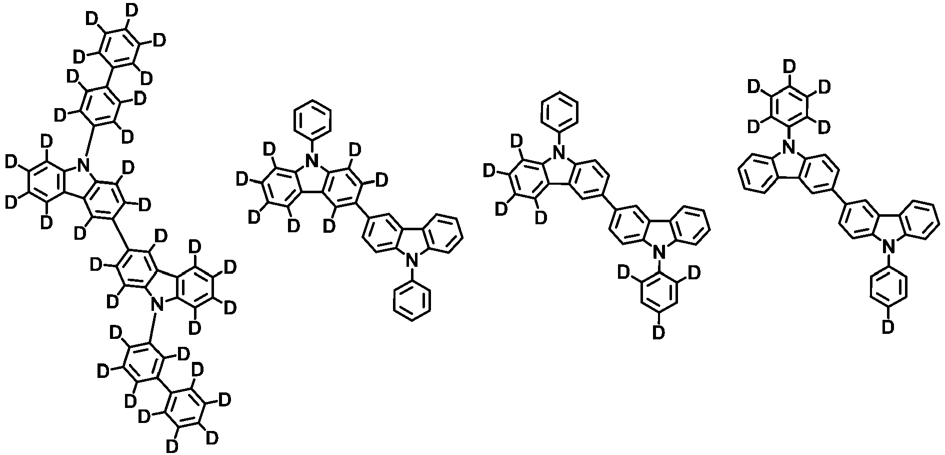

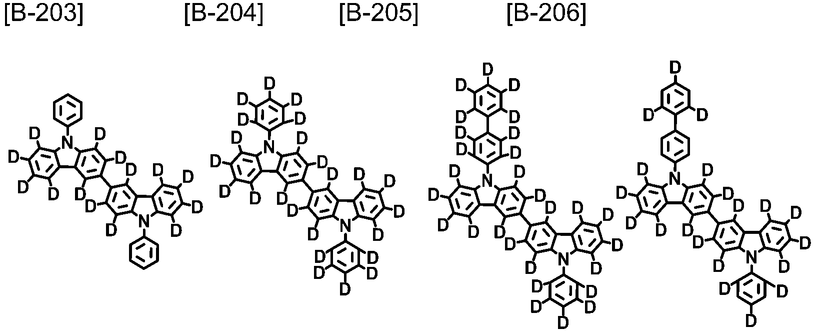

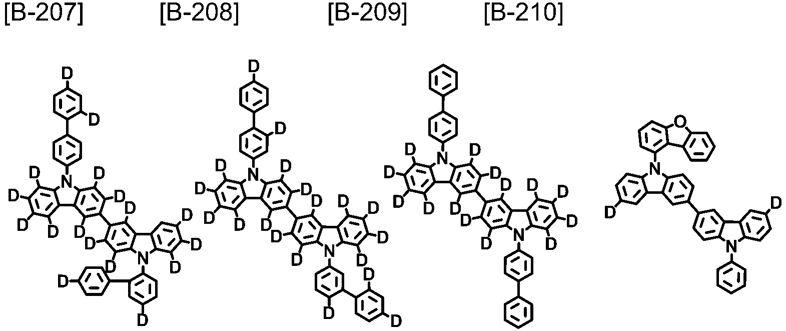

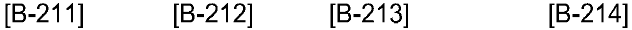

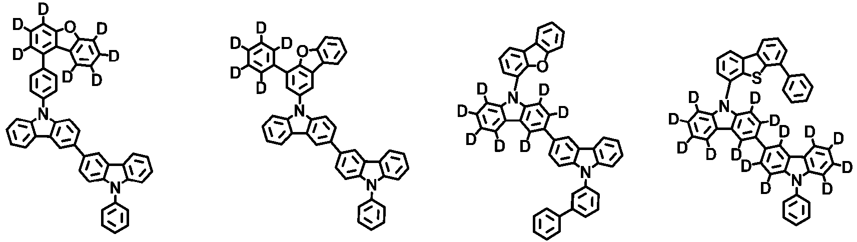

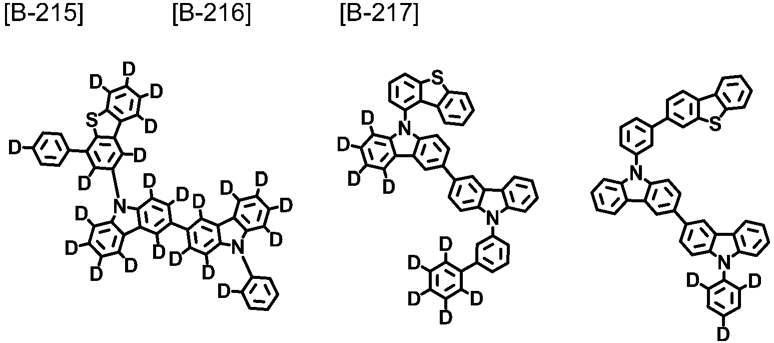

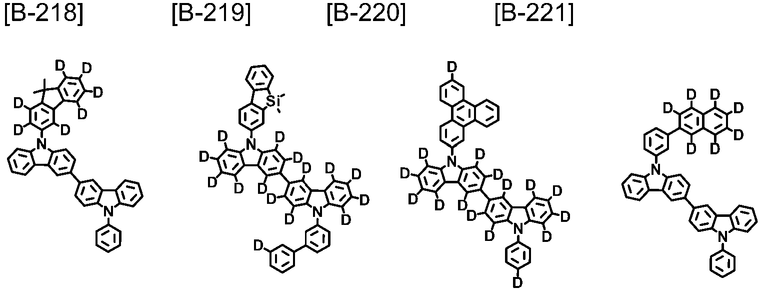

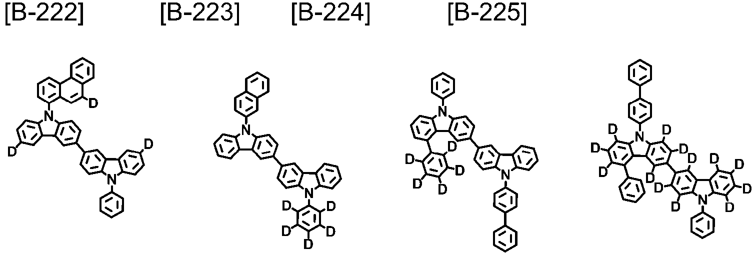

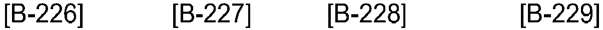

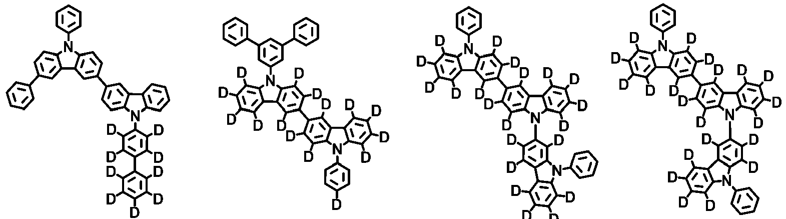

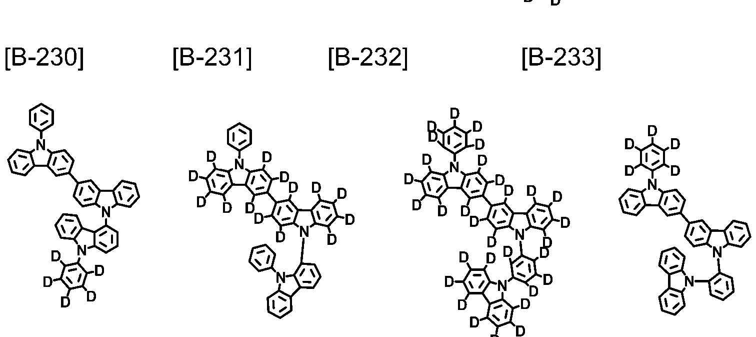

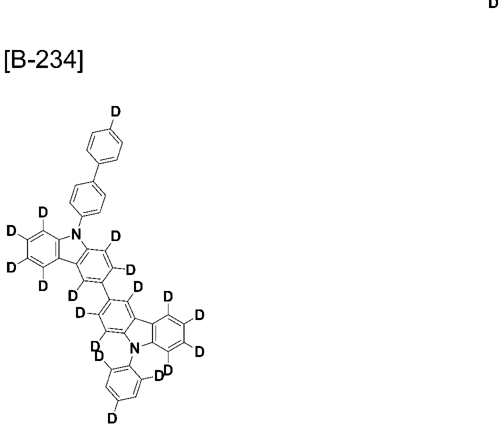

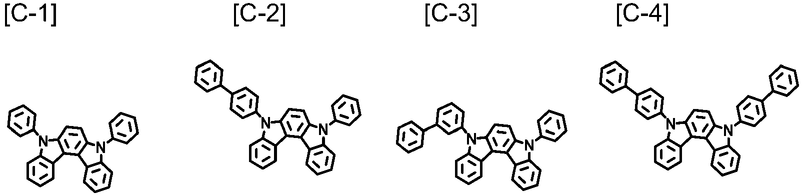

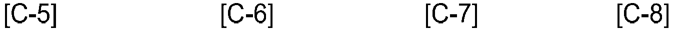

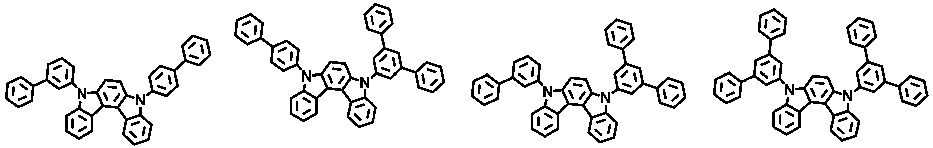

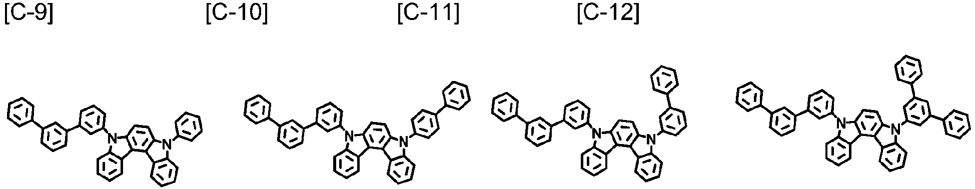

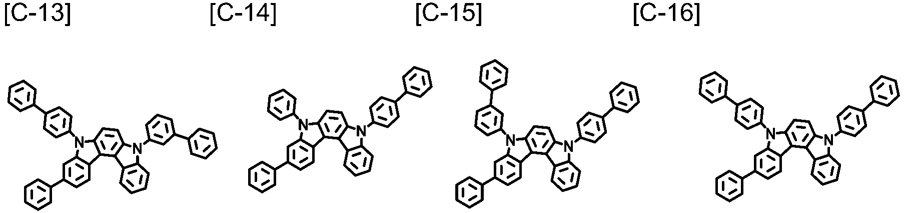

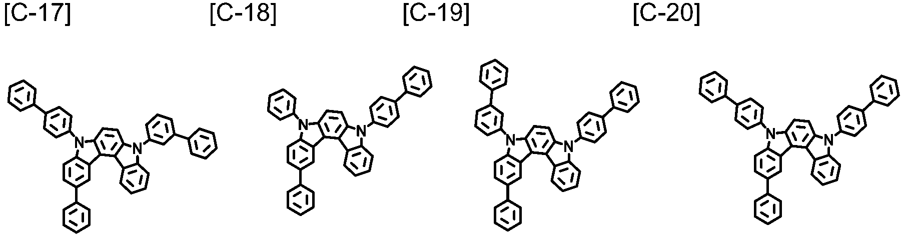

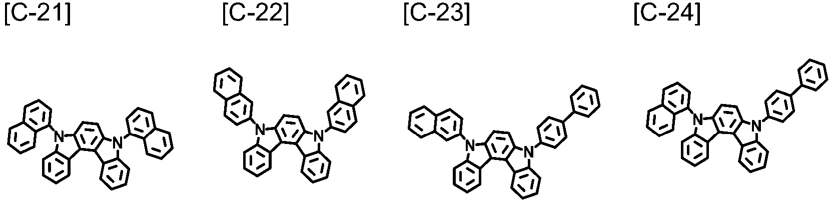

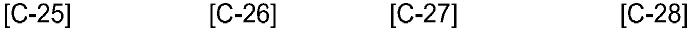

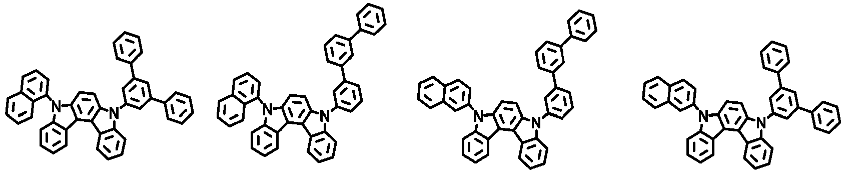

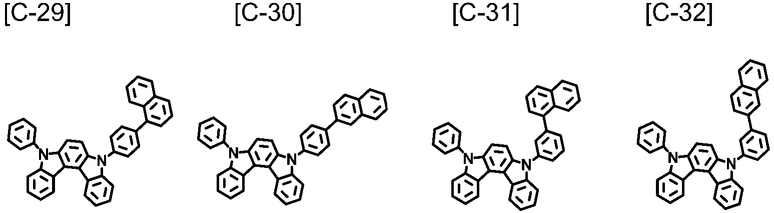

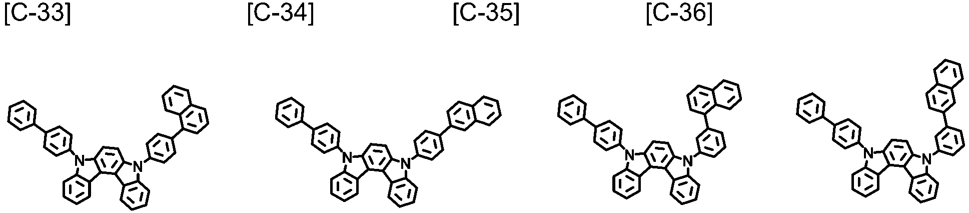

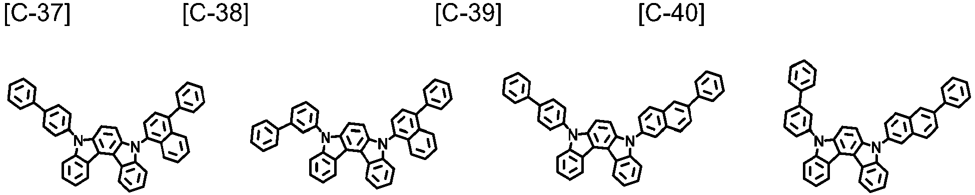

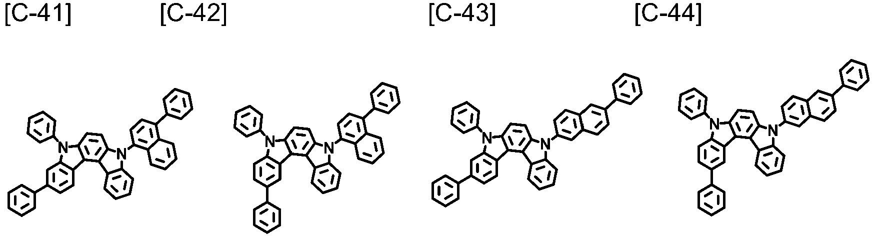

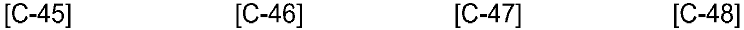

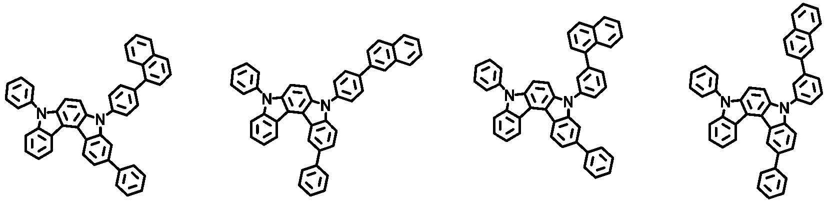

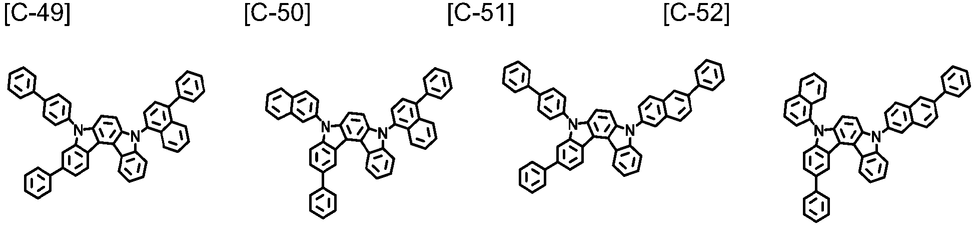

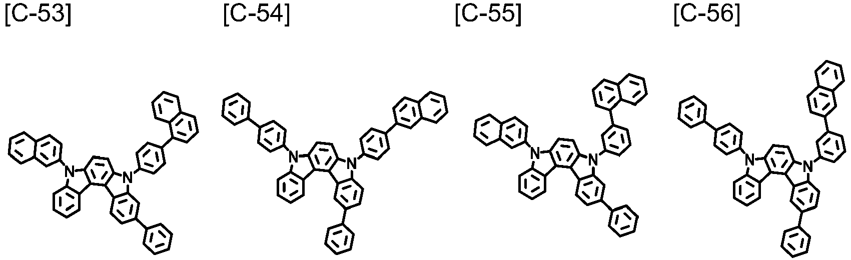

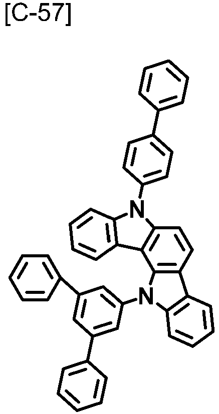

In addition, an example of a form substituted with deuterium for at least one hydrogen in Compound C-1 to Compound C-57 listed in the Group 2 is presented below, but is not limited thereto. 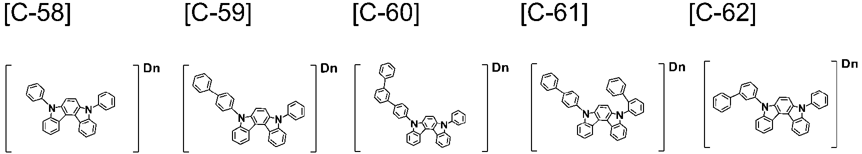 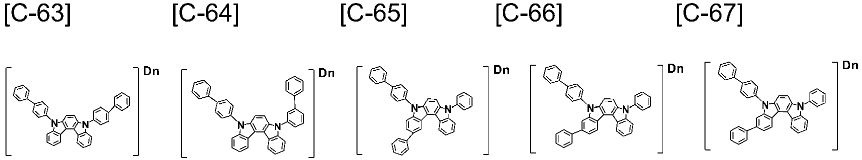 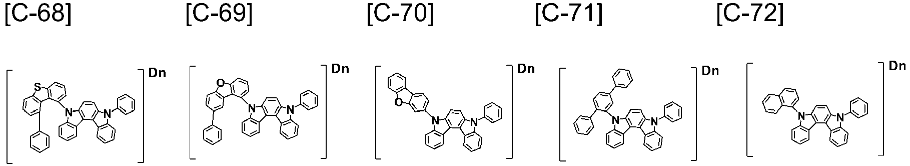 (Dn refers to the number of deuterium substitutions and indicates a structure substituted with one or more deuteriums)

The most specific structures for Compound C-58 to Compound C-72 of Group 2 are presented below as examples according to the position and substitution degree of deuterium substitution, which is not to limit the scope of rights to compounds not shown below.

The scope of the present invention is determined by the claims, and when deuterium is substituted, it is not limited to the compounds exemplified below, and the position of deuterium substitution and the substitution degree of deuterium substitution may include all changeable ranges within the range of Compound C-1 to Compound C-72.

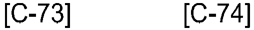

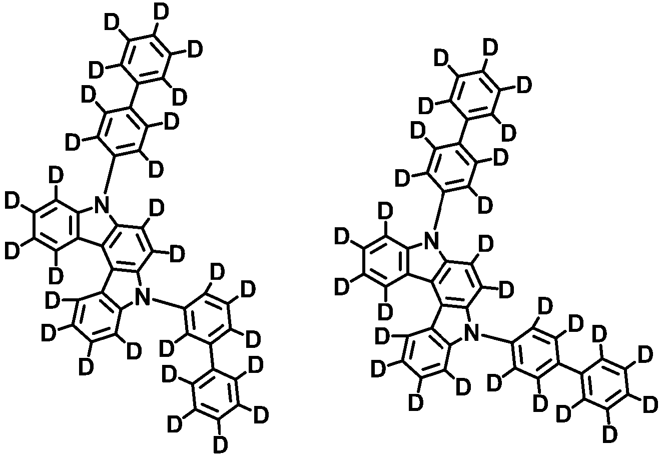

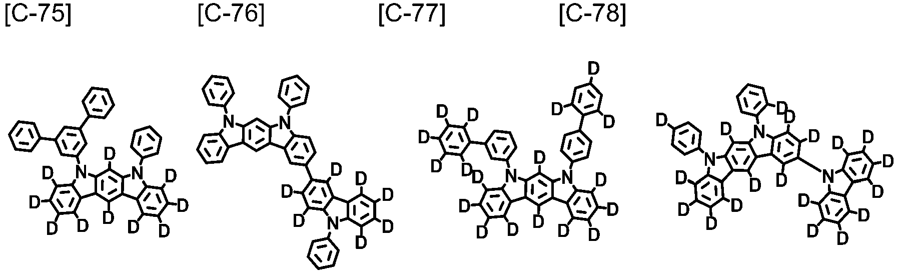

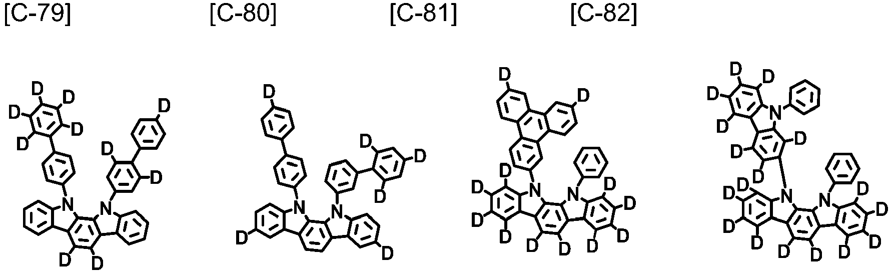

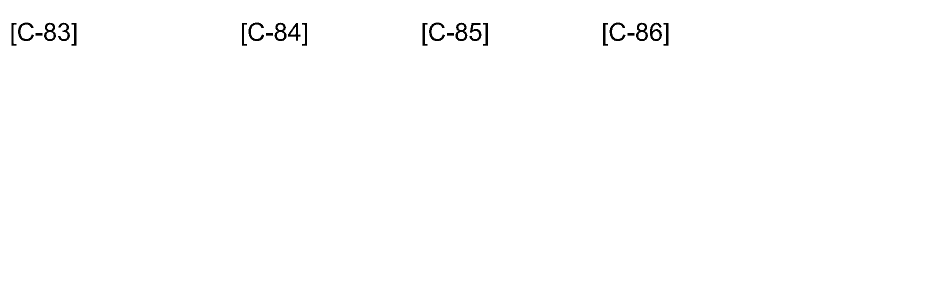

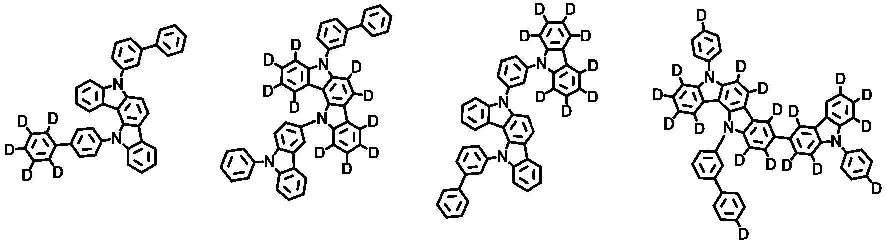

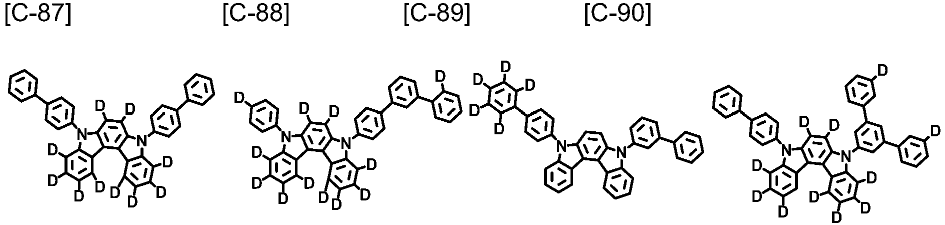

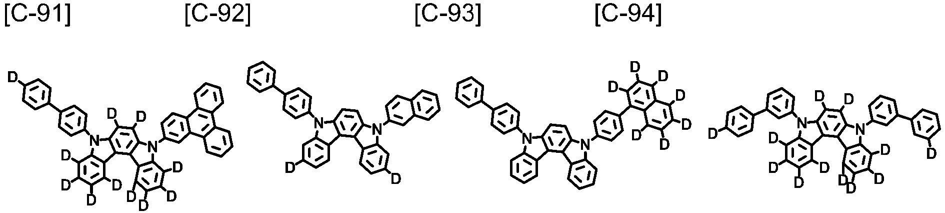

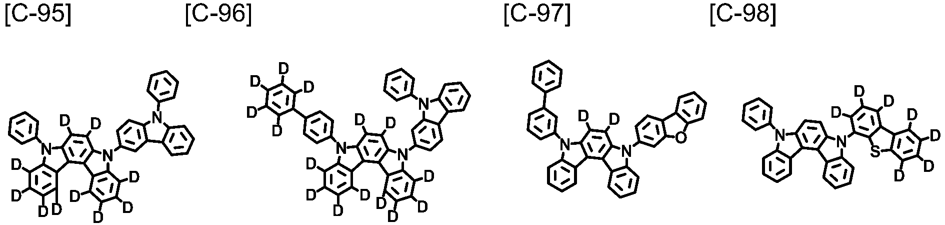

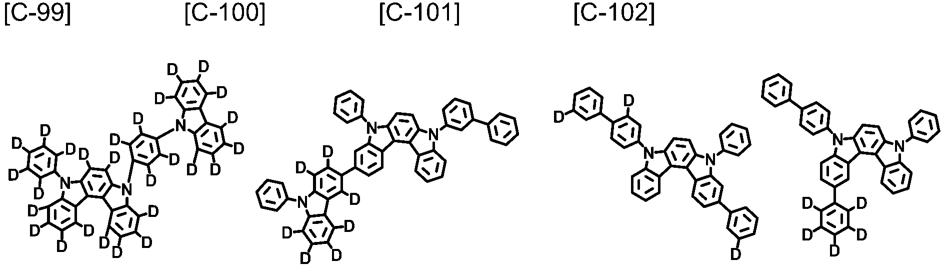

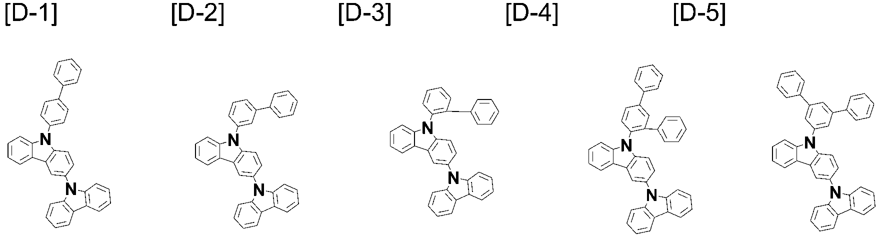

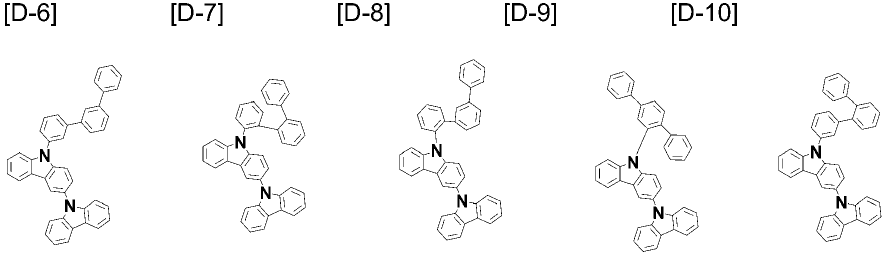

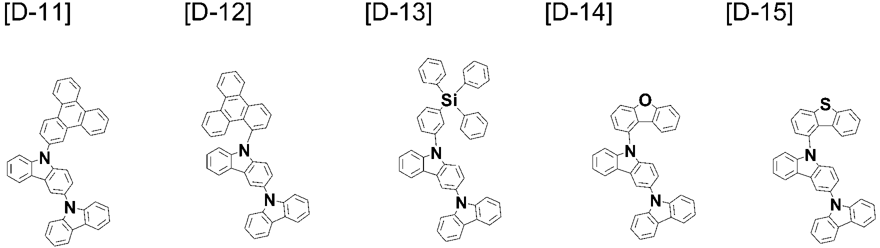

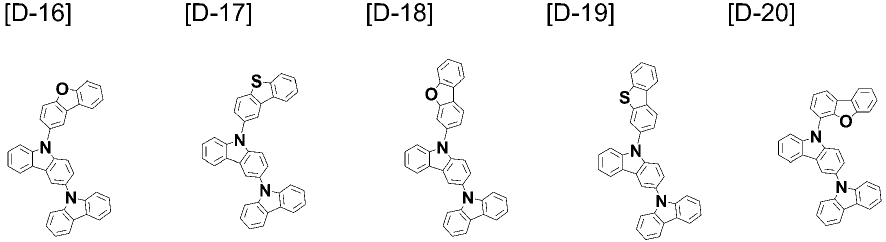

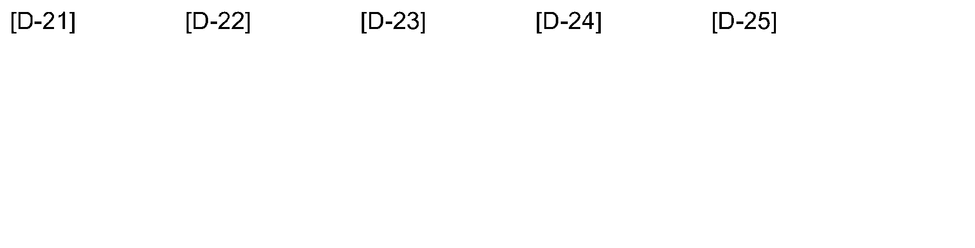

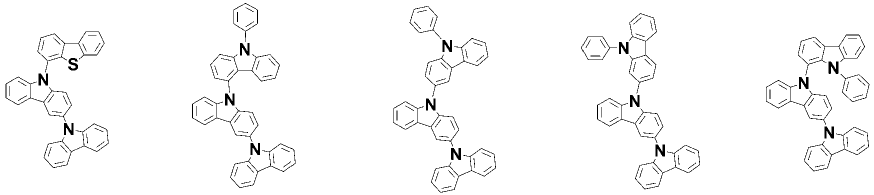

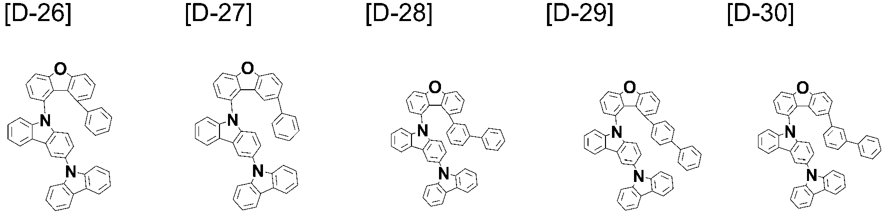

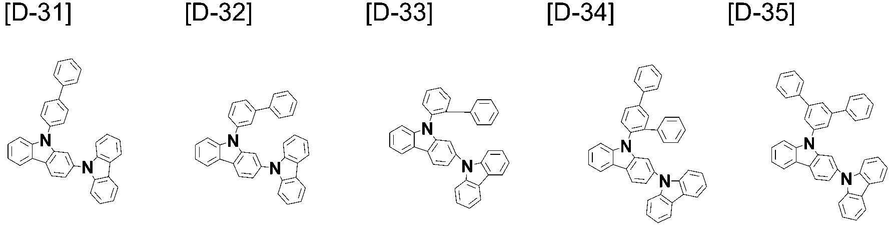

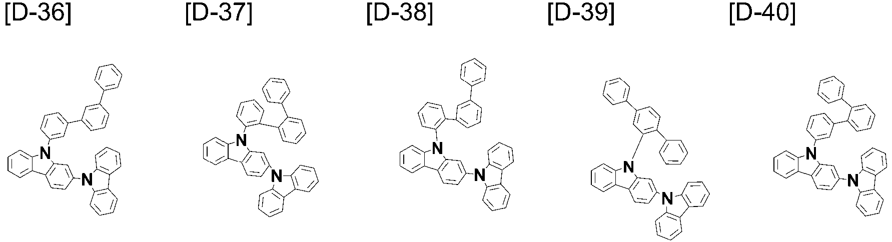

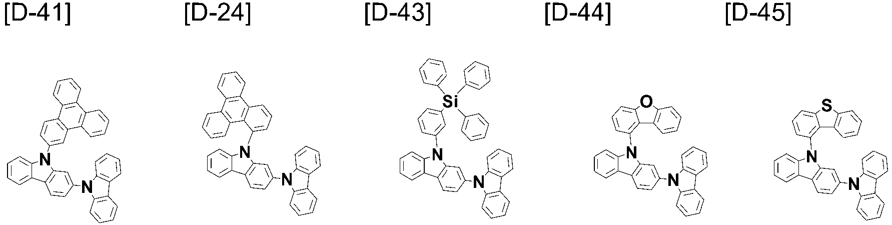

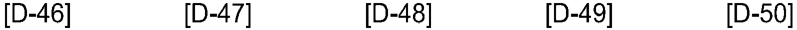

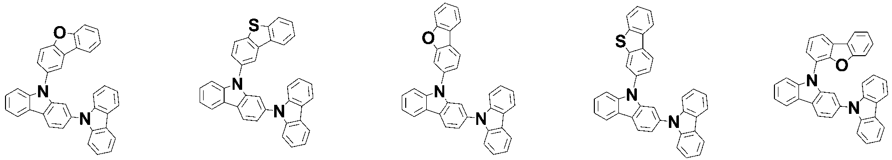

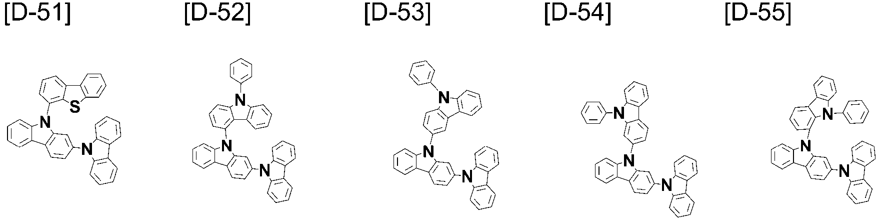

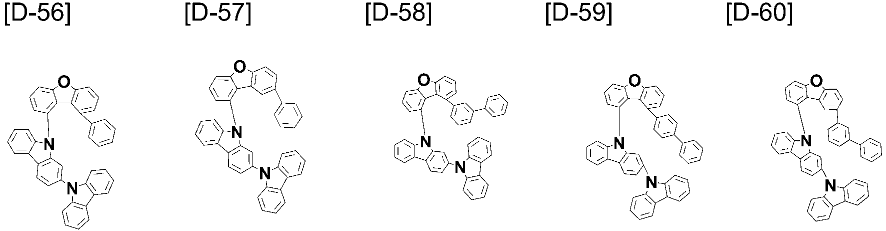

In addition, an example of a form in which at least one hydrogen is substituted with deuterium in the Compound D-1 to Compound D-60 listed in Group 2 is presented below, but is not limited thereto. 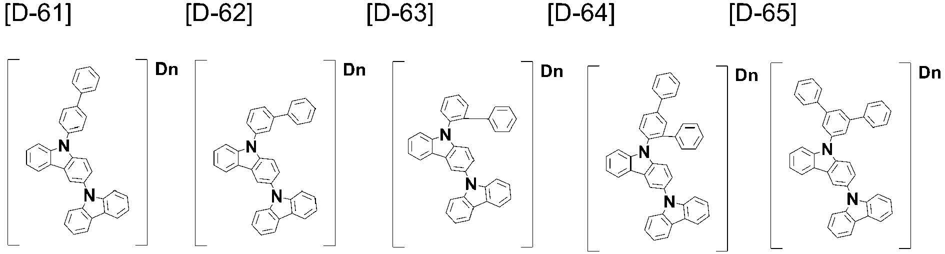 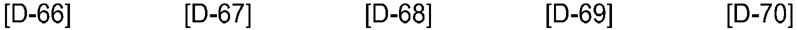 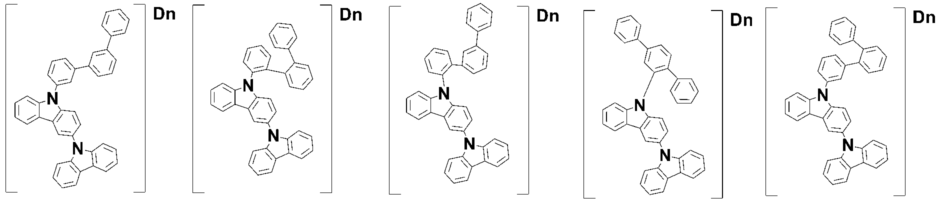 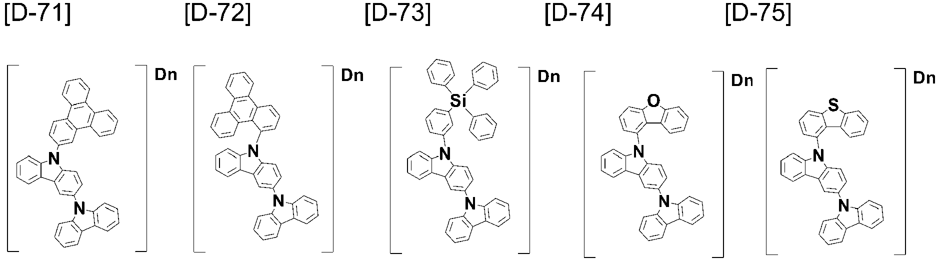 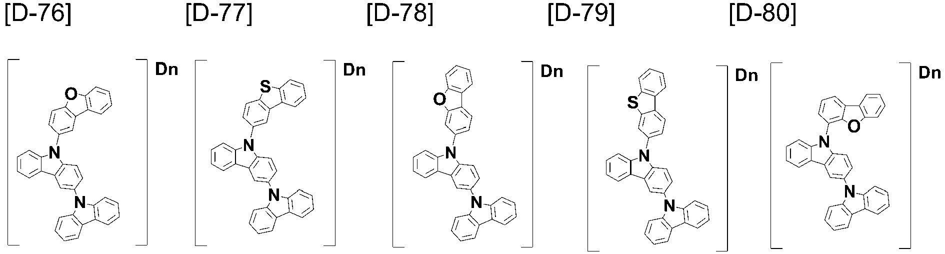 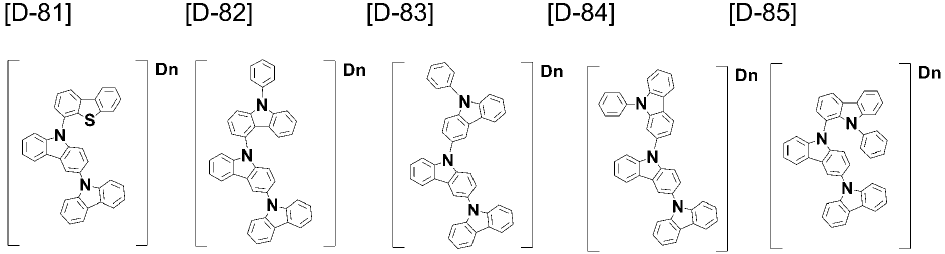 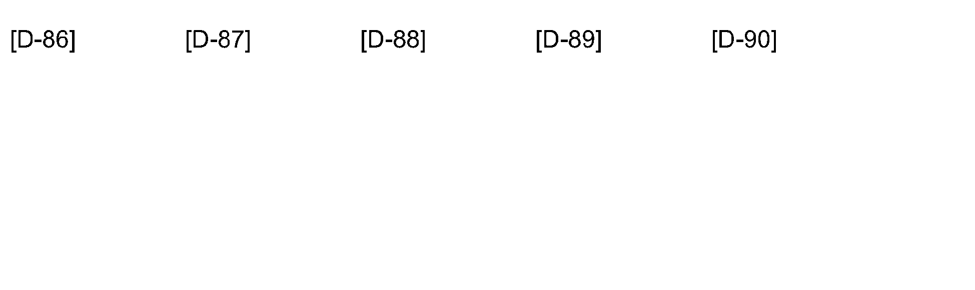 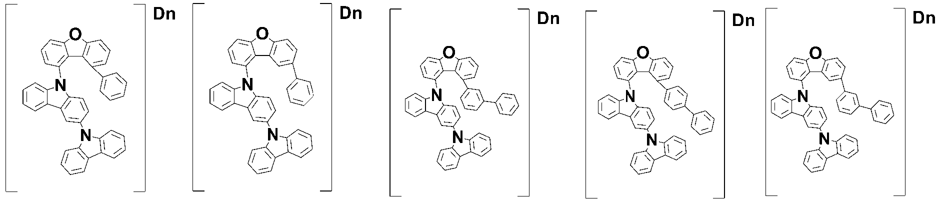 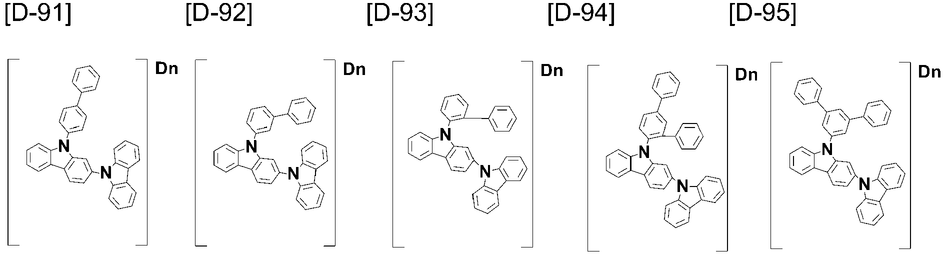 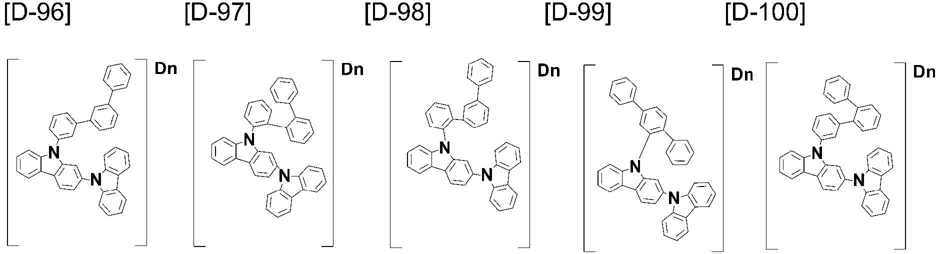 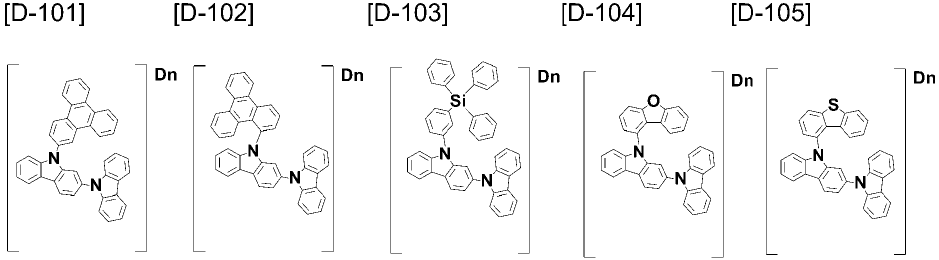 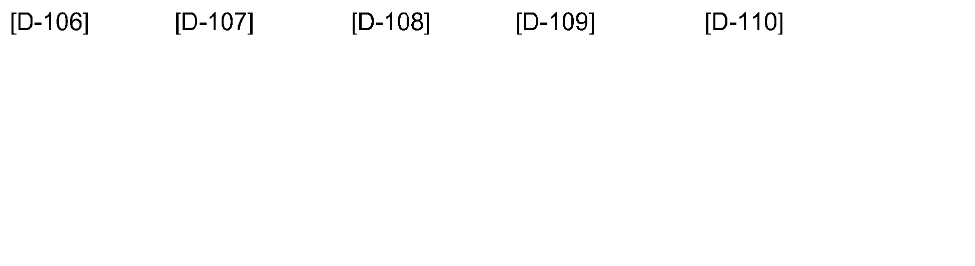 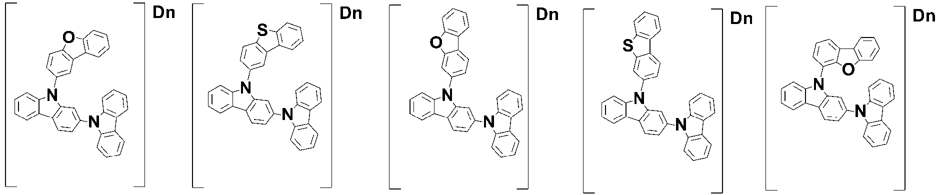 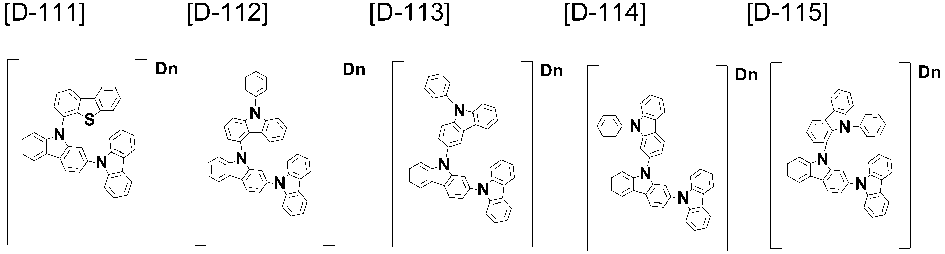 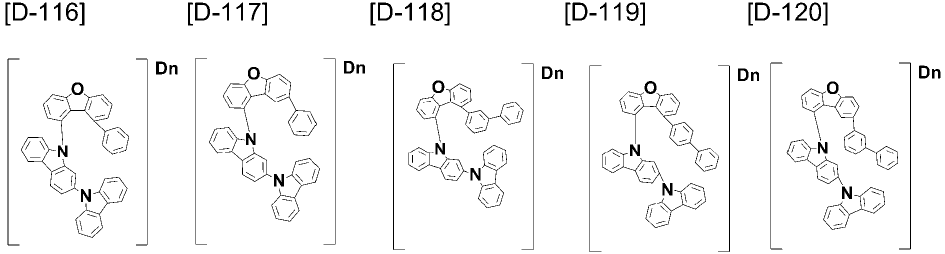 (Dn refers to the number of deuterium substitutions and indicates a structure substituted with one or more deuteriums)

The first compound and the second compound may be included in a weight ratio of, for example, 1:99 to 99:1. Within the above range, bipolar characteristics may be realized by adjusting an appropriate weight ratio using electron transport capability of the first compound and hole transport capability of the second compound, thereby improving efficiency and life-span. Within the above range, they may be included in a weight ratio of, for example, about 10:90 to 90:10, about 20:80 to 80:20, and for example, they may be included in a weight ratio of, for example, about 20:80 to about 70:30, about 20:80 to about 60:40, and about 30:70 to about 60:40. As a specific example, they may be included in a weight ratio of 40:60, 50:50, or 60:40.

Hereinafter, an organic optoelectronic device to which the compound for the organic optoelectronic device or the composition for the organic optoelectronic device described above is applied is described.

The organic optoelectronic device may be a suitable device to convert electrical energy into photoenergy and vice versa, e.g., an organic photoelectric device, an organic light emitting diode, an organic solar cell, or an organic photoconductor drum.

Herein, an organic light emitting diode as one example of an organic optoelectronic device is described referring to drawings.

FIG. 1 is a cross-sectional view showing an organic light emitting diode according to an embodiment.

Referring to FIG. 1, an organic light emitting diode 100 according to an embodiment includes an anode 120 and a cathode 110 facing each other and an organic layer 105 disposed between the anode 120 and cathode 110.

The anode 120 may be made of a conductor having a large work function to help hole injection, and may be for example a metal, a metal oxide and/or a conductive polymer. The anode 120 may be, for example a metal such as nickel, platinum, vanadium, chromium, copper, zinc, gold, and the like or an alloy thereof; a metal oxide such as zinc oxide, indium oxide, indium tin oxide (ITO), indium zinc oxide (IZO), and the like; a combination of a metal and an oxide such as ZnO and Al or SnO₂ and Sb; a conductive polymer such as poly(3-methylthiophene), poly(3,4-(ethylene-1,2-dioxy)thiophene) (PEDOT), polypyrrole, and polyaniline, but is not limited thereto.

The cathode 110 may be made of a conductor having a low work function to facilitate electron injection, for example, and may be made of, for example, a metal, a metal oxide, and/or a conductive polymer. Examples of the cathode 110 include for example, a metal such as magnesium, calcium, sodium, potassium, titanium, indium, yttrium, lithium, gadolinium, aluminum, silver, tin, lead, cesium, and barium or an alloy thereof; and a multi-layered structure material such as LiF/Al, LiO₂/Al, LiF/Ca, LiF/Al, and BaF₂/Ca, but is not limited thereto.

The organic layer 105 may include the aforementioned compound for the organic optoelectronic device or composition for the organic optoelectronic device.

The organic layer 105 includes a light emitting layer 130, the light emitting layer 130 includes a host and a dopant, the host may include the aforementioned compound for the organic optoelectronic device or composition for the organic optoelectronic device, and the dopant may be, for example, a phosphorescent dopant, for example, a red, green, or blue phosphorescent dopant, and for example, a red or green phosphorescent dopant.

The dopant is a material mixed with the compound or composition for an organic optoelectronic device in a small amount to cause light emission, and may be generally a material such as a metal complex that emits light by multiple excitation into a triplet or more. The dopant may be, for example an inorganic, organic, or organic/inorganic compound, and one or more types thereof may be used.

Examples of the dopant may be a phosphorescent dopant and examples of the phosphorescent dopant may be an organic metal compound including Ir, Pt, Os, Ti, Zr, Hf, Eu, Tb, Tm, Fe, Co, Ni, Ru, Rh, Pd, or a combination thereof. The phosphorescent dopant may be, for example a compound represented by Chemical Formula Z, but is not limited thereto.

[Chemical Formula Z] L⁸MX

In Chemical Formula Z, M is a metal, and L⁸ and X are the same or different and are ligands to form a complex compound with M.

The M may be for example Ir, Pt, Os, Ti, Zr, Hf, Eu, Tb, Tm, Fe, Co, Ni, Ru, Rh, Pd, or a combination thereof, and L⁸ and X may be for example a bidentate ligand.

Examples of ligands represented by L⁸ and X may be selected from the chemical formulas listed in Group A, but are not limited thereto.

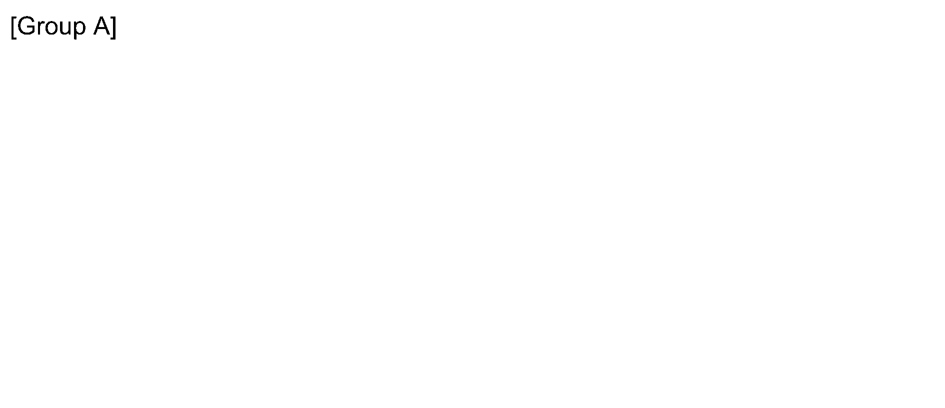

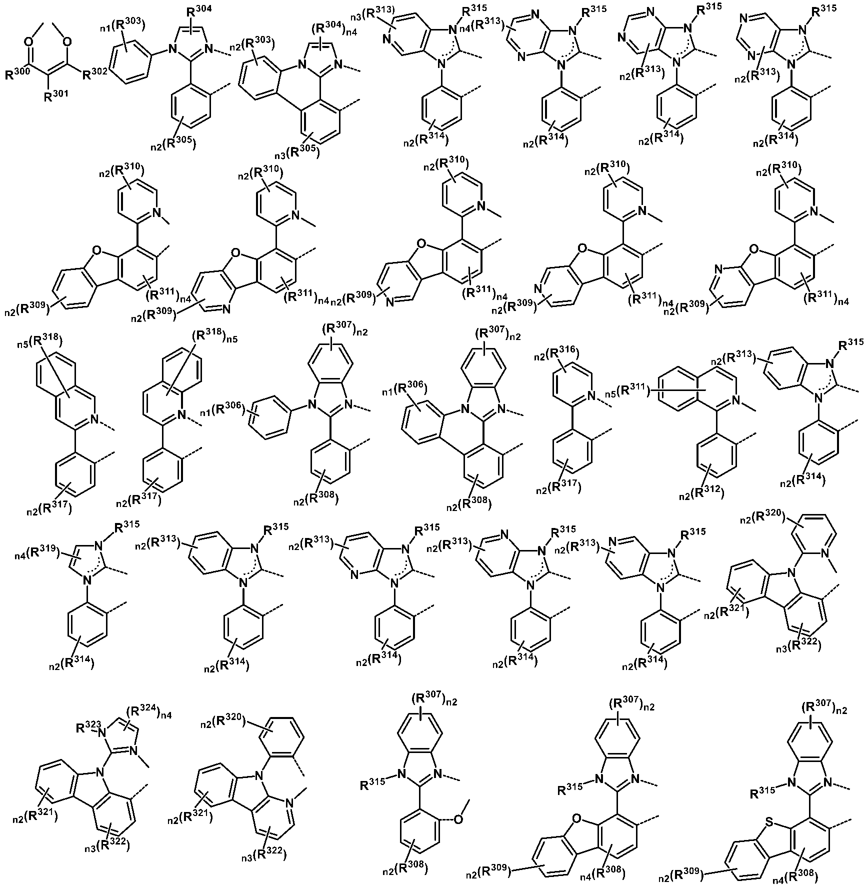

In Group A,
R³⁰⁰ to R¹⁰² are each independently hydrogen, deuterium, a C1 to C30 alkyl group that is substituted or unsubstituted with a halogen, a C6 to C30 aryl group that is substituted or unsubstituted with a C1 to C30 alkyl, or a halogen, and
R³⁰³ to R³²⁴ are each independently hydrogen, deuterium, halogen, a substituted or unsubstituted C1 to C30 alkyl group, a substituted or unsubstituted C1 to C30 alkoxy group, a substituted or unsubstituted C3 to C30 cycloalkyl group, a substituted or unsubstituted C2 to C30 alkenyl group, a substituted or unsubstituted C6 to C30 aryl group, a substituted or unsubstituted C1 to C30 heteroaryl group, a substituted or unsubstituted C1 to C30 amino group, a substituted or unsubstituted C6 to C30 arylamino group, SF₅, a trialkylsilyl group having a substituted or unsubstituted C1 to C30 alkyl group, a dialkylarylsilyl group having a substituted or unsubstituted C1 to C30 alkyl group and a C6 to C30 aryl group, or a triarylsilyl group having a substituted or unsubstituted C6 to C30 aryl group.

In an embodiment, the dopant may be an iridium complex, and may be represented by Chemical Formula 6-1 or Chemical Formula 6-2.

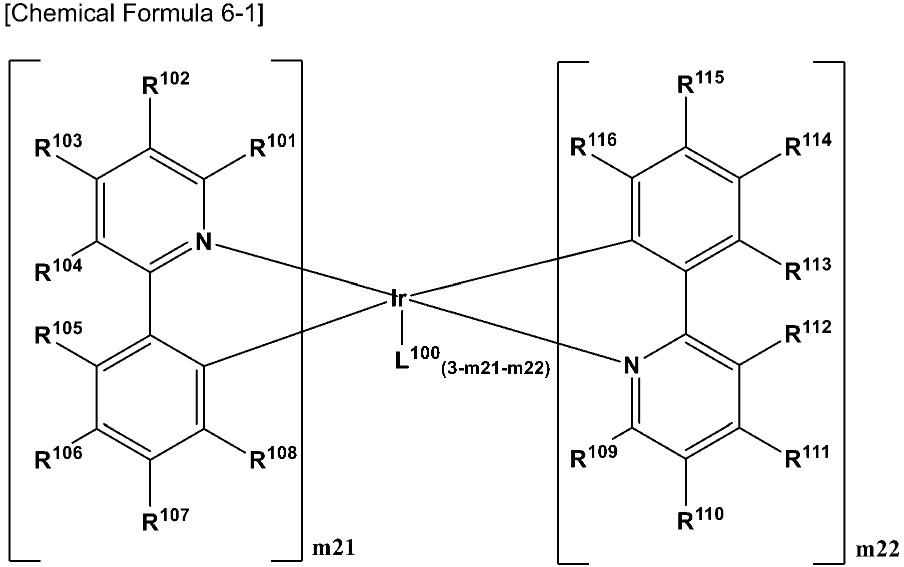

In Chemical Formula 6-1,
R¹⁰¹ to R¹¹⁶ are each independently hydrogen, deuterium, a substituted or unsubstituted C1 to C10 alkyl group, a substituted or unsubstituted C6 to C20 aryl group, or -SiR¹³²R¹³³R¹³⁴,
R¹³² to R¹³⁴ are each independently a substituted or unsubstituted C1 to C6 alkyl group,
at least one of R¹⁰¹ to R¹¹⁶ is a functional group represented by Chemical Formula V-1,
L¹⁰⁰ is a bidentate ligand of a monovalent anion, and is a ligand that coordinates to iridium through a lone pair of carbons or heteroatoms, and
m21 and m22 are each independently any one of integers of 0 to 3, and m21 + m22 is any one of integers of 1 to 3, 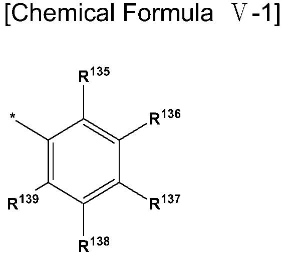
wherein, in Chemical Formula V-1,
R¹³⁵ to R¹³⁹ are each independently hydrogen, deuterium, a substituted or unsubstituted C1 to C10 alkyl group, a substituted or unsubstituted C6 to C20 aryl group, or -SiR¹³²R¹³³R¹³⁴, and
* refers to a portion linked to a carbon atom. 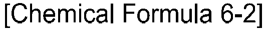 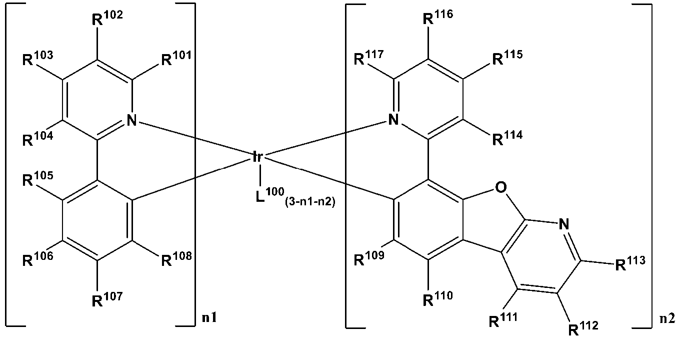
wherein, in Chemical Formula 6-2,
R¹⁰¹ to R¹¹⁷ are each independently hydrogen, deuterium, a substituted or unsubstituted C1 to C10 alkyl group, a substituted or unsubstituted C6 to C20 aryl group, or -SiR¹³³R¹³⁴R¹³⁵,
R¹³³ to R¹³⁵ are each independently a substituted or unsubstituted C1 to C6 alkyl group,
L¹⁰⁰ is a bidentate ligand of a monovalent anion, and is a ligand that coordinates to iridium through a lone pair of carbons or heteroatoms, and
n1 and n2 are independently any integer from 0 to 3, and n1 + n2 is any one of integers of 1 to 3.

In another embodiment, the dopant may be a platinum complex, for example represented by Chemical Formula Z-1.

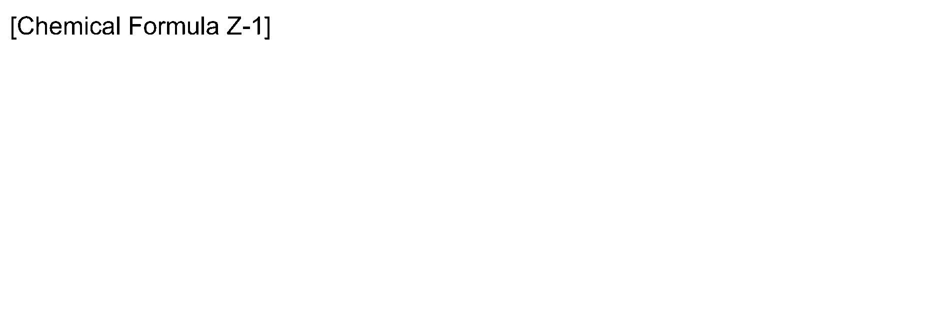

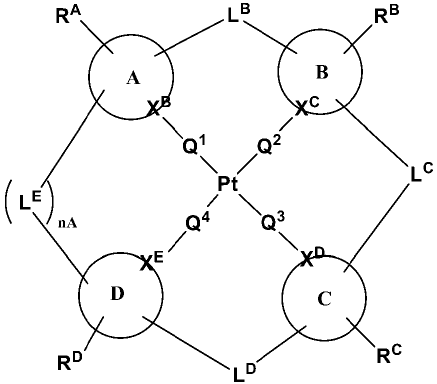

In Chemical Formula Z-1, rings A, B, C, and D independently represent a 5-membered or 6-membered carbocyclic or heterocyclic ring;
R^{A}, R^{B}, R^{C}, and R^{D} independently represent mono-, di-, tri-, or tetra-substitution, or unsubstitution;
L^{B}, L^{C}, and L^{D} are each independent selected from a direct bond, BR, NR, PR, O, S, Se, C=O, S=O, SO₂, CRR', SiRR', GeRR', and a combination thereof;
when nA is 1, L^{E} is selected from a direct bond, BR, NR, PR, O, S, Se, C=O, S=O, SO₂, CRR', SiRR', GeRR', and a combination thereof; when nA is 0, L^{E} does not exist; and
R^{A}, R^{B}, R^{C}, R^{D}, R, and R' are each independently selected from hydrogen, deuterium, a halogen, alkyl group, a cycloalkyl group, a heteroalkyl group, an arylalkyl group, an alkoxy group, an aryloxy group, an amino group, a silyl group, an alkenyl group, a cycloalkenyl group, a heteroalkenyl group, an alkynyl group, an aryl group, a heteroaryl group, an acyl group, a carbonyl group, a carboxylic acid group, an ester group, a nitrile group, an isonitrile group, a sulfanyl group, a sulfinyl group, a sulfonyl group, a phosphino group, and a combination thereof; any adjacent R^{A}, R^{B}, R^{C}, R^{D}, R, and R' are optionally linked to each other to provide a ring; X^{B}, X^{C}, X^{D}, and X^{E} are each independently selected from carbon and nitrogen; and Q¹, Q², Q³, and Q⁴ each represent oxygen or a direct bond.

The platinum complex may be represented, for example, by Chemical Formula 7-1 or Chemical Formula 7-2.

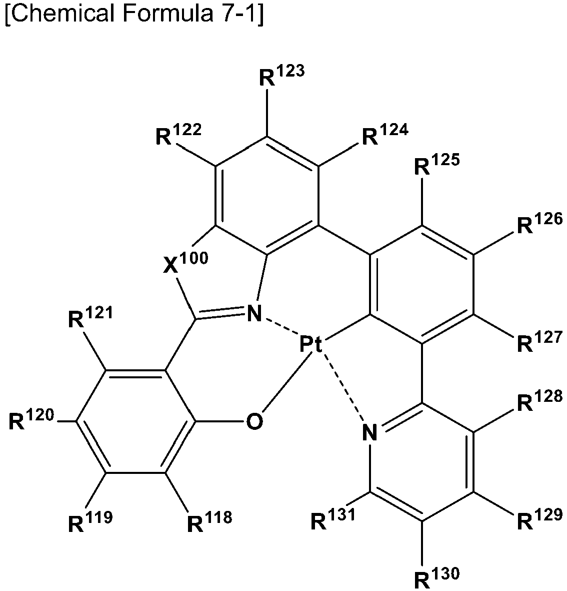

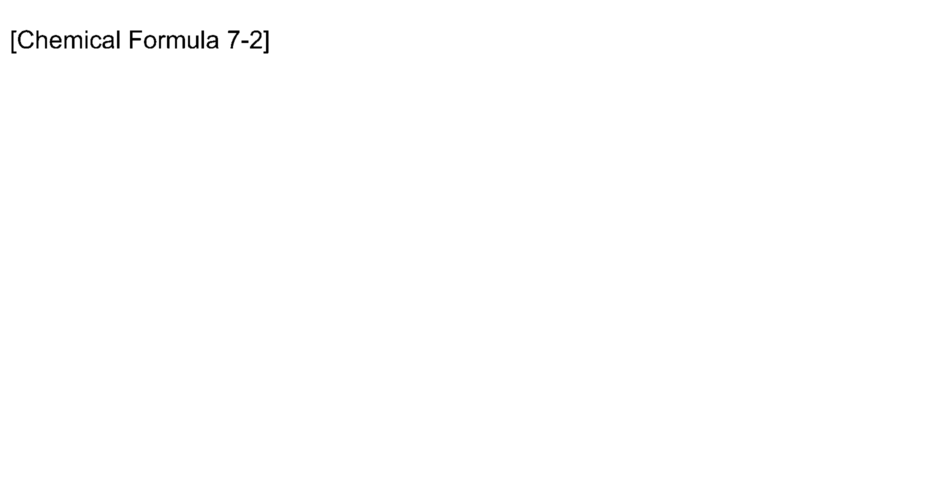

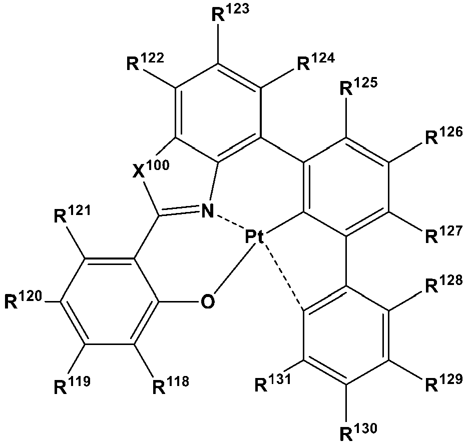

In Chemical Formula 7-1 and Chemical Formula 7-2,
X¹⁰⁰ is selected from O, S, and NR¹³²,
R¹¹⁸ to R¹³² are each independently hydrogen, deuterium, a substituted or unsubstituted C1 to C10 alkyl group, a substituted or unsubstituted C6 to C20 aryl group, or - SiR¹³³R¹³⁴R¹³⁵,
R¹³³ to R¹³⁵ are each independently a substituted or unsubstituted C1 to C6 alkyl group,
at least one of R¹¹⁸ to R¹³² is -SiR¹³³R¹³⁴R¹³⁵ or a tert-butyl group, and
R¹³³ to R¹³⁵ are each independently a substituted or unsubstituted C1 to C6 alkyl group.

The organic layer may further include a charge transport region in addition to the light emitting layer.

The charge transport region may be, for example, a hole transport region 140.

The hole transport region 140 may further increase hole injection and/or hole mobility between the anode 120 and the light emitting layer 130 and block electrons.

Specifically, the hole transport region 140 may include a hole transport layer between the anode 120 and the light emitting layer 130, and a hole transport auxiliary layer between the light emitting layer 130 and the hole transport layer, and at least one of compounds listed in Group B below may be included in at least one layer of the hole transport layer and the hole transport auxiliary layer. 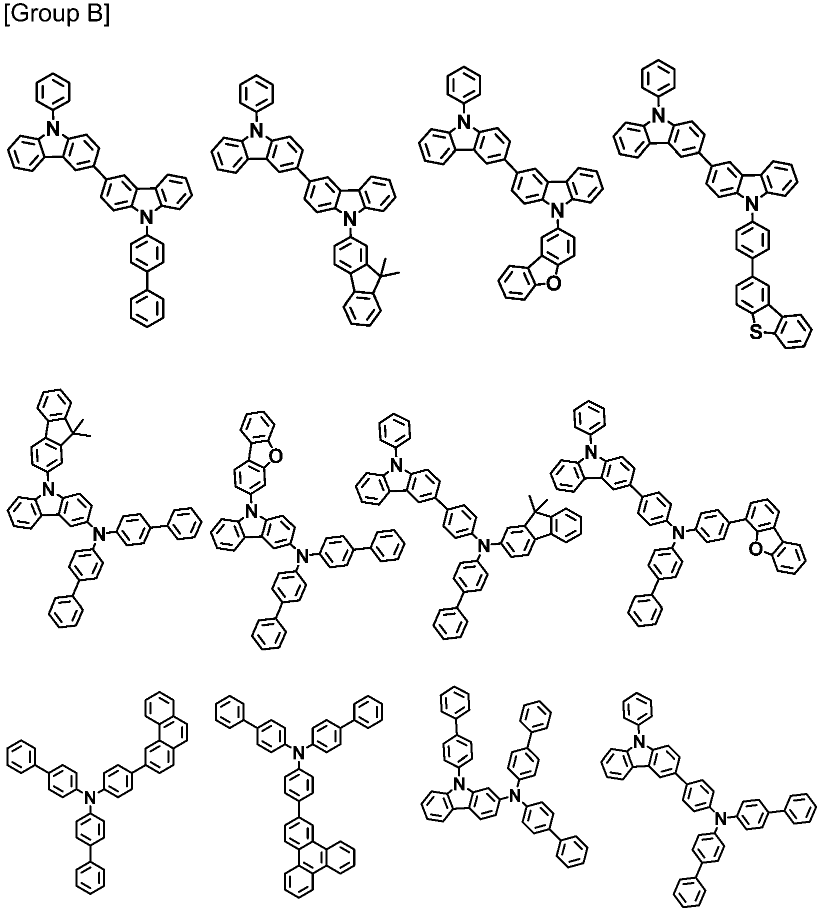 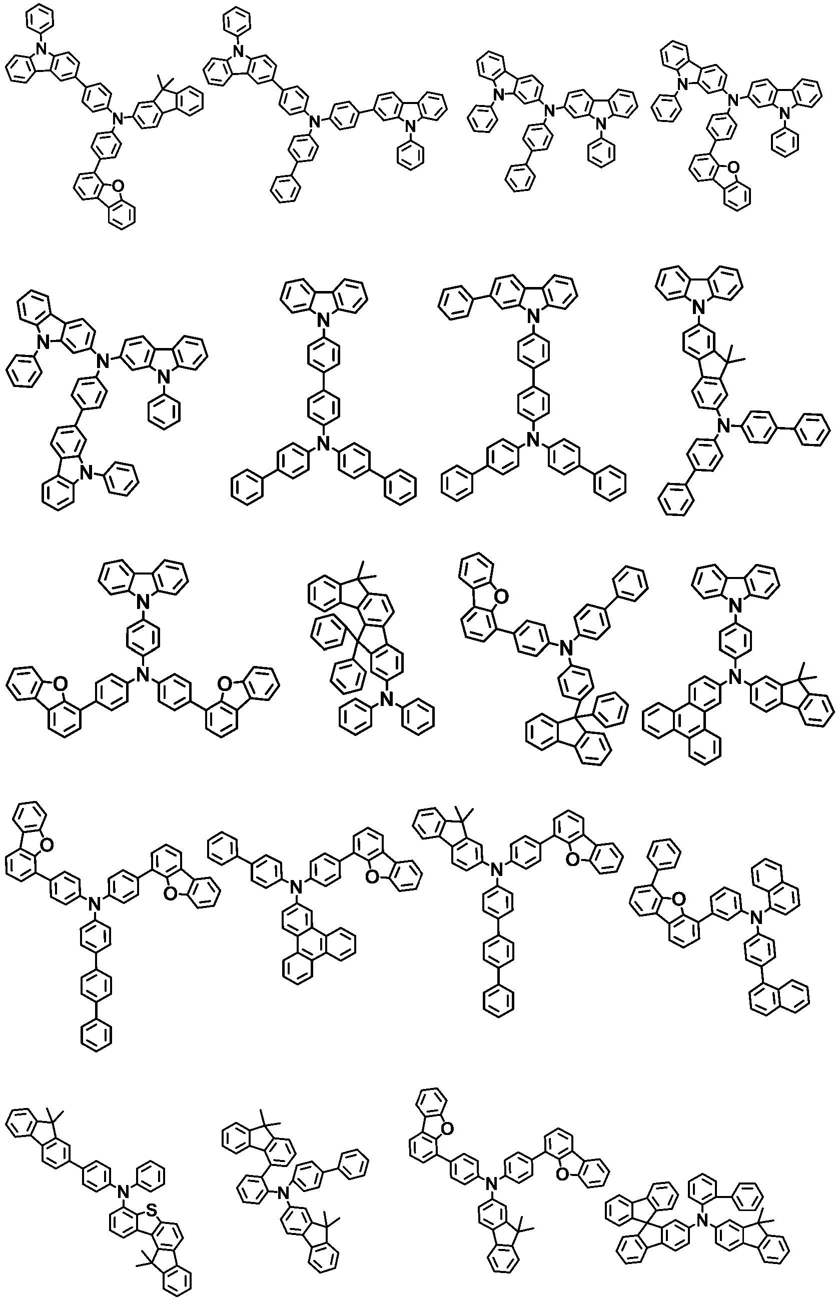 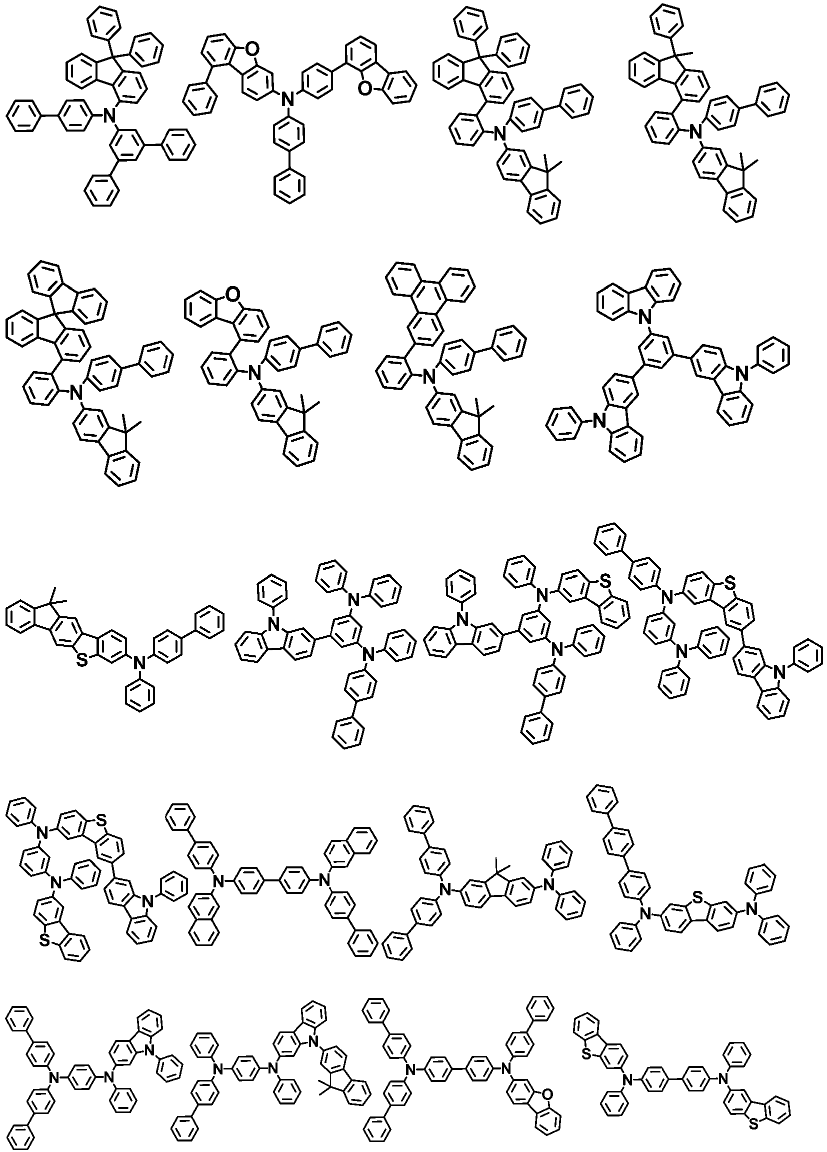 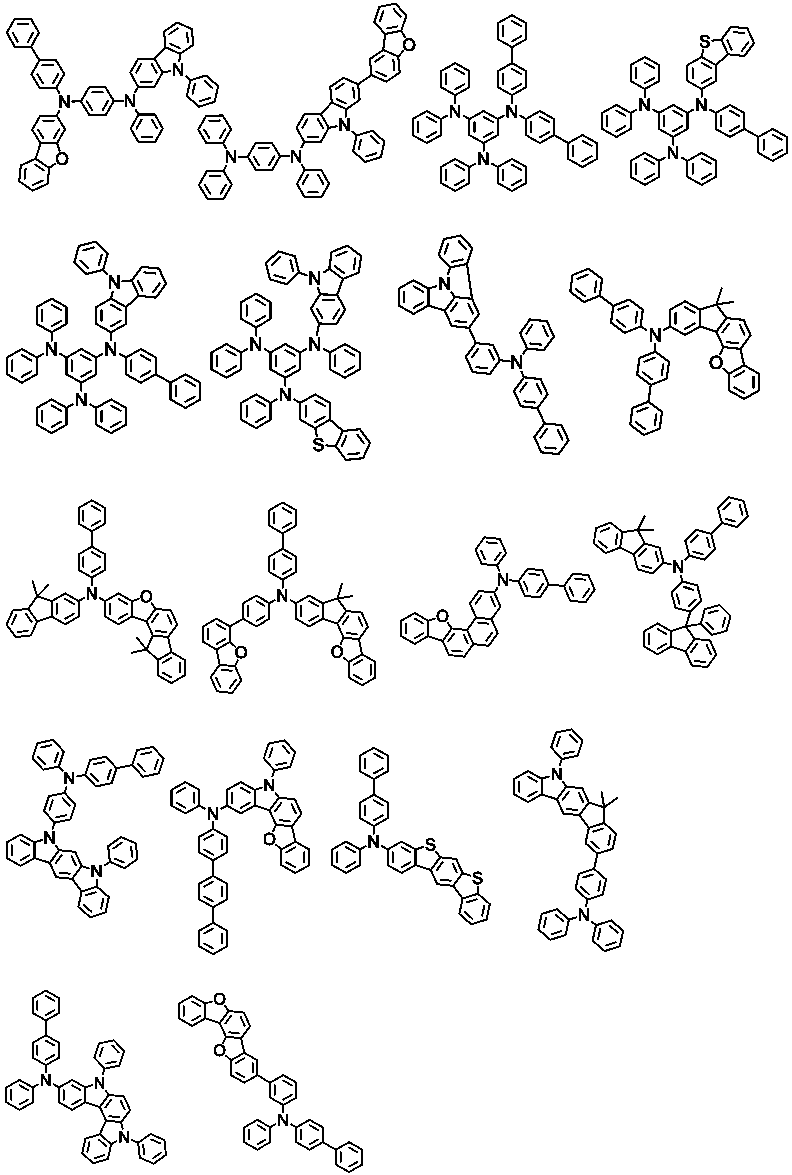 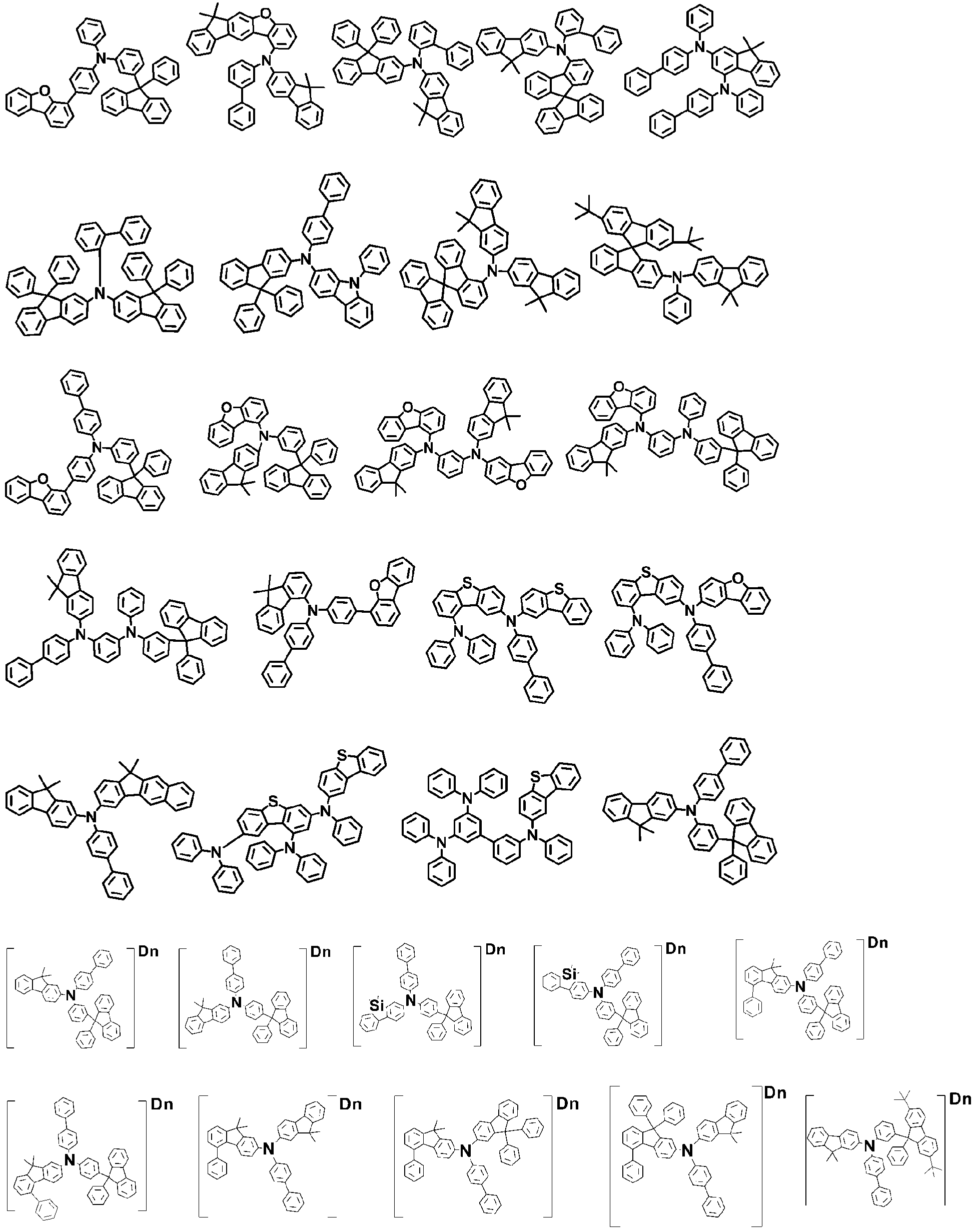 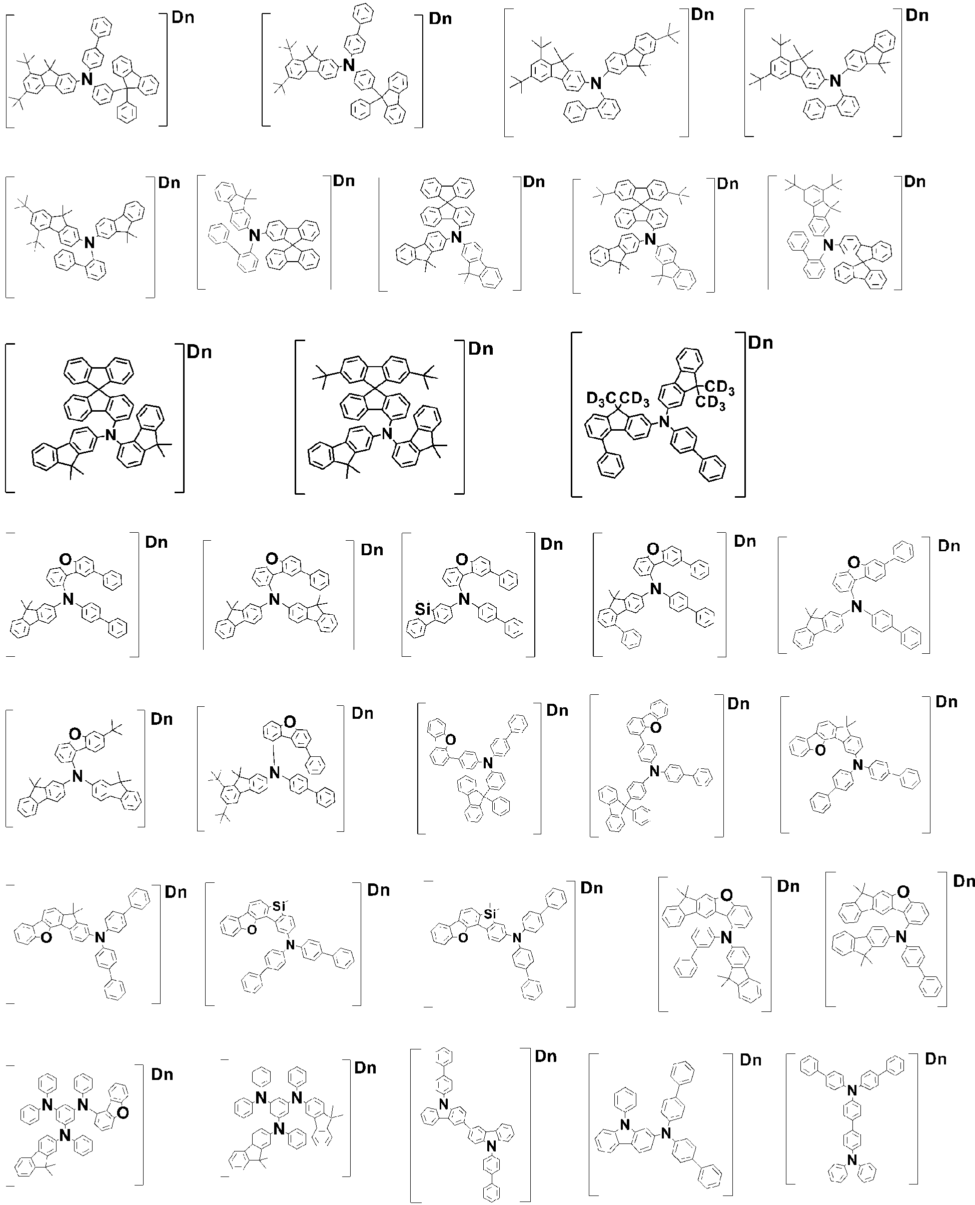 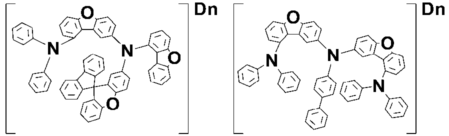 (Dn means the number of substituted deuteriums, and represents a structure in which one or more deuteriums are substituted)

In addition to the compounds described above, known compounds and compounds having structures similar thereto described in US5061569A, JP1993-009471A, WO1995-009147A1, JP1995-126615A, JP1998-095973A, and the like may also be used in the hole transport region 140.

Additionally, the charge transport region may be, for example, an electron transport region 150.

The electron transport region 150 may further increase electron injection and/or electron mobility between the cathode 110 and the light emitting layer 130 and block holes.

Specifically, the electron transport region 150 may include an electron transport layer between the cathode 110 and the light emitting layer 130, and an electron transport auxiliary layer between the light emitting layer 130 and the electron transport layer, and at least one of the compounds listed in Group C below may be included in at least one layer of the electron transport layer and the electron transport auxiliary layer.

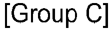

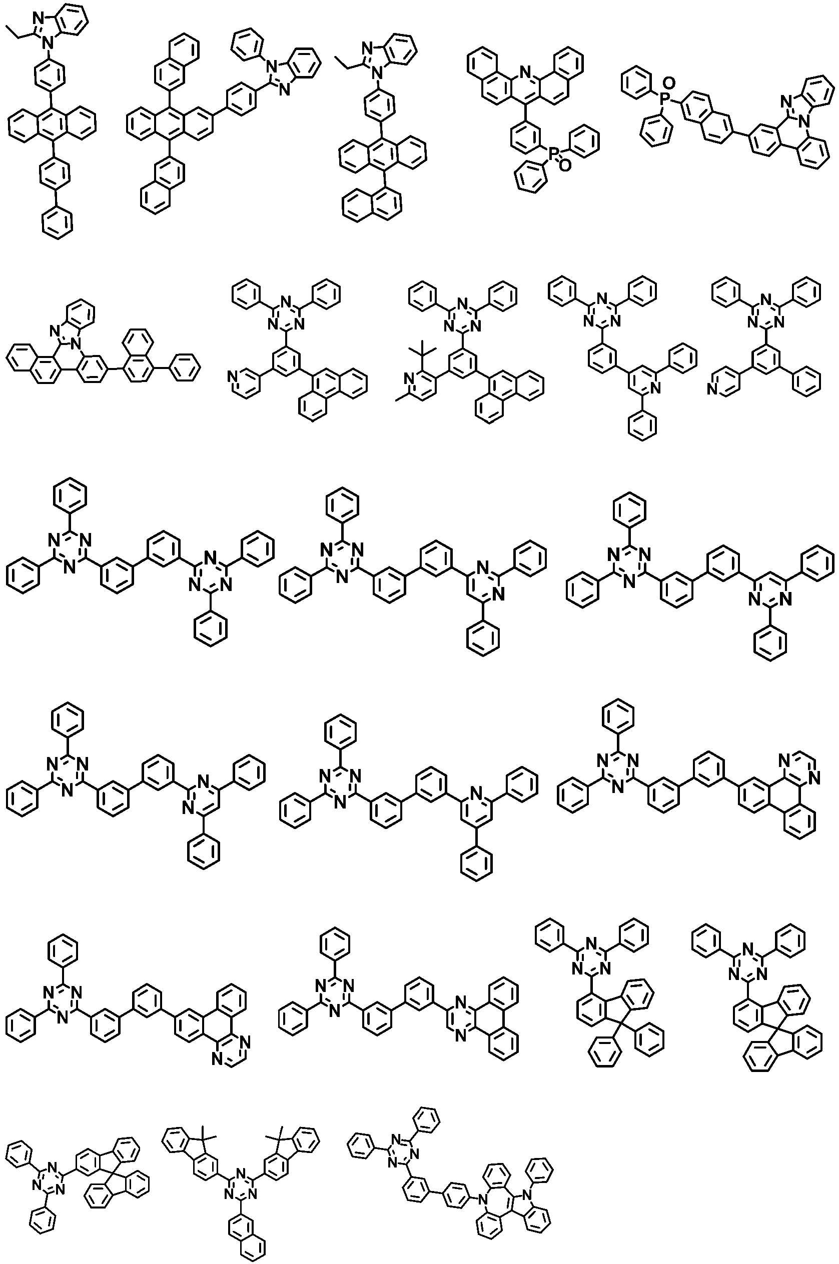

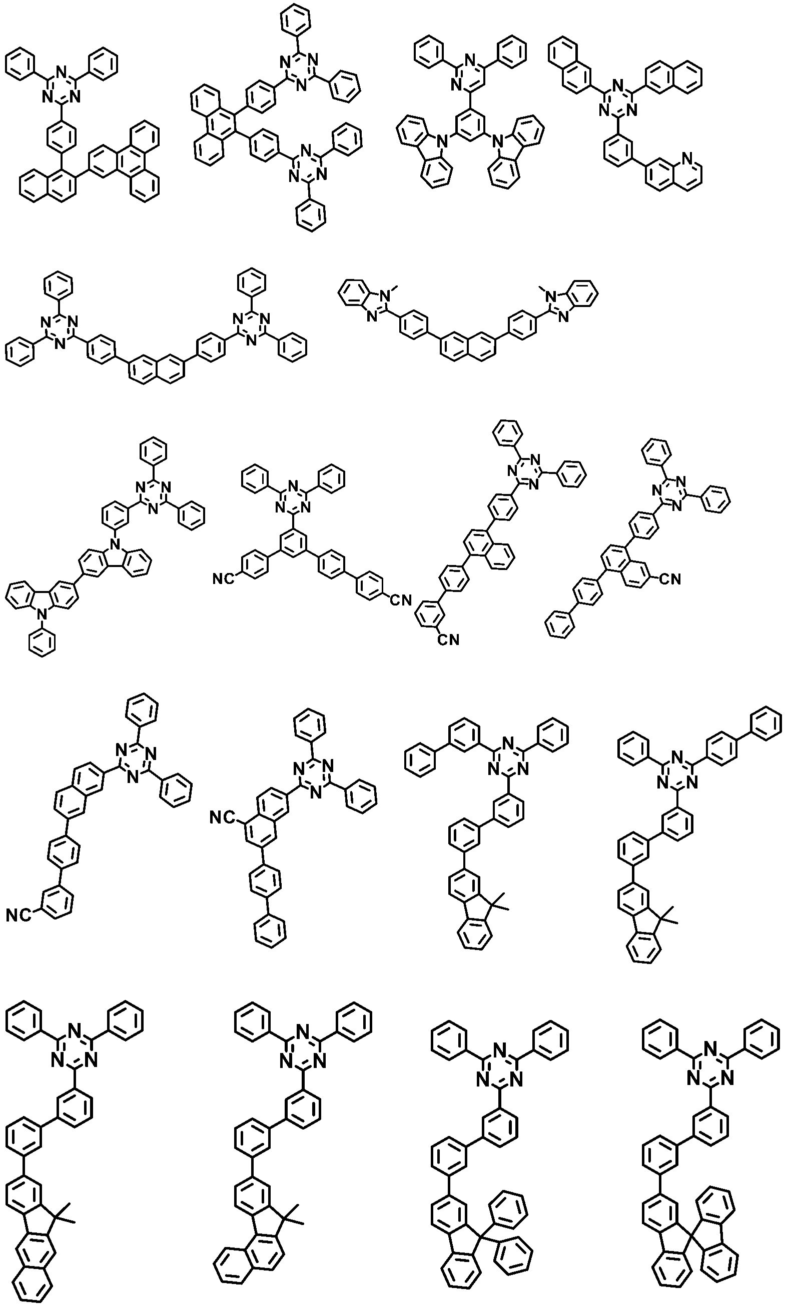

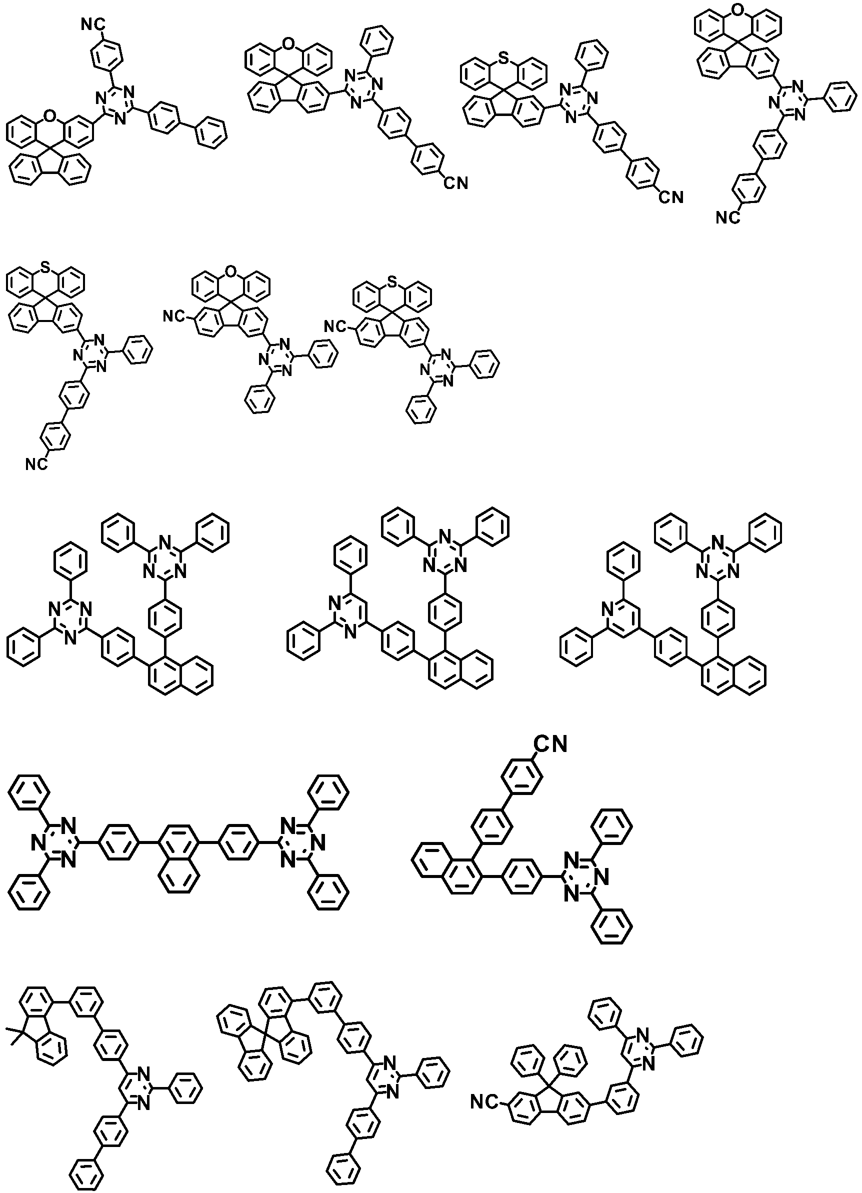

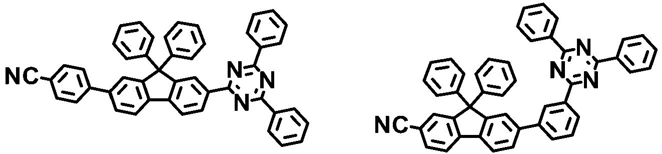

An embodiment may be an organic light emitting diode including a light emitting layer as an organic layer.

Another embodiment may be an organic light emitting diode including a light emitting layer and a hole transport region as an organic layer.

Another embodiment may be an organic light emitting diode including a light emitting layer and an electron transport region as an organic layer.

An organic light emitting diode according to an embodiment of the present invention may include a hole transport region 140 and an electron transport region 150 in addition to a light emitting layer 130 as an organic layer 105 as shown in FIG. 1.

Meanwhile, the organic light emitting diode may additionally include an electron injection layer (not shown), a hole injection layer (not shown), and the like. in addition to the light emitting layer as the aforementioned organic layer.

The organic light emitting diode 100 may be manufactured by forming an anode or a cathode on a substrate, and then forming an organic layer by a dry film method such as vacuum deposition, sputtering, plasma plating and ion plating, and forming a cathode or an anode thereon.

The organic light emitting diode may be applied to an organic light emitting display device.

### [Mode for Invention]

Hereinafter, the embodiments are illustrated in more detail with reference to examples. However, these examples are exemplary, and the present scope is not limited thereto.

Hereinafter, starting materials and reactants used in Examples and Synthesis Examples were purchased from Sigma-Aldrich Co. Ltd., TCI Inc., Tokyo chemical industry, or P&H tech as far as there is no particular comment or were synthesized by known methods.

### (Synthesis of Compound for Organic Optoelectronic Device)

### Synthesis of First Compound

### Synthesis Example 1: Synthesis of Compound 9

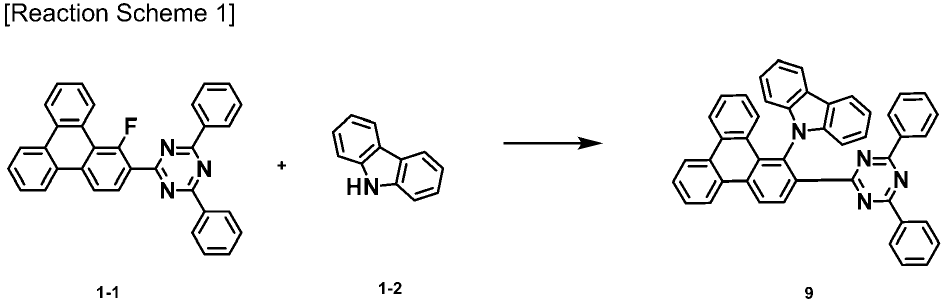

10.0 g (20.9 mmol) of Intermediate 1-1, 3.9 g (23.0 mmol) of Intermediate 1-2, and 8.7 g (62.8 mmol) of K₂CO₃ were placed in a round-bottom flask, and 105 ml of dimethylformamide (DMF) was added and stirred under reflux at 150 °C for 12 hours. After the reaction was completed, it was added to distilled water, stirred, filtered to obtain a solid, which was dissolved in monochlorobenzene (MCB), filtered through silica gel, the organic solvent was removed, and then recrystallized to obtain 9.3 g (71%) of Compound 9.

(LC/MS measured value: 624.23 g/mol, measured value: M+= 625.63 g/mol)

### Synthesis Example 2: Synthesis of Compound 7

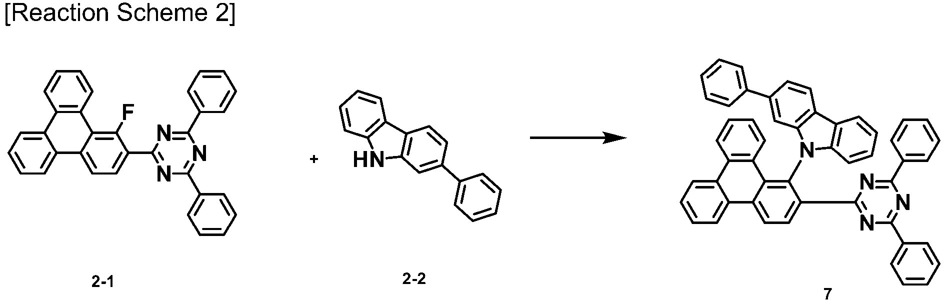

10.0 g (20.9 mmol) of Intermediate 2-1, 5.6 g (23.0 mmol) of Intermediate 2-2, and 8.7 g (62.8 mmol) of K₂CO₃ were placed in a round-bottom flask, and 105 ml of DMF was added and stirred under reflux at 150 °C for 12 hours. After the reaction was completed, it was added to distilled water, stirred, filtered to obtain a solid, which was dissolved in MCB, filtered through silica gel, the organic solvent was removed, and then recrystallized to obtain 10.7 g (73%) of Compound 7.

(LC/MS measured value: 700.26 g/mol, measured value: M+= 701.72 g/mol)

### Synthesis Example 3: Synthesis of Compound 1

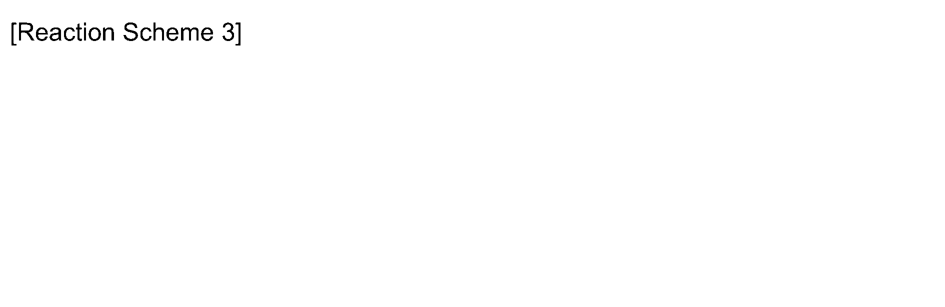

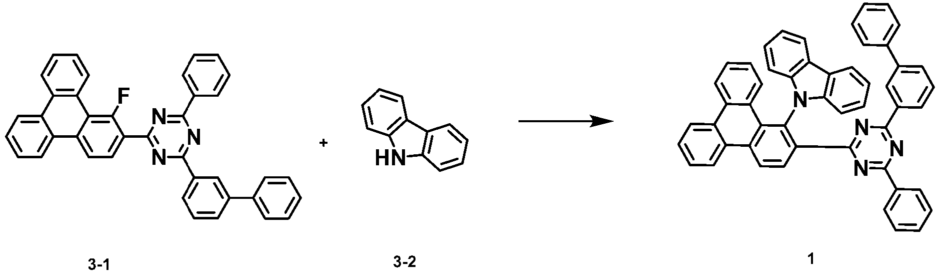

10.0 g (18.1 mmol) of Intermediate 3-1, 3.3 g (19.9 mmol) of Intermediate 3-2, and 7.5 g (54.2 mmol) of K₂CO₃ were placed in a round-bottom flask, and 90 ml of DMF was added and stirred under reflux at 150 °C for 12 hours. After the reaction was completed, it was added to distilled water, stirred, filtered to obtain a solid, which was dissolved in MCB, filtered through silica gel, the organic solvent was removed, and then recrystallized to obtain 9.5 g (75%) of Compound 1.

(LC/MS measured value: 700.26 g/mol, measured value: M+= 701.77 g/mol)

### Synthesis Example 4: Synthesis of Compound 37

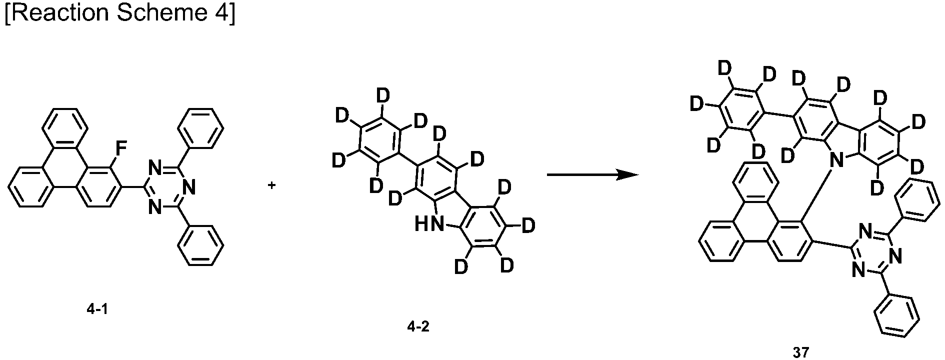

10.0 g (20.9 mmol) of Intermediate 4-1, 5.9 g (23.0 mmol) of Intermediate 4-2, and 8.7 g (62.8mmol) of K₂CO₃ were placed in a round-bottom flask, and 105 ml of DMF was added and stirred under reflux at 150 °C for 12 hours. After the reaction was completed, it was added to distilled water, stirred, filtered to obtain a solid, which was dissolved in MCB, filtered through silica gel, the organic solvent was removed, and then recrystallized to obtain 10.7 g (72%) of Compound 3.

(LC/MS measured value: 712.34 g/mol, measured value: M+= 713.48 g/mol)

### Synthesis Example 5: Synthesis of Compound 57

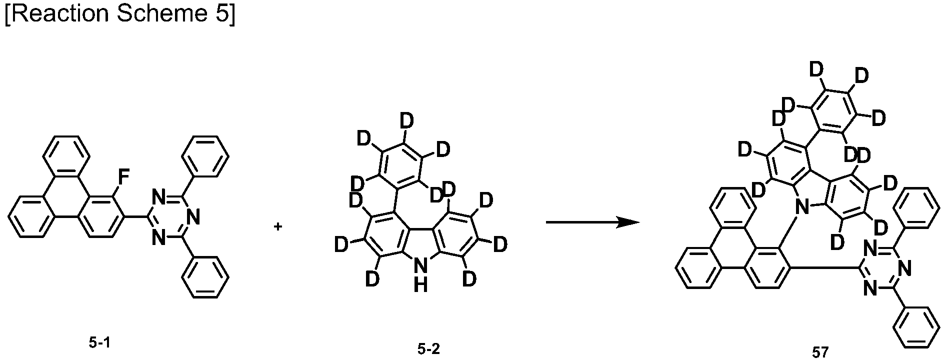

10.0 g (20.9 mmol) of Intermediate 5-1, 5.9 g (23.0 mmol) of Intermediate 5-2, and 8.7 g (62.8 mmol) of K₂CO₃ were placed in a round-bottom flask, and 105 ml of DMF was added and stirred under reflux at 150 °C for 12 hours. After the reaction was completed, it was added to distilled water, stirred, filtered to obtain a solid, which was dissolved in MCB, filtered through silica gel, the organic solvent was removed, and then recrystallized to obtain 10.5 g (70%) of Compound 57.

(LC/MS measured value: 712.34 g/mol, measured value: M+= 713.46 g/mol)

### Synthesis Example 6: Synthesis of Compound R-1

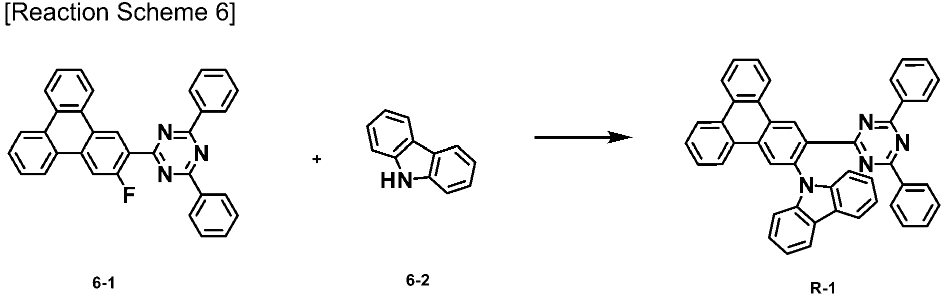

10.0 g (20.9 mmol) of Intermediate 6-1, 3.9 g (23.0 mmol) of Intermediate 6-2, and 8.7 g (62.8 mmol) of K₂CO₃ were placed in a round-bottom flask, and 105 ml of DMF was added and stirred under reflux at 150 °C for 12 hours. After the reaction was completed, it was added to distilled water, stirred, filtered to obtain a solid, which was dissolved in MCB, filtered through silica gel, the organic solvent was removed, and then recrystallized to obtain 8.9 g (68%) of Compound R-1.

(LC/MS measured value: 624.23 g/mol, measured value: M+= 625.61 g/mol)

### Synthesis Example 7: Synthesis of Compound R-2

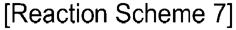

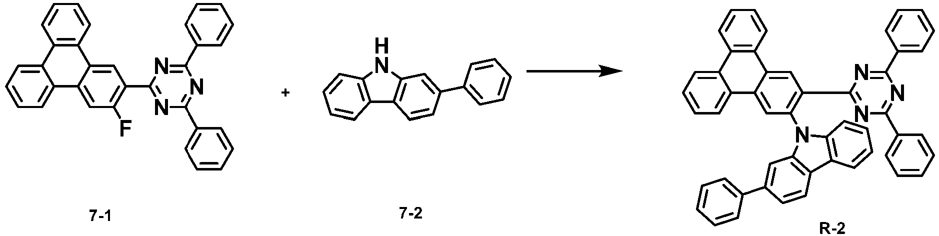

10.0 g (20.9 mmol) of Intermediate 7-1, 5.6 g (23.0 mmol) of Intermediate 7-2, and 8.7 g (62.8 mmol) of K₂CO₃ were placed in a round-bottom flask, and 105 ml of DMF was added and stirred under reflux at 150 °C for 12 hours. . After the reaction was completed, it was added to distilled water, stirred, filtered to obtain a solid, which was dissolved in MCB, filtered through silica gel, the organic solvent was removed, and then recrystallized to obtain 10.1 g (69%) of Compound R-2.

(LC/MS measured value: 700.26 g/mol, measured value: M+= 701.67 g/mol)

### Synthesis Example 8: Synthesis of Compound R-3

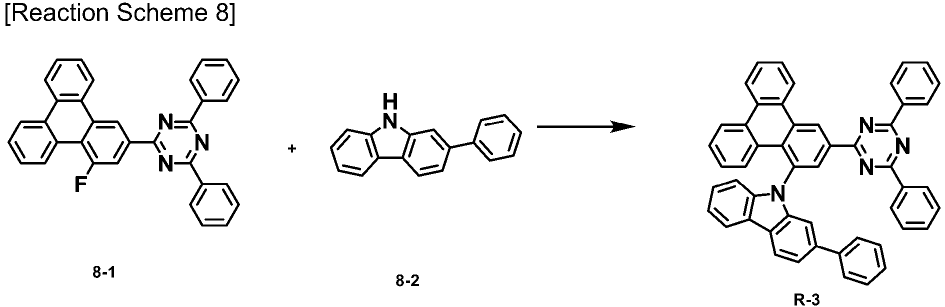

10.0 g (20.9 mmol) of Intermediate 8-1, 5.6 g (23.0 mmol) of Intermediate 8-2, and 8.7 g (62.8 mmol) of K₂CO₃ were placed in a round-bottom flask, and 105 ml of DMF was added and stirred under reflux at 150 °C for 12 hours. After the reaction was completed, it was added to distilled water, stirred, filtered to obtain a solid, which was dissolved in MCB, filtered through silica gel, the organic solvent was removed, and then recrystallized to obtain 9.5 g (65%) of Compound R-3.

(LC/MS measured value: 700.26 g/mol, measured value: M+= 701.69 g/mol)

### Synthesis of Second Compound

### Synthesis Example 9: Synthesis of Compound C-4

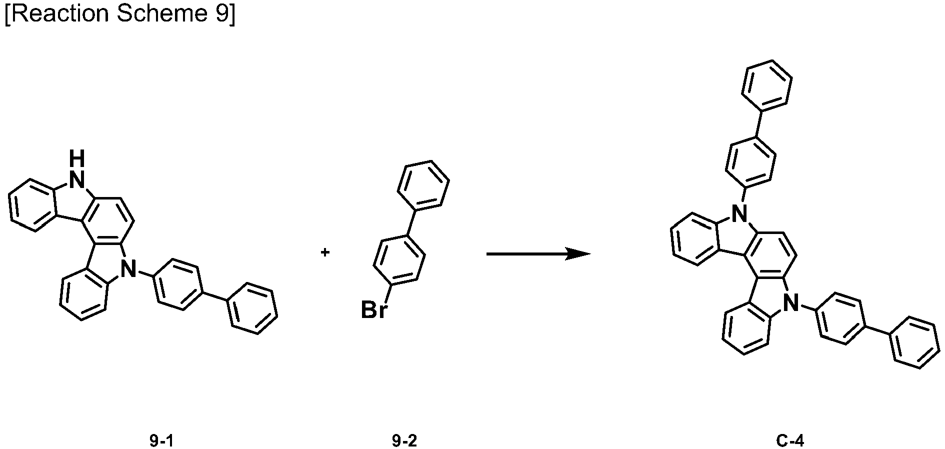

10.0g (24.5mmol) of Intermediate 9-1, 6.3g (26.9mmol) of Intermediate 9-2, 1.1g (1.2mmol) of Pd₂(dba)₃, 3.5g (36.7mmol) of NaOtBu, and 0.7g (3.7mmol) of P(t-Bu)₃ were placed in a round-bottom flask, and 122ml of xylene was added and stirred under reflux at 140 °C for 12 hours. After the reaction was completed, distilled water was added and stirred, then the aqueous layer was removed, and the organic layer was filtered through silica gel and recrystallized to obtain 10.3 g (75%) of Compound C-4.

(LC/MS measured value: 560.23 g/mol, measured value: M+= 561.54 g/mol)

### Synthesis Example 10: Synthesis of Compound C-5

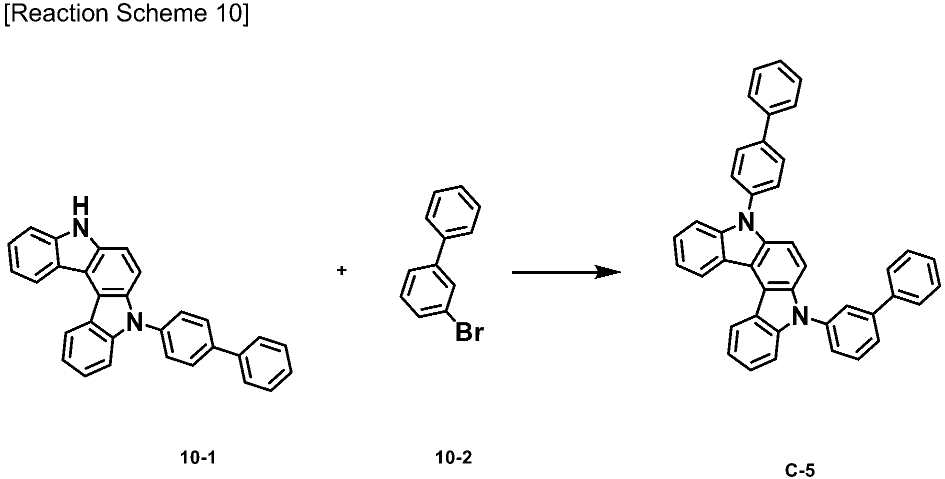

10.0 g (24.5 mmol) of Intermediate 10-1, 6.3 g (26.9 mmol) of Intermediate 10-2, 1.1 g (1.2 mmol) of Pd₂(dba)₃, 3.5 g (36.7 mmol) of NaOtBu, and 0.7 g (3.7 mmol) of P(t-Bu)₃ were placed in a round-bottom flask, and 122ml of Xylene was added and stirred under reflux at 140 °C for 12 hours. After the reaction was completed, distilled water was added and stirred, then the aqueous layer was removed, and the organic layer was filtered through silica gel and recrystallized to obtain 9.7 g (71%) of Compound C-5.

(LC/MS measured value: 560.23 g/mol, measured value: M+= 561.57 g/mol)

### Example 1: Manufacturing of Green Organic Light Emitting Diode (Single Host)

A glass substrate coated with a thin film of ITO (indium tin oxide) was ultrasonically cleaned with distilled water. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. The prepared ITO transparent electrode was used as an anode, Compound A doped with 3% NDP-9 (Novaled GmbH) was vacuum-deposited on the ITO substrate to form a 100 A-thick hole injection layer, and Compound A is deposited on the hole injection layer to a thickness of 1350 Å to form a hole transport layer. Compound B was deposited on the hole transport layer to a thickness of 350 Å to form a hole transport auxiliary layer. On the hole transport auxiliary layer, Compound 9 was used as a host and PhGD was doped at 7 wt% as a dopant to form a light emitting layer with a thickness of 400 Å by vacuum deposition. Subsequently, Compound C was deposited on the light emitting layer to a thickness of 50 Å to form an electron transport auxiliary layer, and Compound D and Liq were simultaneously vacuum-deposited in a weight ratio of 1:1 to form an electron transport layer with a thickness of 300 Å. LiQ (15 Å) and Al (1200 Å) were sequentially vacuum-deposited on the electron transport layer to form a cathode, thereby manufacturing an organic light emitting diode.

It was manufactured with a structure of ITO/Compound A (3% NDP-9 doping, 100 Å)/Compound A (1350 Å)/Compound B (350 Å)/EML [Host (Compound 9) : PhGD = 93 wt% : 7 wt%] (400 Å)/Compound C (50 Å)/Compound D:LiQ (300 Å)/LiQ (15 Å)/Al (1200 Å).
Compound A: N-(biphenyl-4-yl)-9,9-dimethyl-N-(4-(9-phenyl-9H-carbazol-3-yl)phenyl)-9H-fluoren-2-amine
Compound B: N-[4-(4-Dibenzofuranyl)phenyl]-N-[4-(9-phenyl-9H-fluoren-9-yl)phenyl][1,1'-biphenyl]-4-amine
Compound C: 2,4-Diphenyl-6-(4',5',6'-triphenyl[1,1':2',1":3",1‴:3‴,1ʺʺ-quinquephenyl]-3""-yl)-1,3,5-triazine
Compound D: 2-(1,1'-Biphenyl-4-yl)-4-(9,9-diphenylfluoren-4-yl)-6-phenyl-1,3,5-triazine

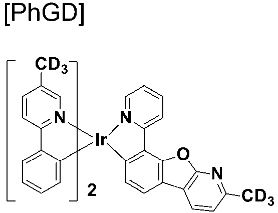

### Examples 2 to 3 and Comparative Examples 1 to 2

Organic light emitting diodes were manufactured in the same method as in Example 1 except for changing to compositions listed in Table 1 below.

### Example 4: Manufacturing of Green Organic Light Emitting Diode (Mixed Host)

A glass substrate coated with a thin film of ITO (indium tin oxide) was ultrasonically cleaned with distilled water. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. The prepared ITO transparent electrode was used as an anode, Compound E doped with 3% NDP-9 (Novaled GmbH) was vacuum-deposited on the ITO substrate to form a 100 A-thick hole injection layer, and Compound E is deposited on the hole injection layer to a thickness of 1350 Å to form a hole transport layer. Compound F was deposited on the hole transport layer to a thickness of 320 Å to form a hole transport auxiliary layer. On the hole transport auxiliary layer, Compound 9 and Compound C-4 were simultaneously used as hosts in a weight ratio of 3:7 and PtGD was doped at 15 wt% as a dopant to form a light emitting layer with a thickness of 380 Å by vacuum deposition. Subsequently, Compound G was deposited on the light emitting layer to a thickness of 50 Å to form an electron transport auxiliary layer, and Compound H and Liq were simultaneously vacuum-deposited in a weight ratio of 1:1 to form an electron transport layer with a thickness of 300 Å. LiQ (15 Å) and Al (1200 Å) were sequentially vacuum-deposited on the electron transport layer to form a cathode, thereby manufacturing an organic light emitting diode.

It was manufactured with a structure of ITO/Compound E (3% NDP-9 doping, 100 Å)/ Compound E (1350 Å)/Compound F (320 Å)/EML [Host (Compound 9 : Compound C-4 = 3 : 7 wt%/wt%) : PtGD = 85 wt% : 15 wt%] (380 Å)/Compound G (50 Å)/Compound H: LiQ (300 Å)/LiQ (15 Å)/Al (1200 Å).
Compound E: N-(9,9-diphenyl-9H-fluoren-2-yl)-N,9-diphenyl-9H-carbazol-2-amine
Compound F: 9,9-dimethyl-N-[3-(9-phenyl-9H-fluoren-9-yl)phenyl]-4-(4-phenylphenyl)-9H-fluoren-2-amine
Compound G: 4-{4-[4-(9,9-dimethyl-9H-fluoren-4-yl)phenyl]phenyl}-2-phenyl-6-(4-phenylphenyl)pyrimidine
Compound H: 2-(4-{1-[4-(diphenyl-1,3,5-triazin-2-yl)phenyl]naphthalene-2-yl}-4,6-diphenyl-1,3,5-triazine

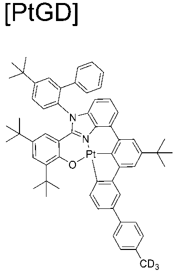

### Examples 5 to 13 and Comparative Examples 3 to 5

Organic light emitting diodes were manufactured in the same method as in Example 4 except for changing to compositions listed in Table 2 below.

### Evaluation

Driving voltage, luminous efficiency, and life-span characteristics of the organic light emitting diodes according to Examples 1 to 13 and Comparative Examples 1 to 8.

Specific measurement methods are as follows, and the results are shown in Table 1 to Table 2.

### (1) Measurement of Current Density Change Depending on Voltage Change

The obtained organic light emitting diodes were measured regarding a current value flowing in the unit diode, while increasing the voltage from 0 V to 10 V using a current-voltage meter (Keithley 2400), and the measured current value was divided by area to provide the results.

### (2) Measurement of Luminance Change Depending on Voltage Change

Luminance was measured by using a luminance meter (Minolta Cs-1000A), while the voltage of the organic light emitting diodes was increased from 0 V to 10 V.

### (3) Measurement of Luminous Efficiency

The luminance and the current density measured from (1) and (2) above and a voltage were used to calculate the current efficiency(cd/A) at the same current density (10 mA/cm²).

The luminous efficiency values of Examples 1 to 3 and Comparative Examples 1 to 2 were calculated as relative values based on Comparative Example 1 and listed in Table 1 below.

The luminous efficiency values of Examples 4 to 8 and Comparative Examples 3 to 5 were calculated as relative values based on Comparative Example 3 and listed in Table 2 below.

The luminous efficiency values of Examples 9 to 13 and Comparative Examples 6 to 8 were calculated as relative values based on Comparative Example 6 and listed in Table 3 below.

### (4) Measurement of Life-span

Results were obtained by maintaining luminance (cd/m²) at 24000 cd/m² and measuring a time until current efficiency (cd/A) decreased to 97%.

The life-span measurement values of Examples 1 to 3 and Comparative Examples 1 to 2 were calculated as relative values based on Comparative Example 1 and listed in Table 1 below.

The life-span measurement values of Examples 4 to 8 and Comparative Examples 3 to 5 were calculated as relative values based on Comparative Example 3 and listed in Table 2 below.

The life-span measurement values of Examples 9 to 13 and Comparative Examples 6 to 8 were calculated as relative values based on Comparative Example 6 and listed in Table 3 below.

**(Table 1)**

| No. | Compound | Luminous efficiency (%) | Life-span (%) |
|---|---|---|---|
| Example 1 | 9 | 108 | 105 |
| Example 2 | 7 | 104 | 105 |
| Example 3 | 1 | 106 | 107 |
| Comparative Example 1 | R-1 | 100 | 100 |
| Comparative Example 2 | R-3 | 90 | 50 |

**(Table 2)**

| No. | First compound | Second compound | Luminous efficiency (%) | Life-span (%) |
|---|---|---|---|---|
| Example 4 | 9 | C-4 | 106 | 111 |
| Example 5 | 7 | C-4 | 103 | 112 |
| Example 6 | 1 | C-4 | 105 | 115 |
| Example 7 | 37 | C-4 | 103 | 139 |
| Example 8 | 57 | C-4 | 105 | 134 |
| Comparative Example 3 | R-1 | C-4 | 100 | 100 |
| Comparative Example 4 | R-2 | C-4 | 100 | 110 |
| Comparative Example 5 | R-3 | C-4 | 95 | 60 |

**(Table 3)**

| No. | First compound | Second compound | Luminous efficiency (%) | Life-span (%) |
|---|---|---|---|---|
| Example 9 | 9 | C-5 | 105 | 115 |
| Example 10 | 7 | C-5 | 103 | 113 |
| Example 11 | 1 | C-5 | 105 | 116 |
| Example 12 | 37 | C-5 | 103 | 148 |
| Example 13 | 57 | C-5 | 104 | 140 |
| Comparative Example 6 | R-1 | C-5 | 100 | 100 |
| Comparative Example 7 | R-2 | C-5 | 100 | 110 |
| Comparative Example 8 | R-3 | C-5 | 95 | 54 |

Referring to Table 1 to Table 3, it can be confirmed that the organic light emitting diodes according to Examples 1 to 13 exhibited significantly improved luminous efficiency and life-span characteristics compared to the organic light emitting diodes according to Comparative Examples 1 to 8.

While this disclosure has been described in connection with what is presently considered to be practical example embodiments, it is to be understood that the invention is not limited to the disclosed embodiments, but, on the contrary, is intended to cover various modifications and equivalent arrangements included within the spirit and scope of the appended claims.

## Claims

1. A compound for an organic optoelectronic device represented by Chemical Formula 1A or Chemical Formula 1B: 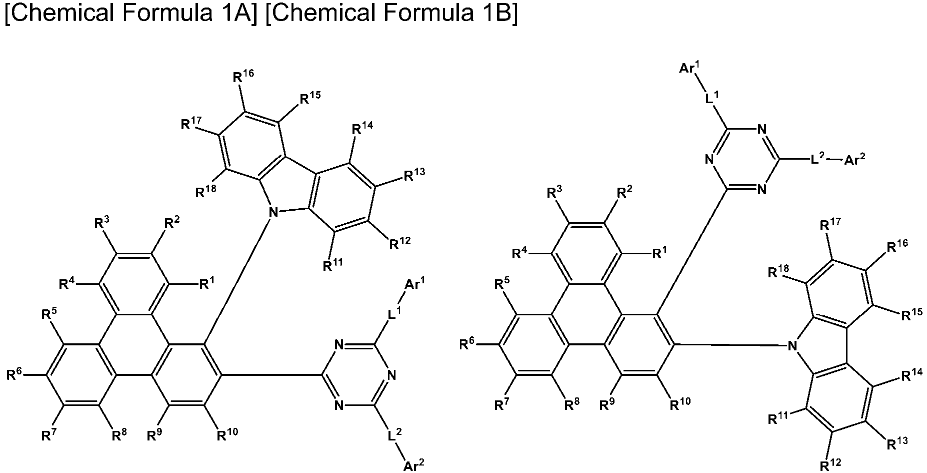 wherein, in Chemical Formula 1A and Chemical Formula 1B,
Ar¹ and Ar² are each independently a substituted or unsubstituted C6 to C30 aryl group, or a substituted or unsubstituted C2 to C30 heterocyclic group,
L¹ and L² are each independently a single bond, a substituted or unsubstituted C6 to C30 arylene group, or a substituted or unsubstituted C2 to C30 heterocyclic group, and
R¹ to R¹⁸ are each independently hydrogen, deuterium, a cyano group, a halogen, a substituted or unsubstituted C1 to C30 alkyl group, a substituted or unsubstituted C6 to C30 aryl group, or a substituted or unsubstituted C2 to C30 heterocyclic group.

2. The compound for the organic optoelectronic device as claimed in claim 1, wherein
Ar¹ and Ar² are each independently a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, a substituted or unsubstituted triphenylene group, a substituted or unsubstituted dibenzofuranyl group, a substituted or unsubstituted dibenzothiophenyl group, a substituted or unsubstituted carbazolyl group, or a substituted or unsubstituted dibenzosilolyl group.

3. The compound for the organic optoelectronic device as claimed in claim 1, wherein
L¹-Ar¹ and L²-Ar² are each independently one selected from substituents listed in Group I : 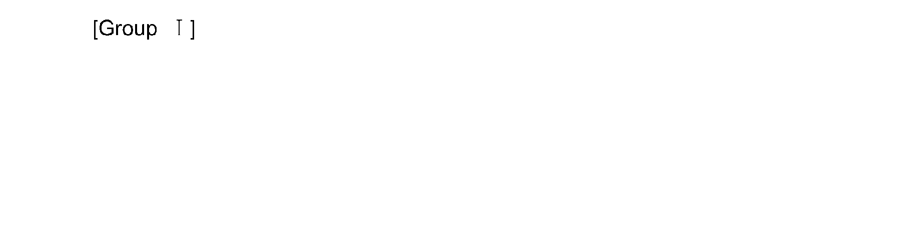 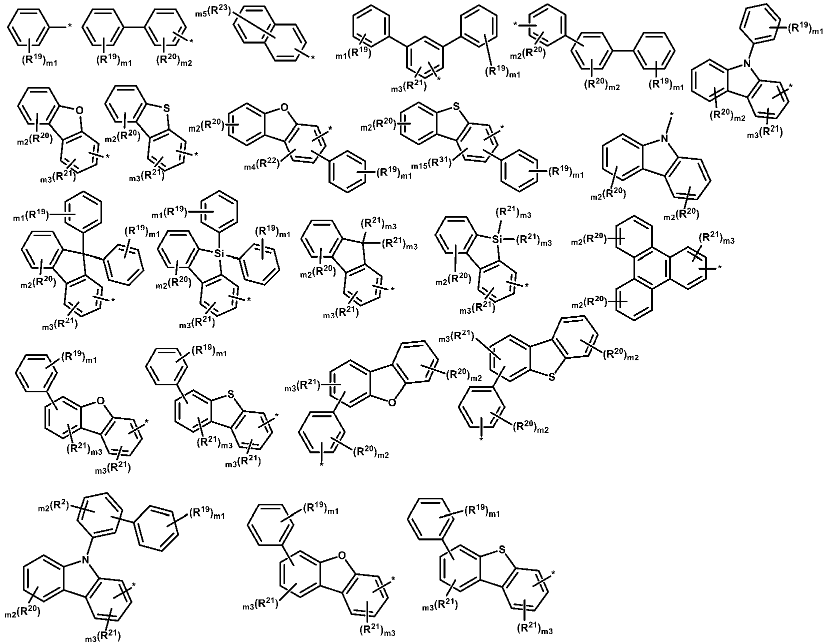
wherein, in Group I ,
R¹⁹ to R²³ are each independently hydrogen, deuterium, a cyano group, a C1 to C10 alkyl group, or a C6 to C12 aryl group,
m1 is one of integers of 1 to 5,
m2 is one of integers of 1 to 4,
m3 is one of integers of 1 to 3,
m4 is an integer of 1 or 2,
m5 is one of integers of 1 to 7, and
* is a linking point.

4. The compound for the organic optoelectronic device as claimed in claim 1, wherein
R¹ to R¹⁸ are each independently hydrogen, deuterium, a substituted or unsubstituted phenyl group, or a substituted or unsubstituted biphenyl group.

5. The compound for the organic optoelectronic device as claimed in claim 1, wherein
the compound is one selected from compounds listed in Group 1: 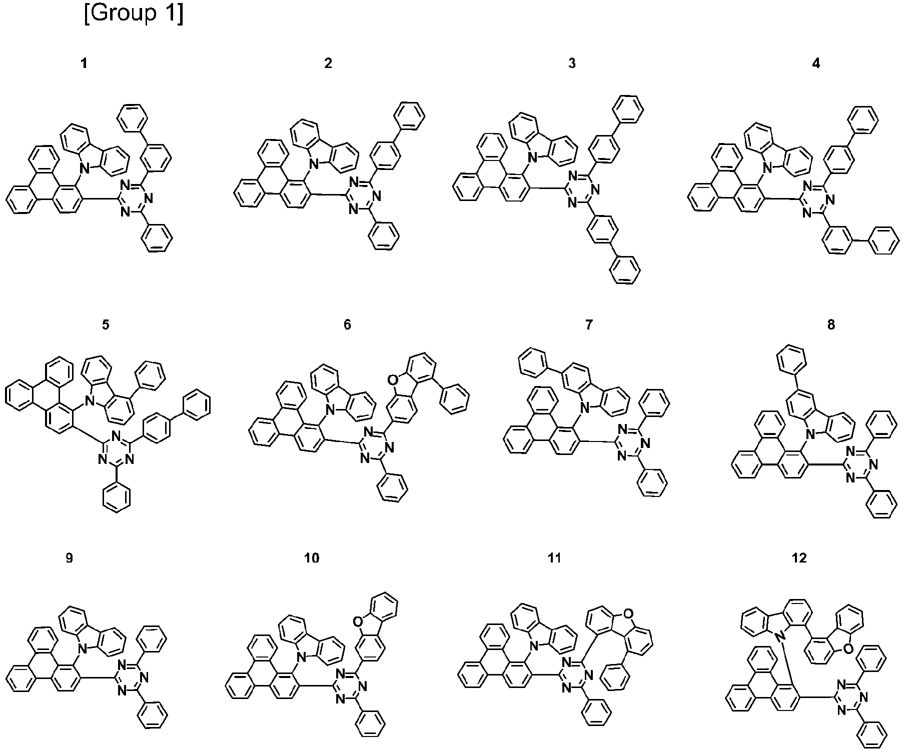 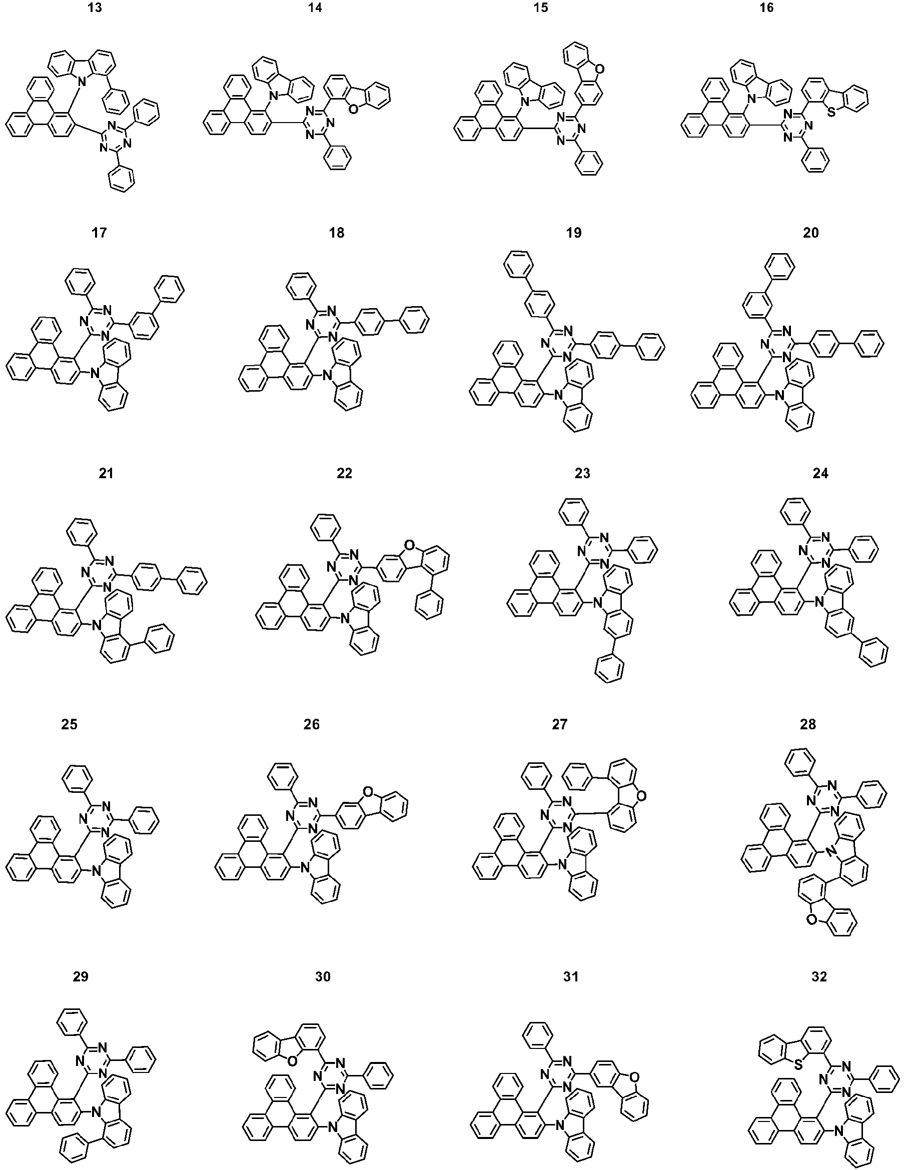 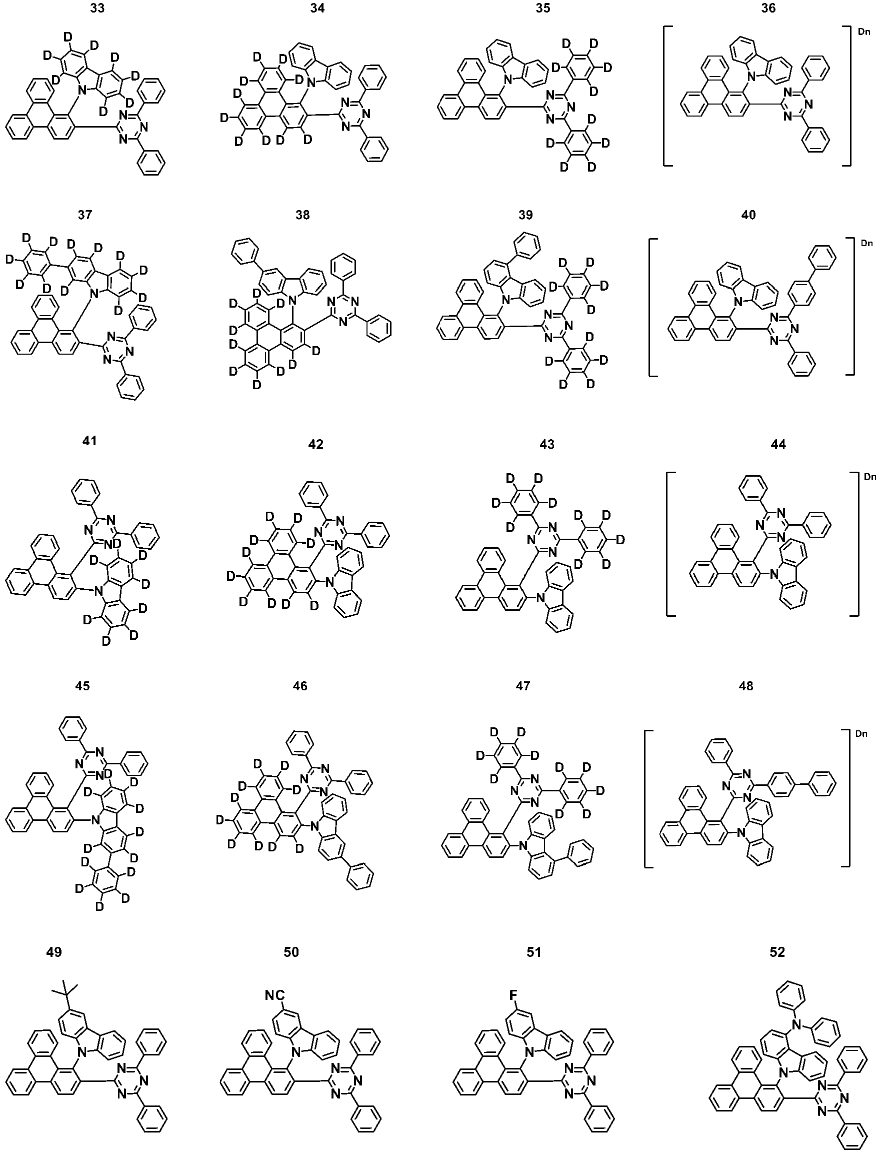 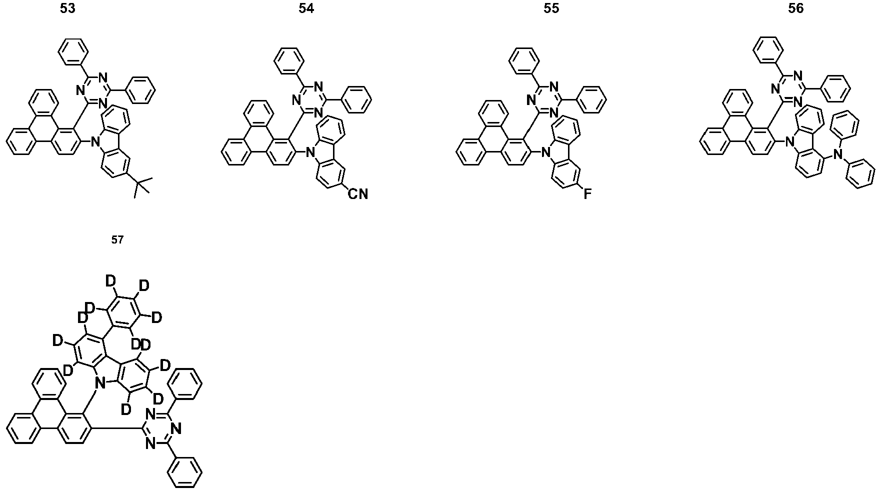
(Dn refers to the number of deuterium substitutions and indicates a structure substituted with one or more deuteriums).

6. A composition for an organic optoelectronic device, comprising
a first compound and a second compound,
wherein the first compound is the compound for the organic optoelectronic device according to claim 1, and
the second compound is represented by Chemical Formula 2; a combination of Chemical Formula 3 and Chemical Formula 4; or Chemical Formula 5: 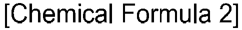 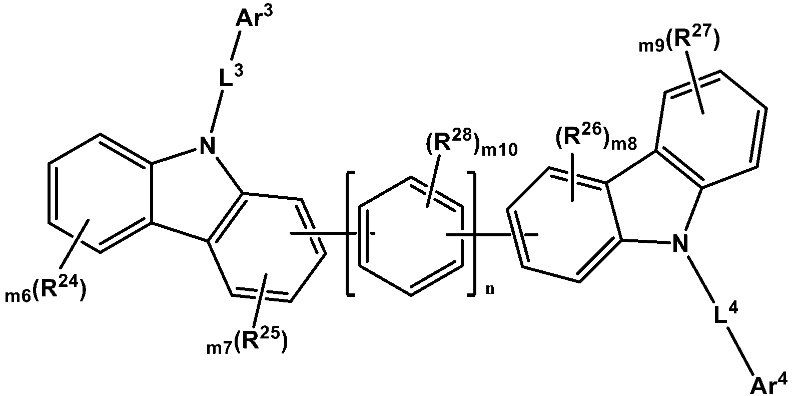
wherein, in Chemical Formula 2,
R²⁴ to R²⁸ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar³ and Ar⁴ are each independently a substituted or unsubstituted C6 to C20 aryl group or a substituted or unsubstituted C2 to C30 heterocyclic group,
L³ and L⁴ are each independently a single bond or a substituted or unsubstituted C6 to C20 arylene group,
m6, m9, and m10 are each independently one of integers of 1 to 4, when m6 is 2 or more, each R²⁴ is the same as or different from each other,
when m9 is 2 or more, each R²⁷ is the same as or different from each other,
when m10 is 2 or more, each R²⁸ is the same as or different from each other,
m7 and m8 are each independently one of integers of 1 to 3, when m7 is 2 or more, each R²⁵ is the same as or different from each other,
when m8 is 2 or more, each R²⁶ is the same as or different from each other, and
n is one of integers of 0 to 2; 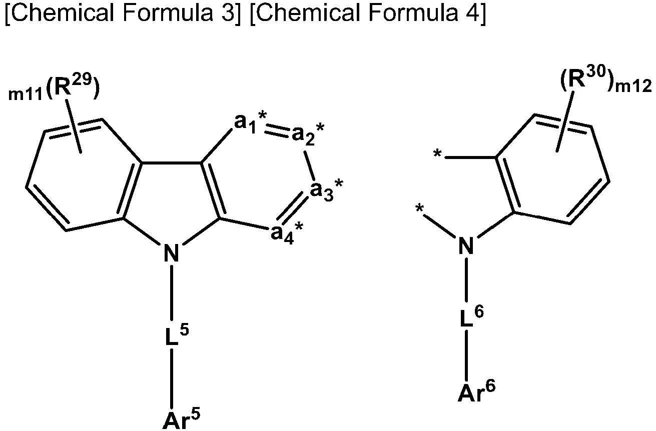
wherein, in Chemical Formula 3 and Chemical Formula 4,
among a1* to a4* in Chemical Formula 3, two adjacent ones are each a linking carbon (C) that is linked to *s in Chemical Formula 4,
among a₁* to a₄* of Chemical Formula 3, the remaining two that are not linked to Chemical Formula 4 are C-L³-R³,
L^{a}, L⁵, and L⁶ are each independently a single bond or a substituted or unsubstituted C6 to C20 arylene group,
R^{a}, R²⁹, and R³⁰ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group,
m11 and m12 are each independently one of integers of 1 to 4,
when m11 is 2 or more, each R²⁹ is the same as or different from each other, and
when m12 is 2 or more, each R³⁰ is the same as or different from each other; 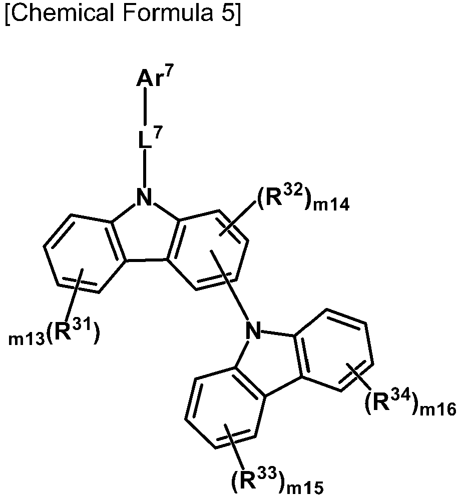
wherein, in Chemical Formula 5,
L⁷'s are each independently a single bond or a substituted or unsubstituted C6 to C20 arylene group,
R³¹ to R³⁴ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁷ is a substituted or unsubstituted C6 to C20 aryl group or a substituted or unsubstituted C2 to C30 heterocyclic group,
m13, m15, and m16 are each independently one of integers of 1 to 4,
m14 is one of integers of 1 to 3,
when m13 is 2 or more, each R³¹ is the same as or different from each other,
when m14 is 2 or more, each R³² is the same as or different from each other,
when m15 is 2 or more, each R³³ is the same as or different from each other, and
when m16 is 2 or more, each R³⁴ is the same as or different from each other.

7. The composition for the organic optoelectronic device as claimed in claim 6, wherein
Chemical Formula 2 is represented by Chemical Formula 2-8: 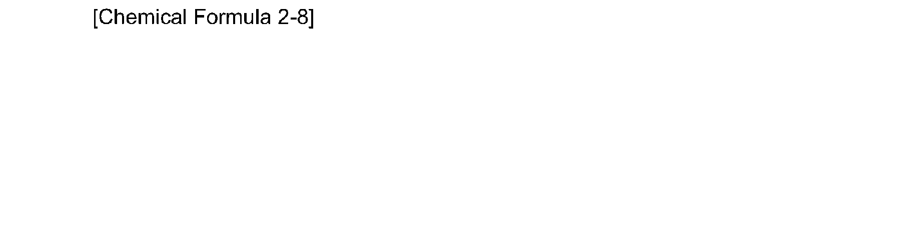 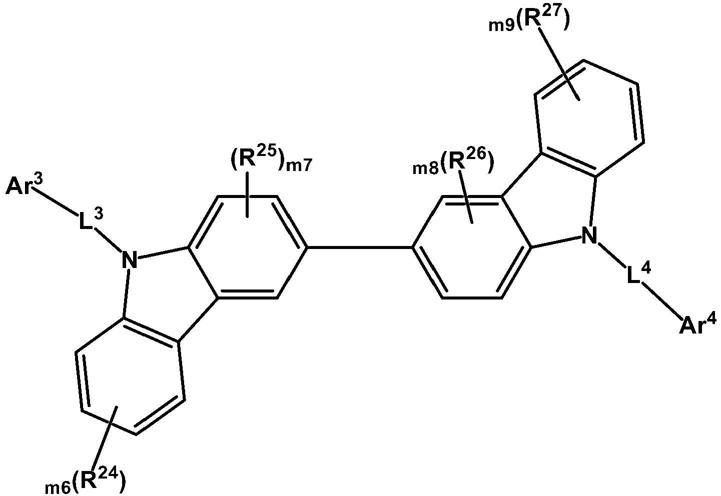
wherein, in Chemical Formula 2-8,
R²⁴ to R²⁷ are each independently hydrogen, deuterium, or a substituted or unsubstituted C6 to C12 aryl group,
m6 and m9 are each independently one of integers of 1 to 4,
m7 and m8 are each independently one of integers of 1 to 3, and
L³-Ar³ and L⁴-Ar⁴ are each independently one of substituents listed in Group II, 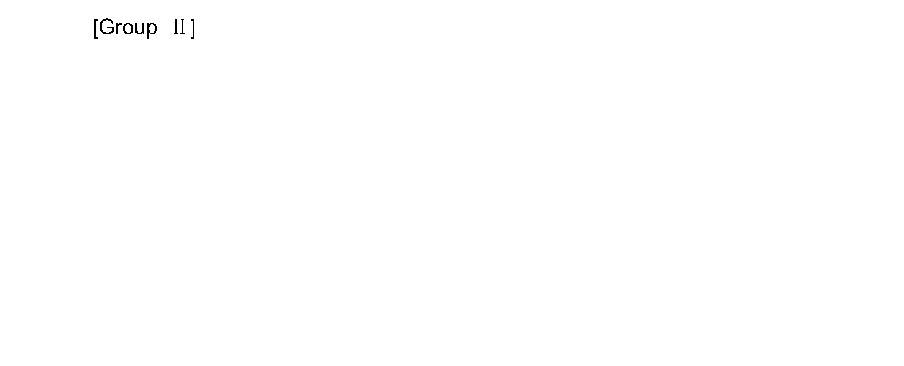 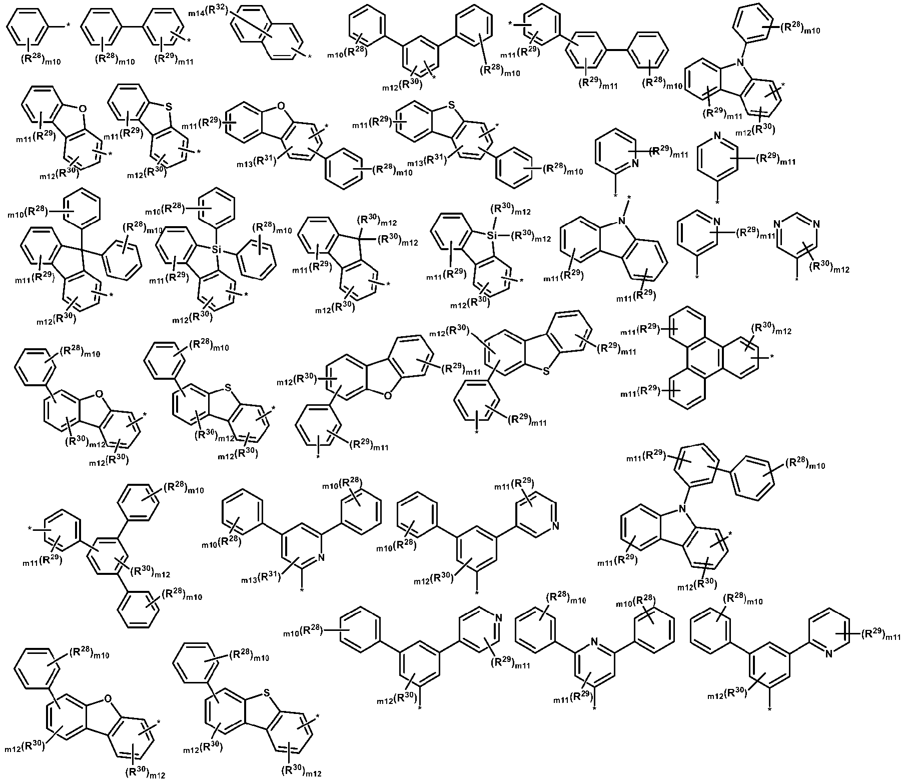
wherein, in Group II,
R²⁸ to R³² are each independently hydrogen, deuterium, a cyano group, a C1 to C10 alkyl group, or a C6 to C12 aryl group,
m10 is one of integers of 1 to 5,
m11 is one of integers of 1 to 4,
m12 is one of integers of 1 to 3,
m13 is an integer of 1 or 2,
m14 is one of integers of 1 to 7, and
* is a linking point.

8. The composition for the organic optoelectronic device as claimed in claim 6, wherein
the combination of Chemical Formula 3 and Chemical Formula 4 is represented by Chemical Formula 3C: 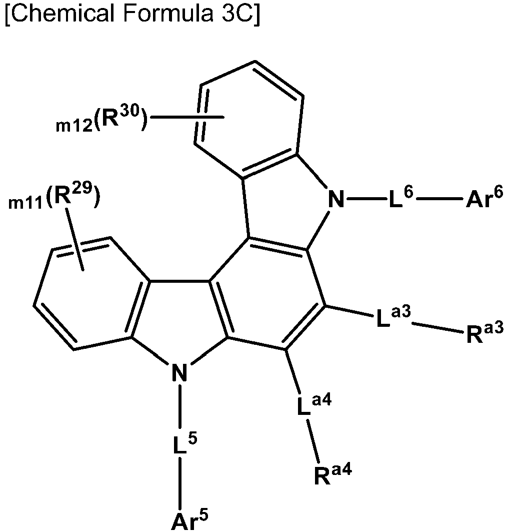
wherein, in Chemical Formula 3C,
L²³ and L^{a4} are a single bond,
R²⁹, R³⁰, R^{a3}, and R^{a4} are each independently hydrogen, deuterium, or a C6 to C12 aryl group,
m11 and m12 are each independently one of integers of 1 to 4, and
L⁵-Ar⁵ and L⁶-Ar⁶ are each independently one of the substituents listed in Group II, 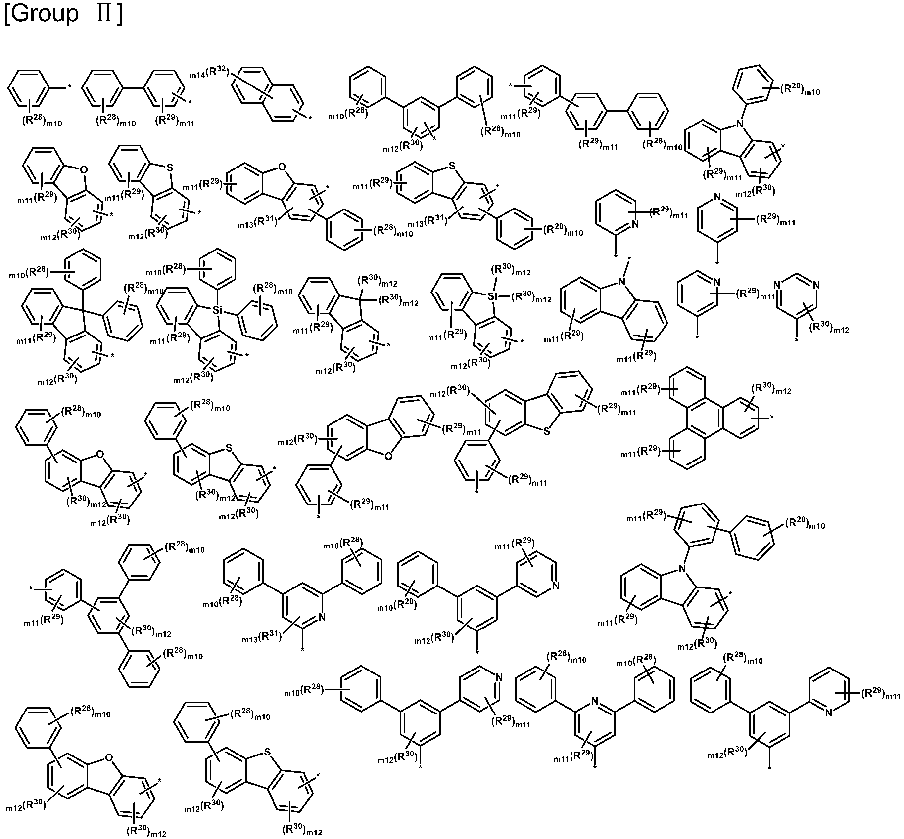
wherein, in Group II,
R²⁸ to R³² are each independently hydrogen, deuterium, a cyano group, a C1 to C10 alkyl group, or a C6 to C12 aryl group,
m10 is one of integers of 1 to 5,
m11 is one of integers of 1 to 4,
m12 is one of integers of 1 to 3,
m13 is an integer of 1 or 2,
m14 is one of integers of 1 to 7, and
* is a linking point.

9. An organic optoelectronic device, comprising
an anode and a cathode facing each other, and
at least one organic layer positioned between the anode and the cathode,
wherein the organic layer comprises
the compound for the organic optoelectronic device as claimed in any one of claims 1 to 5; or
the composition for the organic optoelectronic device as claimed in any one of claims 6 to 8.

10. The organic optoelectronic device as claimed in claim 9, wherein
the organic layer comprises a light emitting layer, and
the light emitting layer comprises the compound for the organic optoelectronic device or the composition for the organic optoelectronic device.

11. A display device comprising the organic optoelectronic device as claimed in claim 9.
